# EUROPEAN PATENT SPECIFICATION

(11) **EP 3 321 279 B1**
(45) Date of publication and mention of the grant of the patent: **12.02.2020**
(21) Application number: 16848039.0
(22) Date of filing: 13.09.2016
(51) Int. Cl.: C07K 14/605, A61K 38/26, A61P 3/10

(54) **EXENATIDE MODIFIER AND USE THEREOF**
EXENATID-MODIFIKATOR UND VERWENDUNG DAVON
MODIFICATEUR DE L'EXÉNATIDE ET UTILISATION DE CE DERNIER

(30) Priority: 25.09.2015 CN 201510619012
(43) Date of publication of application: 16.05.2018
(73) Proprietor: BrightGene Bio-Medical Technology Co., Ltd., BioBay Suzhou Industrial Park Suzhou Jiangsu 215123 (CN)
(72) Inventor: YUAN, Jiandong, Suzhou Jiangsu 215123 (CN); HUANG, Yangqing, Suzhou Jiangsu 215123 (CN); SONG, Yunsong, Suzhou Jiangsu 215123 (CN); YUAN, Fang, Suzhou Jiangsu 215123 (CN)
(74) Representative: J A Kemp LLP
(86) International application number: PCT/CN2016/098844
(87) International publication number: WO 2017/050157

(56) References cited:
- WO-A1-2006/097538
- CN-A- 101 980 725
- CN-A- 103 237 561
- US-A1- 2010 184 641
- JUHO LEE ET AL: "Preparation and evaluation of palmitic acid-conjugated exendin-4 with delayed absorption and prolonged circulation for longer hypoglycemia", INTERNATIONAL JOURNAL OF PHARMACEUTICS, vol. 424, no. 1-2, 1 March 2012 (2012-03-01), pages 50-57, XP055069410, ISSN: 0378-5173, DOI: 10.1016/j.ijpharm.2011.12.050
- CHAE S Y ET AL: "The fatty acid conjugated exendin-4 analogs for type 2 antidiabetic therapeutics", JOURNAL OF CONTROLLED RELEASE, ELSEVIER, AMSTERDAM, NL, vol. 144, no. 1, 21 May 2010 (2010-05-21), pages 10-16, XP027036550, ISSN: 0168-3659 [retrieved on 2010-04-30]
- SHECHTER YORAM ET AL: "Newly designed modifier prolongs the action of short-lived peptides and proteins by allowing their binding to serum albumin.", BIOCONJUGATE CHEMISTRY 15 AUG 2012, vol. 23, no. 8, 15 August 2012 (2012-08-15), pages 1577-1586, XP002779559, ISSN: 1520-4812
- KONG J H ET AL: "Long acting hyaluronate @? exendin 4 conjugate for the treatment of type 2 diabetes", BIOMATERIALS, ELSEVIER SCIENCE PUBLISHERS BV., BARKING, GB, vol. 31, no. 14, 1 May 2010 (2010-05-01), pages 4121-4128, XP026947588, ISSN: 0142-9612, DOI: 10.1016/J.BIOMATERIALS.2010.01.091 [retrieved on 2010-02-10]
- ZHOU J ET AL: "Preparation and PEGylation of exendin-4 peptide secreted from yeast Pichia pastoris", EUROPEAN JOURNAL OF PHARMACEUTICS AND BIOPHARMACEUTICS, ELSEVIER SCIENCE PUBLISHERS B.V., AMSTERDAM, NL, vol. 72, no. 2, 1 June 2009 (2009-06-01), pages 412-417, XP026119126, ISSN: 0939-6411, DOI: 10.1016/J.EJPB.2009.02.001 [retrieved on 2009-02-07]

## Description

### FIELD

The present invention relates to the field of therapeutic peptides, particularly to exenatide modifiers, a pharmaceutical composition containing the same, and the use of the modifiers and composition in the treatment of diseases associated with glycometabolism.

### BACKGROUND

Exenatide (or Exendin-4, trade name by Byetta) is a polypeptide of 39 amino acids with a molecular weight of 4186.6, the molecular formula of which is C₁₈₄H₂₈₂N₅₀O₆₀S, and the amino acid sequence is: His-Gly-Glu-Gly-Thr-Phe-Thr-Ser-Asp-Leu-Ser-Lys-Gln-Met-Glu-Glu-Glu-Ala-Val-Arg-Leu -Phe-Ile-Glu-Trp-Leu-Lys-Asn-Gly-Gly-Pro-Ser-Ser-Gly-Ala-Pro-Pro-Pro-Ser-NH2; which is produced and sold by Amylin Pharmaceuticals and Eli Lilly company (Eli Lillyand Company). Exenatide has been approved by FDA in April 2005, which belongs to subcutaneous injection preparation, with effects of promoting the glucose-dependent insulin secretion, recovering the insulin secretion of the first phase, inhibiting the glucagon secretion, slowing the emptying of gastric contents, improving the function of pancreatic β cells, and the like, being very useful in the treatment of type II diabetes, for example, to improve and control the blood glucose of patients with type II diabetes which are not ideal when treated by metformin and sulfonylurea drugs.

Exenatide is a synthetic form of the hormone, exendin-4, in the saliva of lizard, Heloderma suspectum (Gilamonster) grown in several states in the southwestern United States (J.Biol.Chem.1990, 265, 20259-20262; J.Biol.Chem.1992, 267, 7402-7405), which is an analogue of human glucagon-like peptide-1 (GLP-1), the amino acid sequence of which is partially overlapped with the amino acid sequence of GLP-1, being a potent GLP-1 receptor agonist, and also being known as an incretin agonist since that exenatide simulates the glucose regulation effect of GLP-1. Unlike sulfonylureas and meglitinides, exenatide increases the synthesis and secretion of insulin only in the presence of glucose, reducing the risk of hypoglycemia. Some physicians will also use Byetta in the treatment of insulin resistance.

Nevertheless, properties such as short half-life in vivo, poor physical and chemical stabilities, susceptible to degradation by various proteases in vivo are common in the protein/ /polypeptide drugs, such that these drugs often require multiple injections in a day, bring patients lots of pain and inconvenience. PEGylation emerged in 1970s has been proven to be a technology suitable for the field of the current administration of proteins/polypeptides. However, after being modified simply using PEG, the activities of drugs will generally decline.

A series of different methods have been used to modify the structures of GLP-1 analogues, so as to provide a longer duration of action in vivo. CN1384755 discloses novel exendin agonist preparations and their dosing methods, which discloses the compound structure of exenatide and its preparation. CN102532303 discloses the method of synthesizing exenatide conjugated with polyethylene glycol, by conjugating the methoxy polyethylene glycol residue with the amino of lysine residue in the molecule of exenatide or the amino of the histidine residue at the N terminal; CN101980725 discloses the structure of fatty acid-PEG-exenatide, with the modification site of PEG on the N terminal of His; WO2005028516 and WO2012035139 also disclose the structure of fatty acid-PEG-exenatide. Chinese patent CN101215324 discloses a mimetic peptide of short exenatide peptide obtaining from the restructuring of exenatide. Chinese patent CN101125207 reports the PEG modification on Exendin-4. WO99/43708 discloses the GLP-1 (7-35) and GLP-1 (7-36) derivatives with lipophilic substituates linked to the amino acid residues at the C terminal. WO2013059323A1 discloses a PEG-conjugated exenatide and its preparation.

CN102397558 discloses the use of PEG or PEG modification with methyl substitution at the terminal, after substituting some amino acids in exendin-4 for cysteine. CN102421796 discloses that one or more polyethylene glycols polymerize to the cysteine of exendin variants, discloses an exenatide in which one amino acid is substituted with a cysteine, and then modified with polyethylene glycol on the cysteine. CN102827270 discloses an exendin-4-Cys-PEG derivative, specifically introducing one cysteine at the C-terminal of the inactive area of the exenatide molecule, and coupling with maleimido polyethylene glycol, wherein it is terminated with methyl at the polyethylene glycol terminal.

Notwithstanding these efforts in so many aspects have been made, the current existing exendin-4 or its variants and various modifications still possess some drawbacks, including the high dosing frequency when used in vivo, bring patients great burdens on their body, mentality and economy, restricting the compliance of patients, and being incapable of widely application. There remains a great requirement on the active long-acting GLP-1 analogues for diabetic populations, so it is a need to develop new exenatide derivatives, making them with long durations of action, good stabilities, good hypoglycemic effects, while maintaining low toxicities and good activities.

### SUMMARY

The present invention is to overcome the drawbacks of the presently disclosed exenatide modifiers GLP-1 receptor, with low binding force and short hypoglycemia duration, thus causing poor effects or frequent injections in clinical use.

It is known to those skilled in the art that, in the bioactive molecule with a conjugated polymeric group, the biological activity of the conjugated biological molecule will gradually decrease exponentially as the molecular weight of the conjugated group increases. It is also known to those skilled in the art that as the molecular weight of the polymeric group increases, the biological half-life and/or plasma half-life and the systematic drug exposure of the conjugated biological molecule will gradually prolong or increase.

It is discovered unexpectedly by those skilled in the present invention that, through the modification of exenatide by those skilled in the present invention, the pharmacokinetical properties have been improved, thus increasing the hypoglycemia duration. And compared with exenatide, the molecules of the present invention still retain most of the activities of GLP-1 receptor agonists, which means that the molecules of the present invention are high in the activity of the GLP-1 receptor agonist, and have long hypoglycemia duration, with a possibility of being drugs with long durations of action, good stabilities, and good hypoglycemic effects in the future clinical applications.

On the one hand, the present invention provides such an exenatide modifier
or pharmaceutically acceptable salts thereof having GLP-1 receptor agonist activity, as shown in formula (**I**):

(Ex-4)-L-Y (**I**)

wherein, Ex-4 is Exendin-4; L is 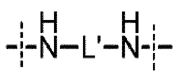 for connecting Ex-4 with Y; L' is a hydrophilic chain containing an ether group; Y is an aliphatic chain with a terminal carboxyl group, wherein the exenatide modifier is: 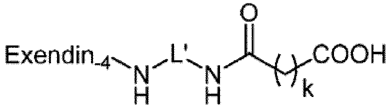 k is any integer between 6-20.

Furthermore, in the exenatide modifier (Ex-4)-L-Y of the present invention, the hydrophilic linking arm L may be selected from:
(1) 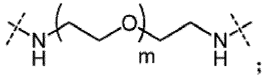
(2) 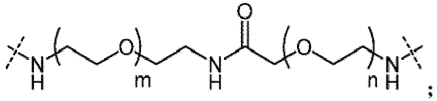
(3) 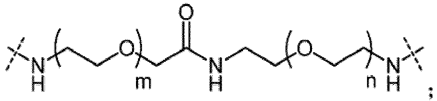
(4) 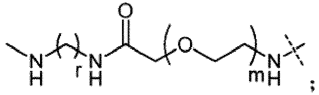
(5) 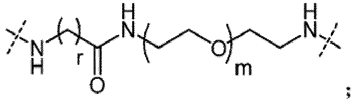
(6) 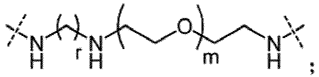
(7) 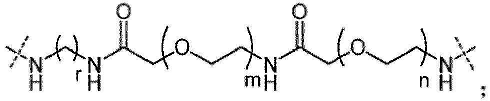
(8) 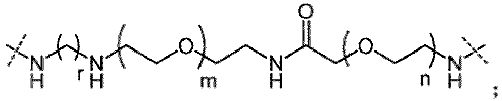
(9) 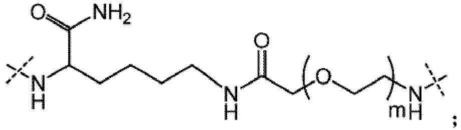
(10) 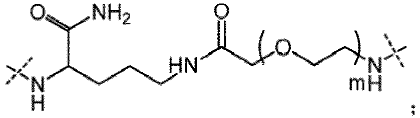
(11) 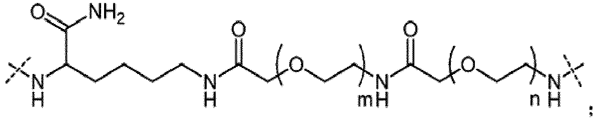
(12) 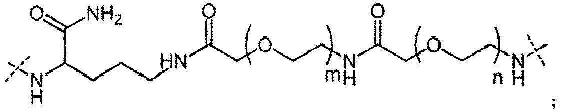
(13) 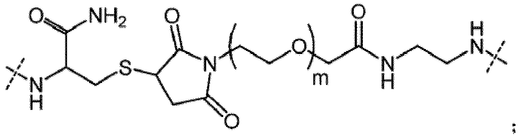
(14) 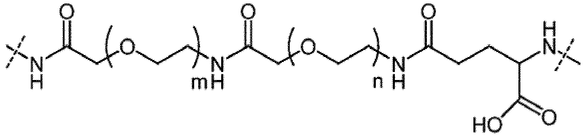
wherein m is any integer between 2-20; n is any integer between 2-20; r is any integer between 1-6.

The specific structure is as follow:

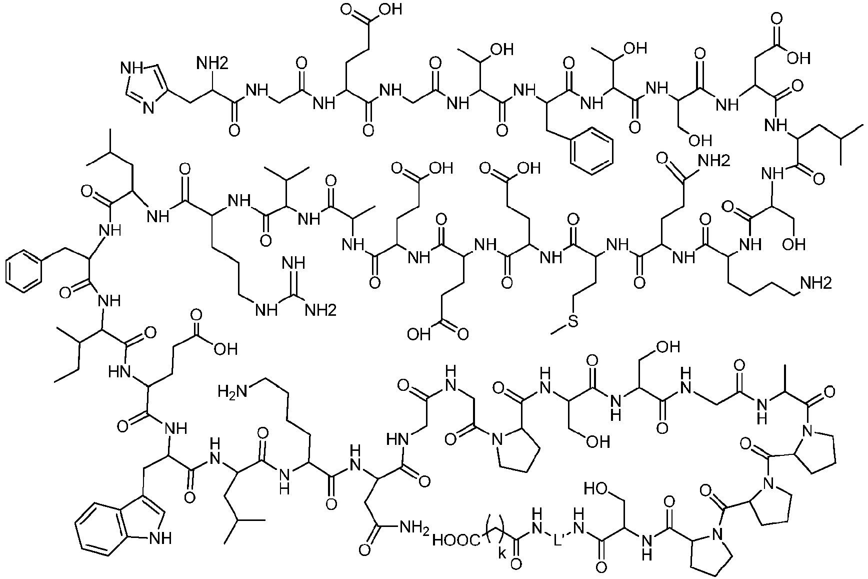

The present invention preferably provides such exenatide modifiers: 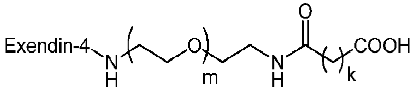 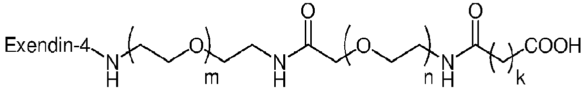 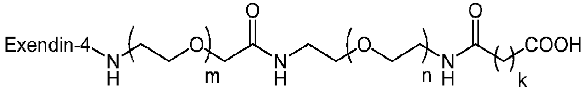 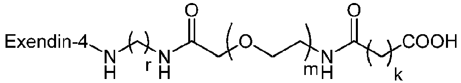 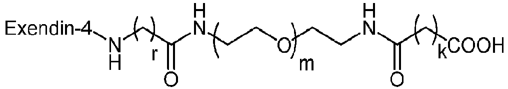 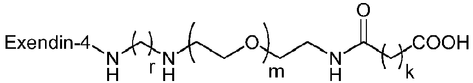 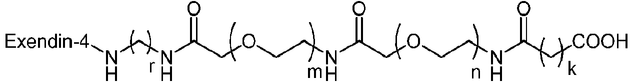 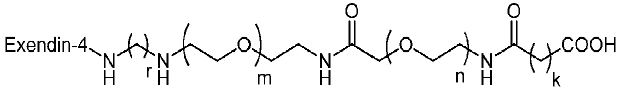 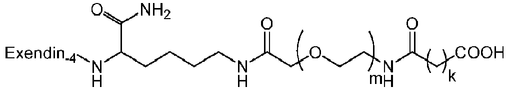 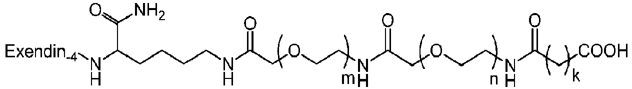 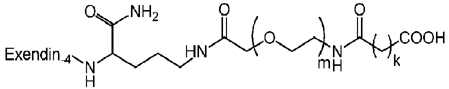 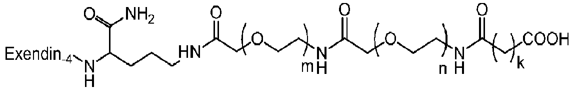 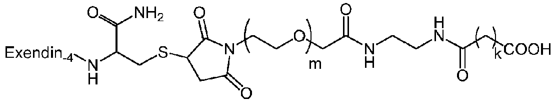 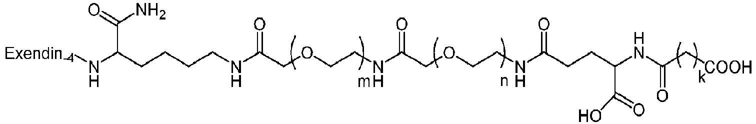 wherein m is any integer between 2-20; n is any integer between 2-20; r is any integer between 1-6; k is any integer between 6-20.

More specifically, the present invention provides the following exenatide modifiers:
Series 1: see Examples 1-5

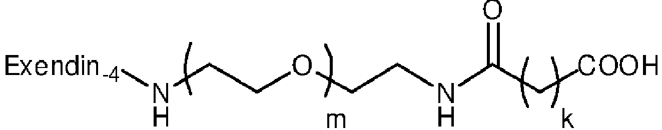

| Compound | Compound 1 | Compound 2 | Compound 3 | Compound 4 | Compound 5 |
|---|---|---|---|---|---|
| m | 5 | 2 | 20 | 14 | 10 |
| k | 16 | 20 | 6 | 10 | 14 |

Series 2: see Examples 6-10

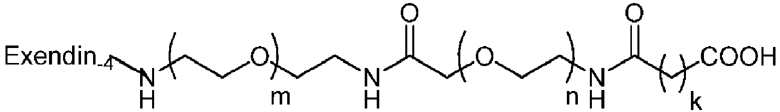

| Compound | Compound 6 | Compound 7 | Compound 8 | Compound 9 | Compound 10 |
|---|---|---|---|---|---|
| m | 5 | 2 | 20 | 10 | 11 |
| n | 3 | 7 | 2 | 20 | 15 |
| k | 16 | 20 | 12 | 6 | 10 |

Series3: see Examples 11-15

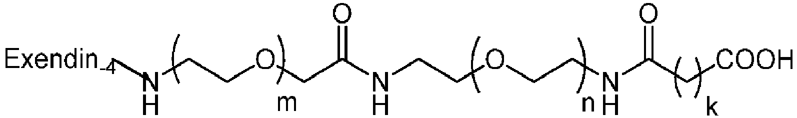

| Compound | Compound 11 | Compound 12 | Compound 13 | Compound 14 | Compound 15 |
|---|---|---|---|---|---|
| m | 3 | 2 | 20 | 10 | 11 |
| n | 5 | 9 | 2 | 20 | 15 |
| k | 16 | 20 | 12 | 6 | 10 |

Series 4: see Examples 16-20

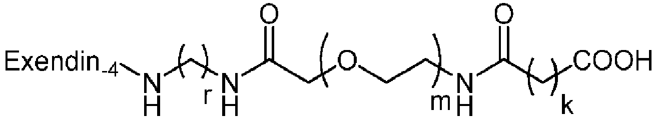

| Compound | Compound 16 | Compound 17 | Compound 18 | Compound 19 | Compound 20 |
|---|---|---|---|---|---|
| r | 2 | 3 | 3 | 3 | 6 |
| m | 6 | 2 | 15 | 10 | 20 |
| k | 16 | 18 | 10 | 20 | 6 |

Series 5: see Examples 21-25

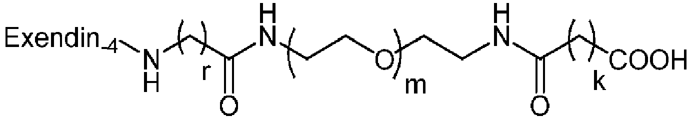

| Compound | Compound 21 | Compound 22 | Compound 23 | Compound 24 | Compound 25 |
|---|---|---|---|---|---|
| r | 1 | 2 | 3 | 3 | 6 |
| m | 5 | 2 | 15 | 10 | 20 |
| k | 16 | 18 | 10 | 20 | 6 |

Series 6: see Examples 26-30

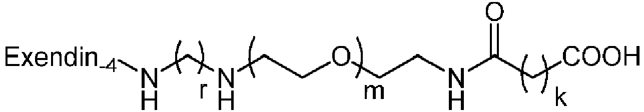

| Compound | Compound 26 | Compound 27 | Compound 28 | Compound 29 | Compound 30 |
|---|---|---|---|---|---|
| r | 2 | 2 | 3 | 3 | 6 |
| m | 5 | 2 | 15 | 10 | 20 |
| k | 16 | 14 | 10 | 20 | 6 |

Series 7: see Examples 31-35

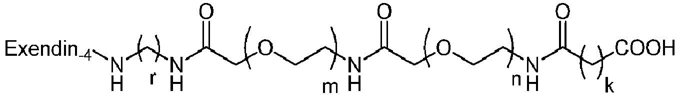

| Compound | Compound 31 | Compound 32 | Compound 33 | Compound 34 | Compound 35 |
|---|---|---|---|---|---|
| r | 2 | 2 | 3 | 3 | 6 |
| m | 6 | 2 | 15 | 10 | 20 |
| n | 3 | 20 | 5 | 10 | 2 |
| k | 16 | 14 | 10 | 20 | 6 |

Series 8: see Examples 36-40

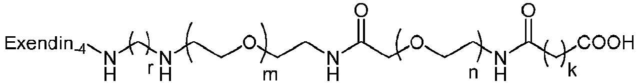

| Compound | Compound 36 | Compound 37 | Compound 38 | Compound 39 | Compound 40 |
|---|---|---|---|---|---|
| r | 2 | 2 | 3 | 3 | 6 |
| m | 5 | 2 | 15 | 10 | 20 |
| n | 6 | 20 | 5 | 10 | 2 |
| k | 16 | 14 | 10 | 20 | 6 |

Series 9: see Examples 41-45

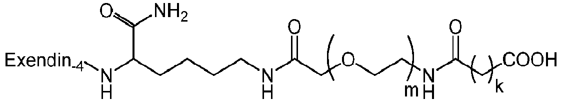

| Compound | Compound 41 | Compound 42 | Compound 43 | Compound 44 | Compound 45 |
|---|---|---|---|---|---|
| m | 6 | 2 | 20 | 10 | 15 |
| k | 16 | 14 | 10 | 20 | 6 |

Series 10: see Examples 46-50

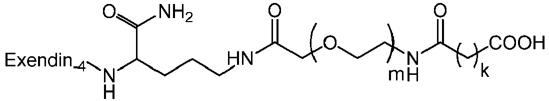

| Compound | Compound 46 | Compound 47 | Compound 48 | Compound 49 | Compound 50 |
|---|---|---|---|---|---|
| m | 6 | 2 | 20 | 10 | 15 |
| k | 16 | 14 | 10 | 20 | 6 |

Series 11: see Examples 51-55

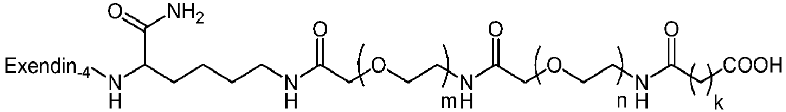

| Compound | Compound 51 | Compound 52 | Compound 53 | Compound 54 | Compound 55 |
|---|---|---|---|---|---|
| m | 6 | 2 | 20 | 10 | 15 |
| n | 3 | 9 | 16 | 2 | 20 |
| k | 16 | 14 | 10 | 20 | 6 |

Series 12: see Examples 56-60

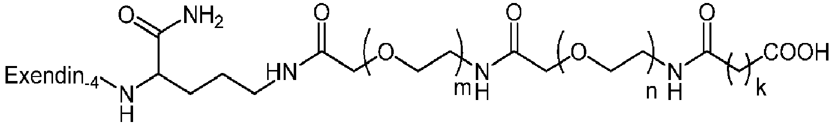

| Compound | Compound 56 | Compound 57 | Compound 58 | Compound 59 | Compound 60 |
|---|---|---|---|---|---|
| m | 6 | 2 | 20 | 10 | 15 |
| n | 3 | 9 | 16 | 2 | 20 |
| k | 16 | 14 | 10 | 20 | 6 |

Series 13: see Examples 61-66

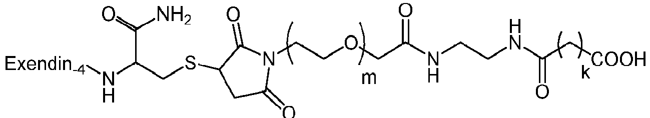

| Compound | Compound 61 | Compound 62 | Compound 63 | Compound 64 | Compound 65 | Compound 66 |
|---|---|---|---|---|---|---|
| m | 2 | 4 | 5 | 7 | 9 | 10 |
| k | 20 | 10 | 16 | 8 | 16 | 6 |

Series 14: see Example 67 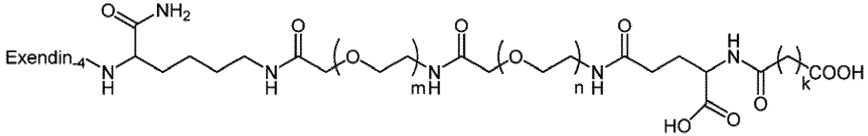 m = 6; n = 3; k = 16

Also discussed herein is the use of the exenatide modifier or pharmaceutically acceptable salts thereof in preparing drugs serving as a GLP-1 receptor agonist and a use in preparing drugs for preventing and/or treating diseases and/or symptoms associated with a low GLP-1 receptor activity. The present invention also provides the above described exenatide modifiers for use in the treatment of diseases and/or symptoms associated with glycometabolism, use in the treatment of diabetes, use in treatment of fatty liver, and use in a method for losing weight.

In the third aspect, the present invention provides a composition comprising an exenatide modifier or pharmaceutically acceptable salts thereof and optionally pharmaceutically acceptable carriers.

Also described herein is the use of the above described composition in preparing drugs serving as a GLP-1 receptor agonist and a use in preparing drugs for preventing and/or treating diseases and/or symptoms associated with a low GLP-1 receptor activity. The present invention also provides the above described composition for use in treating diseases and/or symptoms associated with glycometabolism, use in the treatment of diabetes, use in the treatment of fatty liver, and use in a method for losing weight.

The exenatide modifiers provided in the present invention not only possess higher GLP-1 receptor agonistic activities, but also long durations of hypoglycemia. It is illustrated through the following pharmacological tests.

Each test sample was repectively dissolved in double distilled water to a final concentration of 1.0×10⁻²mol/L, and stored at 4°C. PC12 cells were cultured in a 25cm² culture flask placed in the CO₂ incubator (37°C, 95% air, 5% CO₂), with the culture medium of DMEM (Dulbecco's Modified Eagle's Medium, pH=7.4, high glucose), in which there were added 5% fetal bovine serum and 10% horse serum. The well-grown PC12 cells were digested with 0.25% pancreatin, the cell concentration being adjusted to 1.0×10⁵ cells/ml, seeded in a 24-well plate. When cells grew to the density of 60-70%, they were washed twice with PBS (Phosphate Buffer Saline) with the addition of PBS containg 1% BSA (Bovine Serum Albumin) for 1ml each, and the test drugs were respectively divided into 5 gradients of concentration (10⁻¹⁰, 10⁻⁹, 10⁻⁸, 10⁻⁷, 10⁻⁶ mol/L) and co-incubated with IBMX (3-isobutyl-1-methylxanthine, 100µmol/L) for 30min, 3 operations of multiple holes being done for each concentration of the samples. Once the intervention time of drugs end, the cells were collected immediately, suspended with cold PBS, and the cell concentration was adjusted to 1.0×10⁷/ml. One volume of IN HCl was immediately added into 9 volumes of cell suspension, incubated for 10min at room temperature, ultrasonicated with an ultrasonic apparatus for 15s. At 4°C, they were centrifuged for 10min at 1000rpm to remove cell debris. The supernatant was added into 1N NaOH of equal volme with 1N HCl to neutralization (wherein 1N represents one equivalent), the resulting solution being the sample solution containing cAMP, stored at -20°C ready for detection. Non-Interference Protein Assay Kit was employed to detect the total protein concentration in the sample. The content of cAMP in the cell lysate was detected using ELISA kit following the instruction of the kit, and the OD (Optical Density) value was determined at 450nm by the BIO-RAD 680 Microplate Reader. Based on the OD value of the standard, CurveExpert 1.3 software was employed to fit curves and compute the standard curve formula, and calculate the concentration of each sample. Computer programs Microsoft Excel and GraphPad Prism 5 software were used for data processing and charting to calculate EC₅₀ (half effective concentration, Concentration for 50% of Maximal Effect) of each test drug.

**Table 1 Effects of the compounds on the cAMP activities in cells**

| **Compound** | **EC₅₀** | **Compound** | **EC₅₀** | **Compound** | **EC₅₀** |
|---|---|---|---|---|---|
| 1 | 5.879 | 23 | 8.014 | 45 | 6.204 |
| 2 | 6.423 | 24 | 7.963 | 46 | 5.674 |
| 3 | 6.174 | 25 | 8.257 | 47 | 5.916 |
| 4 | 6.075 | 26 | 8.019 | 48 | 5.705 |
| 5 | 6.278 | 27 | 8.742 | 49 | 6.342 |
| 6 | 6.346 | 28 | 7.878 | 50 | 6.154 |
| 7 | 7.217 | 29 | 8.042 | 51 | 5.341 |
| 8 | 7.064 | 30 | 8.173 | 52 | 5.462 |
| 9 | 5.974 | 31 | 8.425 | 53 | 5.674 |
| 10 | 6.127 | 32 | 8.053 | 54 | 5.553 |
| 11 | 7.236 | 33 | 8.172 | 55 | 5.697 |
| 12 | 8.042 | 34 | 8.345 | 56 | 6.247 |
| 13 | 7.578 | 35 | 8.296 | 57 | 5.969 |
| 14 | 7.642 | 36 | 8.247 | 58 | 6.374 |
| 15 | 7.539 | 37 | 7.942 | 59 | 6.545 |
| 16 | 8.742 | 38 | 8.296 | 60 | 6.278 |
| 17 | 9.416 | 39 | 8.472 | 61 | 6.212 |
| 18 | 7.753 | 40 | 8.257 | 62 | 5.774 |
| 19 | 7.942 | 41 | 5.554 | 63 | 5.692 |
| 20 | 8.363 | 42 | 5.872 | 64 | 5.726 |
| 21 | 8.567 | 43 | 5.742 | 65 | 5.948 |
| 22 | 9.642 | 44 | 6.117 | 66 | 5.970 |
| | | | | 67 | 5.742 |
| Exendin-4 | 5.096 nmol/L | | | | |

After binding, GLP-1 and GLP-1 receptors (G coupling proteins of β receptor family) activate cyclic adenosine monophosphate (cAMP) and mitogen-activated protein kinase (MAPK) pathway. GLP-1 receptors of mature pancreatic β cells coupled with Gs, activating the adenylate cyclase and producing cAMP, the latter, coordinated with glucose, stimulating the synthesis and secretion of insulin, stimulating the gene transcription of insulin and the biosynthesis of proinsulin, reducing the glucagon concentration and inhibiting the secretion of glucagon, enhancing the sensitivity of cells on insulin, stimulating the insulin-dependent glycogen synthesis, reducing the postprandial blood sugar concentration. The smaller the EC₅₀ was, indicating the higher drug GLP-1 receptor agonistic activities.

It was shown from the results in Table 1 that, the compounds of the present invention were comparable to exenatide in activity or only slightly decreased, indicating that the modifications on exenatide in the present invention have no influences on the GLP-1 receptor agonistic activities.

### Hypoglycemic effects on spontaneous type 2 diabetes db/db mice

C57BL/6db/db9 mice (male) at the age of 5∼6 weeks were purchased from Model Animal Research Center of Nanjing University, the experimental animals being feed in the SPF animal houses. The animal houses were well-ventilated, equipped with air conditioners, keeping the temperature at 20∼25°C and the humidity at 40%∼70%, with the ventilation rate of 10∼15 times/h, light and dark each for 12 hours. Experimental animals had free access to food and water, and each mouse was marked with an ear tag. Mice were used in the experiment once a week, with the period of no more than three weeks. After one week acclimation, the capillary blood glucoses at the tail tip of mice were determined by MAJOR glucose meter. 340 mice with blood glucose level greater than 16.7mmol/L were chosen and randomly grouped into 68 groups according to the blood glucose level. The model control group was given 5 mL/kg PBS (pH=7.4) by subcutaneous injection, the positive control group 1 was given Exenatide (10 µg/kg, 5 mL/kg) by subcutaneous injection, the dosing groups were respectively injected compounds 1-15(10 µg/kg, 5 mL/kg) subcutaneously. After administration, the blood glucoses at 0, 1, 2, 4, 8, 12, 18, 24, 30, 36, 42, 48, 72h were determined by a glucose meter, and all data was input into Graphpad Prism to calculate the mean blood glucose. The maximum hypoglycemic effect (the maximum reduction rate compared with the model group), the maximum hypoglycemic time (the last time point at which the blood glucose decreased significantly compared with the model group), and the area under the curve were calculated.

**Table 2 Hypoglycemic effects on spontaneous type 2 diabetes db/db mice (h)**

| Compound | Maximum hypoglycemic time | Compound | Maximum hypoglycemic time | Compound | Maximum hypoglycemic time |
|---|---|---|---|---|---|
| 1 | 36 | 23 | 42 | 45 | 30 |
| 2 | 36 | 24 | 42 | 46 | 42 |
| 3 | 30 | 25 | 36 | 47 | 42 |
| 4 | 36 | 26 | 36 | 48 | 48 |
| 5 | 42 | 27 | 30 | 49 | 48 |
| 6 | 36 | 28 | 36 | 50 | 36 |
| 7 | 42 | 29 | 42 | 51 | 48 |
| 8 | 42 | 30 | 30 | 52 | 48 |
| 9 | 36 | 31 | 36 | 53 | 42 |
| 10 | 36 | 32 | 36 | 54 | 48 |
| 11 | 30 | 33 | 30 | 55 | 42 |
| 12 | 42 | 34 | 48 | 56 | 48 |
| 13 | 36 | 35 | 30 | 57 | 48 |
| 14 | 30 | 36 | 42 | 58 | 42 |
| 15 | 36 | 37 | 42 | 59 | 48 |
| 16 | 36 | 38 | 36 | 60 | 42 |
| 17 | 42 | 39 | 48 | 61 | 48 |
| 18 | 42 | 40 | 36 | 62 | 42 |
| 19 | 48 | 41 | 42 | 63 | 48 |
| 20 | 30 | 42 | 42 | 64 | 36 |
| 21 | 48 | 43 | 42 | 65 | 40 |
| 22 | 48 | 44 | 48 | 66 | 36 |
| | | | | 67 | 42 |
| Exendin-4 | | | 4 | | |

It was shown from the results in Table 2 that compared with Exenatide, the compounds of the present invention have a great advantage in terms of maintaining the hypoglycemia time, prolonging the maximum hypoglycemic time from 4h to 30h-48h.

Based on the results of the above two tests, the preferred compounds of the present invention are compounds 41, 42, 43, 46, 47, 48, 51-63, 65, 67.

In summary, the exenatide modifiers of the present invention were comparable to exenatide in activity or only slightly decreased, retained most of the GLP-1 receptor agonistic activities, and the modifications on exenatide had no influences on the GLP-1 receptor agonistic activities. Meanwhile, the exenatide modifiers of the present invention have a great advantage in terms of maintaining the hypoglycemia time, prolonging the maximum hypoglycemic time from 4h to 30h-48h. The molecules of the present invention not only have high GLP-1 receptor agonistic activities, but also long hypoglycemia durations, with the possibility of becoming drugs with long action in vivo, good stabilities and good hypoglycemic effects in the future clinical applications.

### Specific Examples

Amino acids and their abbreviations and short names in English are shown in the following table:

| Protected amino acids required in the Fmoc process solid-phase synthesis and their abbreviations | | | |
|---|---|---|---|
| Name | Protected Amino Acids | Abbreviation of three letters | Abbreviation of single letter |
| Alanine | Fmoc-Ala-OH | Ala | A |
| Aspartic acid | Fmoc-Asp(OtBu)-OH | Asp | D |
| Glutamic acid | Fmoc-Glu(OtBu)-OH | Glu | G |
| Phenylalanine | Fmoc-Phe-OH | Phe | F |
| Glycine | Fmoc-Gly-OH | Gly | G |
| Histidine | Fmoc-His(Trt)-OH | His | H |
| Isoleucine | Fmoc-Ile-OH | Ile | I |
| Lysine | Fmoc-Lys(Boc)-OH | Lys | K |
| Leucine | Fmoc-Leu-OH | Leu | L |
| Methionine | Fmoc-Met-OH | Met | M |
| Asparagine | Fmoc-Asn(Trt)-OH | Asn | N |
| Proline | Fmoc-Pro-OH | Pro | P |
| Glutamine | Fmoc-Gln(Trt)-OH | Gln | Q |
| Arginine | Fmoc-Arg (Pbf)-OH | Arg | R |
| Serine | Fmoc-Ser(tBu)-OH | Ser | S |
| Threonine | Fmoc-Thr(tBu)-OH | Thr | T |
| Valine | Fmoc-Val-OH | Val | V |
| Tryptophan | Fmoc-Trp(Boc)-OH | Trp | W |
| Tyrosine | Fmoc-Tyr(tBu)-OH | Tyr | Y |
| Lysine | Fmoc-Lys(Alloc)-OH | Lys | K |
| Ornithine | Fmoc-Orn(Alloc)-OH | Orn | |

### Example 1 Preparation of compound 1

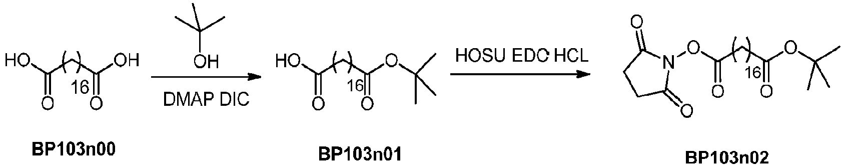

### Preparation of BP103n01

To a 50mL three-necked flask were added 1.0g compound BP103n00 (1.0eq, wherein eq represents the equavilent, the same below), 10ml dichloromethane, 10ml tert-butanol, 0.40g DIC(1.0eq), and 0.39g DMAP (1.0eq, 4-dimethylaminopyridine). They were stirred overnight at room temperature, monitored by TLC (thin-layer chromatography) until the completion of the reaction, diluted with ether, and then washed with water for 3 times, washed with saturated brine, dried over anhydrous sodium sulfate, and chromatographed in a column to give 10.4g BP103n0 as a foamy powder.

### Preparation of BP103n02

To a 100mL three-necked flask were added 0.95 g N-hydroxy succinimide (HOSU), 2.0g compound 19 and 15 ml dichloromethane, into which 1.58g EDC•HCl was added and reacted for 2h at room temperature. After the completion of the reaction under the monitor of TLC, they were diluted with dichloromethane, and then washed with 50mmol/L aqueous solution of potassium dihydrogen phosphate at pH=6.0 for 2 times, washed with saturated brine, dried over anhydrous sodium sulfate, and concentrated to give 2.6g compound BP103n02 as a white solid.

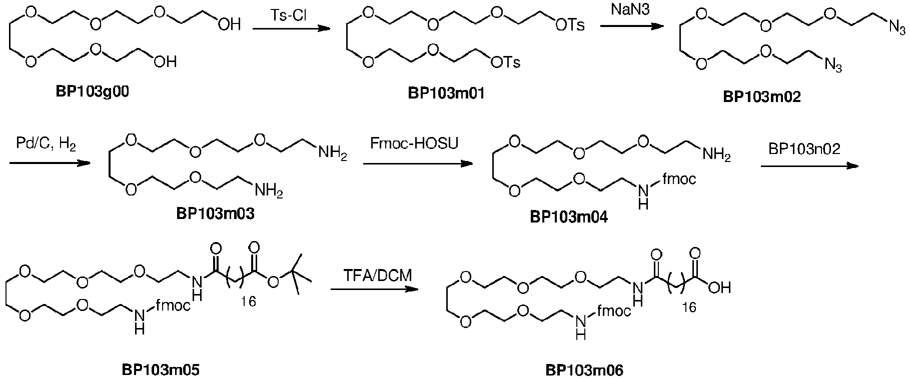

### Preparation of BP103m01

Under the protection of nitrogen, to a 500ml three-necked flask were added 200 mL pyridine, 50 g BP103g00 (1.0eq), stirred and cooled down to 0°C. 70.7g TsCl (2.1eq) was added in batches, stirred for 1h, and then slowly warmed up to room temperature, continuing to stir for 3-4h. After the completion of the reaction, the reaction liquid was poured into the ice-cold solution of diluted hydrochloric acid, extracted with ethyl acetate. The ethyl acetate layer was washed once with diluted hydrochloric acid, washed with saturated sodium bicarbonate and saturated brine, and dried over anhydrous Na₂SO₄. The solvents were evaporated off at reduced pressure, and chromatographed in a silica gel column to give 52g pure BP103m01.

### Preparation of BP103m02

To a 500 mL three-necked flask were added 50g BP103m01 (1.0eq) and 150mL DMSO (dimethyl sulfoxide), and stirred evenly, into which was then added NaN₃ 22.0 g (4.0 eq), heated to 50°C and reacted for 3 hours, cooled down to room temperature. The reaction liquid was poured into water, extracted with ethyl acetate for many times. The organic phases were combined, dried over anhydrous sodium sulfate, and concentrated to give 25.3g BP103m02 as a colourless liquid.

### Preparation of BP103m03

To a 1L hydrogenation reactor were added BP103m03 25g, methanol 200 mL, palladium on carbon 6.0g, stirred, with nitrogen replaced by introducing hydrogen to react for 3-4h. After the completion of the reaction under the monitor of TLC, the reaction liquid was filtered, and the filtrate was concentrated to give 20.4g BP103m03 as an oil.

### Preparation of BP103m04

To a 500mL three-necked flask were added compound BP103m03 20.0g (1.0eq), dichloromethane 200ml, and Fmoc-HOSU 24.0g (1.0 eq), stirred and cooled down to 0°C. 9.2g DIEA (1.0eq, N,N-diisopropyl ethylamine) was added dropwise, and stirred overnight. After the completion of the reaction under the monitor of TLC, it was washed with water and saturated brine, dried over anhydrous sodium sulfate, and then chromatographed in a column to give 27.3g BP103m04 as an oil.

### Preparation of BP103m05

To a 200mL flask were added 5.0g BP103m04 (1.0eq), 50 ml water, 1.7 g NaHCO₃ (2.0eq), and stirred. A solution of 4.7g compound BP103n02(1.0eq) in 50ml DME (ethylene glycol dimethyl ether) was added dropwise, replenished with 50ml THF (tetrahydrofuran), and stirred overnight. After the completion of the reaction under the monitor of TLC, the organic solvents were evaporated off, adjusted to pH=4 with acetic acid, extracted with ethyl acetate, dried over anhydrous sodium sulfate, and concentrated to give 6.4g compound BP103m05 as an off-white solid.

### Preparation of BP103m06

To a 100mL flask were added 6.0 g compound BP103m05, 30 ml dichloromethane, 30ml TFA (trifluoroacetic acid), and stirred at 20°C. After the completion of the reaction under the monitor of TLC, the organic solvents were evaporated off, slurried in petroleum ether, suction filtrated, and dried to give 5.1g BP103m06 as an off-white solid.

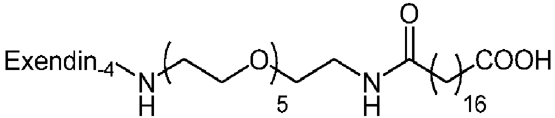

### Synthesis of Target Compound

To a 20ml reaction column were added 1.0g 2Cl-Trt resin, 240mg BP103m06, 5ml dichloromethane, and 300ul DIEA, into which nitrogen was bubbled for 40min. 5ml dichloromethane, 1ml methanol, and 1ml DIEA were added and reacted for 20min, after which they were washed with DMF (N,N-dimethylformamide), producing BP103m06 resin. HOBT/DIC (i.e, 1-hydroxy benzotriazole/N,N-diisopropylcarbodiimide) was used as the coupling reagent, with DMF as the reactive solvent. The reaction was monitored by employing the ninhydrin detection method, successively connecting the following protected amino acids onto the resin: Fmoc-Ser(tBu)-OH, Fmoc-Pro-OH, Fmoc-Pro-OH, Fmoc-Pro-OH, Fmoc-Ala-OH, Fmoc-Gly-OH, Fmoc-Ser(tBu)-OH, Fmoc-Ser(tBu)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg (Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Ser(tBu)-OH, Fmoc-Leu-OH, Fmoc-Asp(OtBu)OH, Fmoc-Ser(tBu)-OH, Fmoc-Thr(tBu)-OH, Fmoc-Phe-OH, Fmoc-Thr(tBu)-OH, Fmoc-Gly-OH, Fmoc-Glu(OtBu)-OH, Fmoc-Gly-OH, Fmoc-His(Trt)-OH, with Fmoc protection finally removed. The pyrolysis of the resin was achieved by employing 82.5% TFA/5% phenol/5% water/2.5% EDT/5% thioanisole, and then they were precipitated with ice-cold methyl tert-butyl ether (MTBE), and washed, and crude products were purified by reverse HPLC to give 32mg pure target peptide.
MS(ESI⁺, m/e):4743.53[M+H]⁺

### Example 2 Preparation of compound 2

Compound 2 was prepared with reference to the method of Example 1:

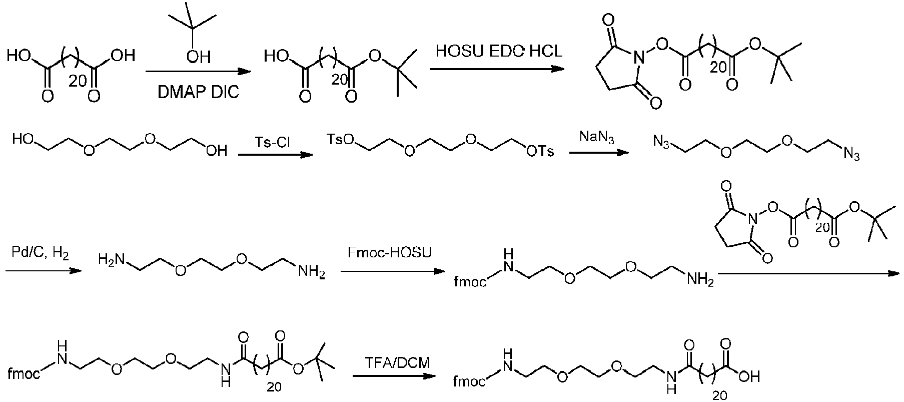

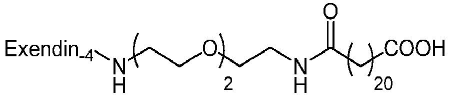

31.2mg pure target peptide was finally obtained.
MS(ESI⁺, m/e): 4667.52[M+H]⁺

### Example 3 Preparation of compound 3

Compound 3 was prepared with reference to the method of Example 1:

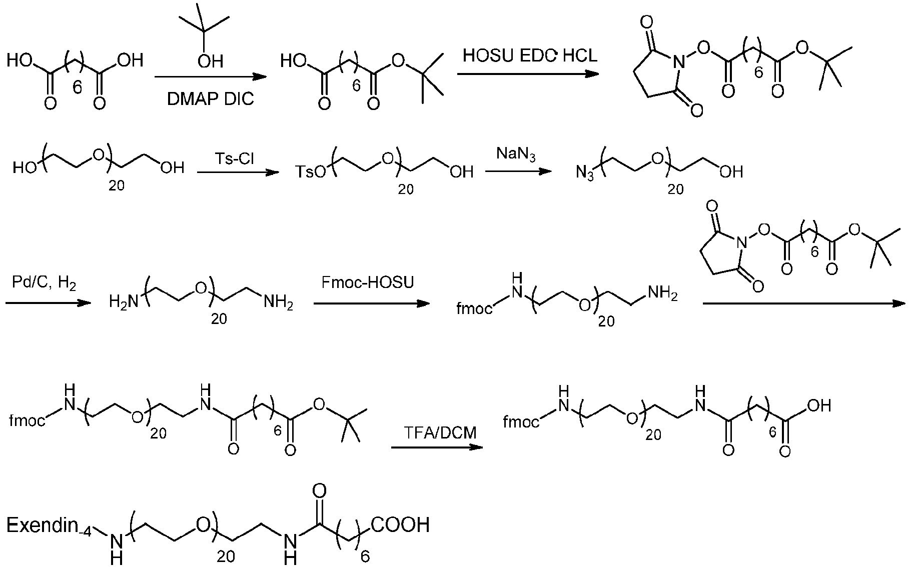

32.3mg pure target peptide was finally obtained.
MS(ESI⁺, m/e): 5263.78[M+H]⁺

### Example 4 Preparation of compound 4

Compound 4 was prepared with reference to the method of Example 1:

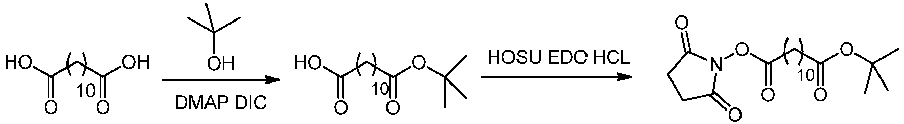

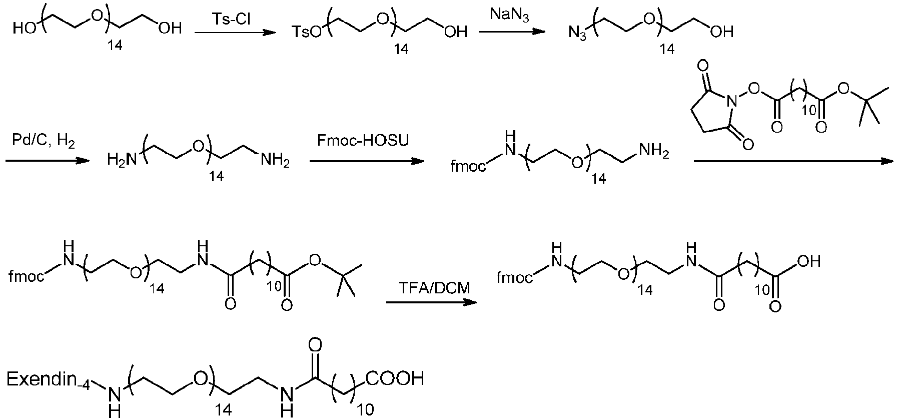

31.8mg pure target peptide was finally obtained.
MS(ESI⁺, m/e): 5055.68[M+H]⁺

### Example 5 Preparation of compound 5

Compound 5 was prepared with reference to the method of Example 1:

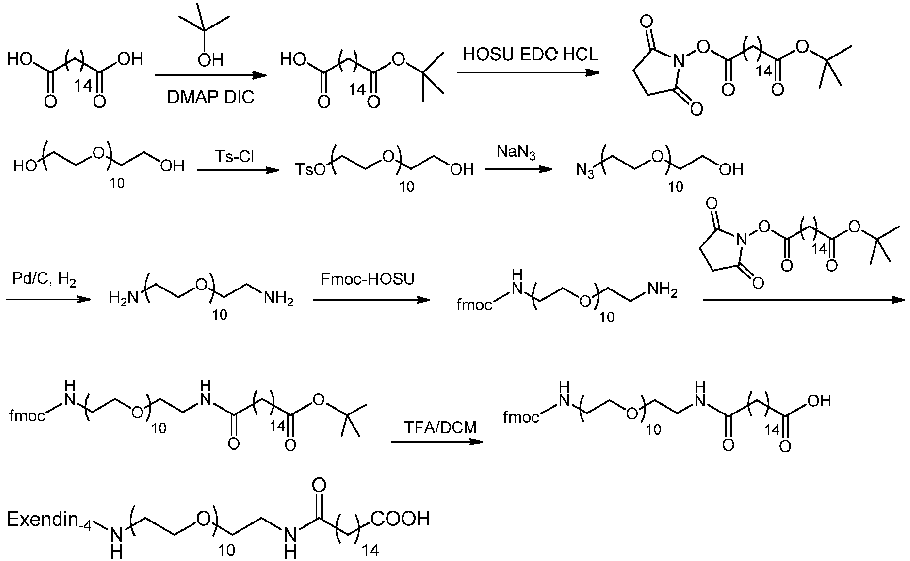

31.0mg pure target peptide was finally obtained.
MS(ESI⁺, m/e): 4935.64[M+H]⁺

### Example 6 Preparation of compound 6

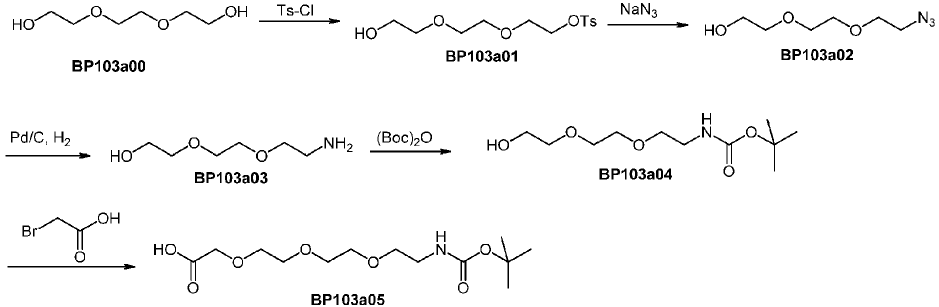

BP103a01 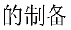

### Preparation of BP103a01

Under the protection of nitrogen, to a 1000ml three-necked flask were added 200 mL pyridine, 120 g BP103a00(1.0eq), stirred and cooled down to 0°C. 151.8g TsCl (1.0eq) was added in batches, stirred for 1h, then slowly warmed up to room temperature, and kept stirring for 3-4h. After the completion of the reaction, the reaction liquid was poured into ice-cold dilute hydrochloric acid solution, extracted with ethyl acetate. The ethyl acetate layer was washed once with dilute hydrochloric acid, washed with saturated sodium bicarbonate, washed with saturated brine, and dried over anhydrous Na₂SO₄. The solvents were evaporated off at reduced pressure, and chromatographed in a silica gel column to give 55g pure BP103a01.

### Preparation of BP103a02

To a 1000 mL three-necked flask were added 55 g BP103a01 (1.0eq) and 160mL DMSO, stirred evenly, in which was then added NaN₃ 23.52 g (2.0 eq), heated to 50°C and reacted for 3 hours, and cooled down to room temperature. The reaction liquid was poured into water, extracted with ethyl acetate. The organic phases were combined, dried over anhydrous sodium sulfate, and concentrated to give 29.2g BP103a02 as a colourless liquid.

### Preparation of BP103a03

To a 1L hydrogenation reactor were added 29g BP103a02, methanol 360 mL, palladium on carbon 5.0g, and stirred. Nitrogen was replaced, and hydrogen was introduced to react for 3-4h. After the completion of the reaction under the monitor of TLC, the reaction liquid was filtered, and the filtrate was concentrated to give 23.5g BP103a03 as an oil.

### Preparation of BP103a04

To a 1 L three-necked flask were added 23.5 g compound BP103a03 (1.0eq), 68.6g (Boc)₂O (2.0 eq), a mixed solution of methanol:triethylamine (9:1) 500ml, stirred and warmed to reflux, and reacted for 1h. After the completion of the reaction under the monitor of TLC, methanol triethylamine was evaporated off, and dissolved with water. Dichloromethane was extracted for 3 times. The organic layers were combined and washed once with water, dried over anhydrous sodium sulfate. The solvents were evaporated off, and dried to give 34.8g BP103a04 as a solid.

### Preparation of BP103a05

To a 1000mL three-necked flask were added 34.8 g compound BP103a04 (1.0eq), toluene and THF 150ml for each, bromoacetic acid 58.2 g (3eq), stirred, heated to 45∼50°C, then added sodium hydroxide 33.5g (6eq), and reacted overnight. After the completion of the reaction under the monitor of TLC, the reaction liquid was evaporated off, extracted with water and ethyl acetate, and the aqueous phase was adjusted to pH 3. The aqueous phase was extracted with dichloromethane, and the dichloromethane layers were combined, dried over anhydrous sodium sulfate, and then concentrated to give 18g BP103a05 compound as an oil.

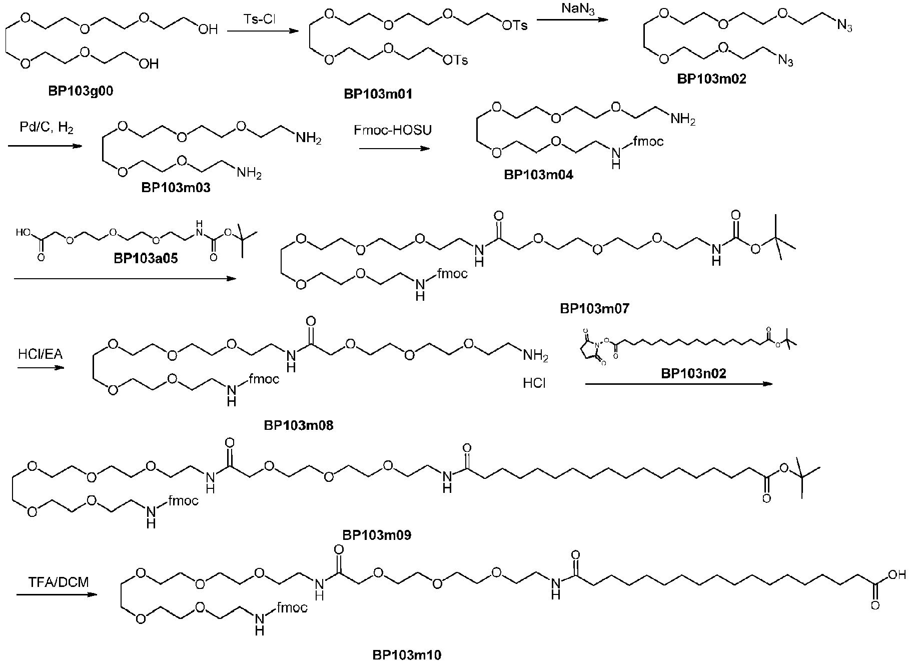

### Preparation of BP103m07

To a 100mL three-necked flask were added compound BP103m04 5.0g (1.05eq), 2.9g BP103a05 (1.0eq), dichloromethane 50ml, DIEA 3.8g (3.0 eq), DEPC 2.4g (1.5 eq, diethyl cyanophosphonate). After the completion of the reaction under the monitor of TLC, the reaction was washed with 0.1mol/L HCl/water, sodium bicarbonate, water, and saturated brine, and dried over anhydrous sodium sulfate, and then chromatographed in a column to give 6.3 g BP103m07 as an oil.

### Preparation of BP103m08

To a 100mL flask were added 6.3g compound BP103m07, 30 ml ethyl acetate, cooled to 0°C and added 7.0mol HCl/ethyl acetate. After the completion of the reaction under the monitor of TLC, the organic solvents were evaporated off, slurried with petroleum ether, suction filtrated, and dried to give 5.3g BP103m08 as an off-white solid.

### Preparation of BP103m09

To a 200mL flask were added 5.0g BP103m08(1.0eq), 50 ml water, 1.2 g NaHCO₃(2.0eq), and stirred. The solution of 3.2g compound BP103n02(1.0eq) in 50ml DME (ethylene glycol dimethyl ether) was added dropwise, replenished with 50ml THF, and stirred overnight. After the completion of the reaction under the monitor of TLC, the organic solvents were evaporated off, adjusted to pH=4 with acetic acid, extracted with ethyl acetate, dried over anhydrous sodium sulfate, and concentrated to give 6.1g compound BP103m09 as an off-white solid.

### Preparation of BP103m10

To a 100mL flask were added 6.0 g compound BP103m09, 30 ml dichloromethane, 30ml TFA, and stirred at 20°C. After the completion of the reaction under the monitor of TLC, the organic solvents were evaporated off, slurried with petroleum ether, suction filtrated, and dried to give 4.8g BP103m10 as an off-white solid.

### Preparation of Target Peptide

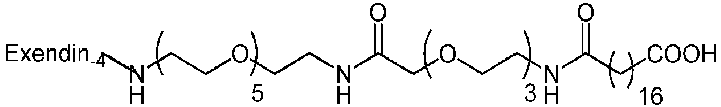

To a 20ml reaction column were added 1.0g 2Cl-Trt resin, 296mg BP103m10, 5ml dichloromethane, and 300ul DIEA, into which nitrogen was bubbled for 40min. 5ml dichloromethane, 1ml methanol, and 1ml DIEA were added and reacted for 20min, after which they were washed with DMF, producing BP103m06 resin. 20% piperidine/DMF was used for the removal of Fmoc, the reaction was kept for 20 minutes, HOBT/DIC was used as the coupling reagent, and the reactive solvent was DMF. The reaction was monitored by employing the ninhydrin detection method, successively connecting the following protected amino acids onto the resin: Fmoc-Ser(tBu)-OH, Fmoc-Pro-OH, Fmoc-Pro-OH, Fmoc-Pro-OH, Fmoc-Ala-OH, Fmoc-Gly-OH, Fmoc-Ser(tBu)-OH, Fmoc-Ser(tBu)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg (Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Ser(tBu)-OH, Fmoc-Leu-OH, Fmoc-Asp(OtBu)OH, Fmoc-Ser(tBu)-OH, Fmoc-Thr(tBu)-OH, Fmoc-Phe-OH, Fmoc-Thr(tBu)-OH, Fmoc-Gly-OH, Fmoc-Glu(OtBu)-OH, Fmoc-Gly-OH, Fmoc-His(Trt)-OH, with Fmoc protection finally removed. The pyrolysis of the resin was achieved by employing 82.5% TFA/5% phenol/5% water/2.5% EDT/5% thioanisole, and then they were precipitated with ice-cold methyl tert-butyl ether (MTBE), and washed and crude products were purified by reverse HPLC to give 39mg pure target peptide.
MS(ESI⁺, m/e): 4932.65[M+H]⁺

### Example 7 Preparation of compound 7

Compound 7 was prepared with reference to the method of Example 6:

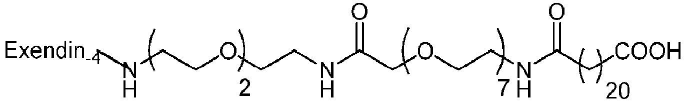

39.6mg pure target peptide was finally obtained.
MS(ESI⁺, m/e): 5032.76[M+H]⁺

### Example 8 Preparation of compound 8

Compound 8 was prepared with reference to the method of Example 6:

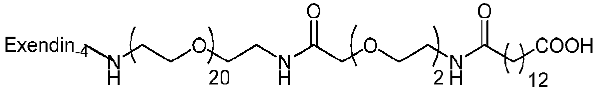

40.2mg pure target peptide was finally obtained.
MS(ESI⁺, m/e): 5492.99[M+H]⁺

### Example 9 Preparation of compound 9

Compound 9 was prepared with reference to the method of Example 6:

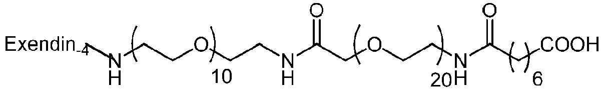

40.7mg pure target peptide was finally obtained.
MS(ESI⁺, m/e): 5761.11[M+H]⁺

### Example 10 Preparation of compound 10

Compound 10 was prepared with reference to the method of Example 6:

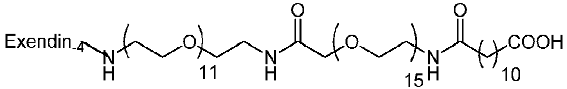

40.7mg pure target peptide was finally obtained.
MS(ESI⁺, m/e): 5641.07[M+H]⁺

### Example 11 Preparation of compound 11

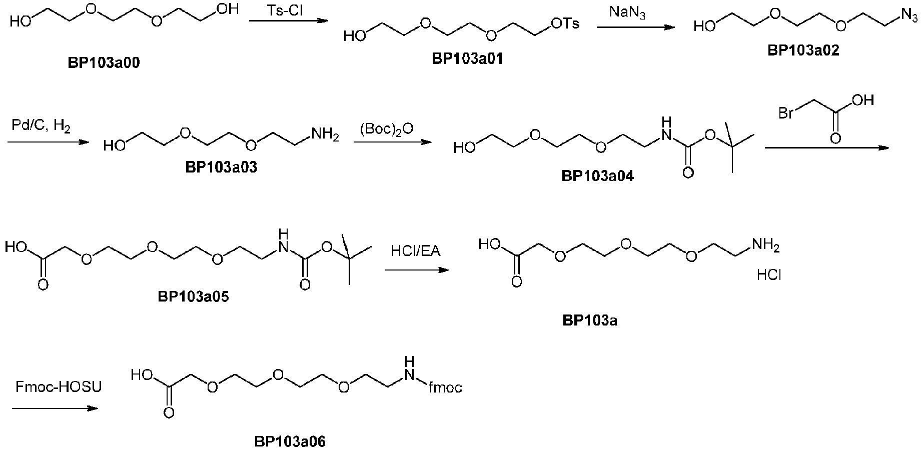

### Preparation of BP103a

To a 250mL three-necked flask were added 18g compound BP103a05, 100ml ethyl acetate, stirred to be dissolved and then cooled down to 0°C, with the addition of 150 ml ethyl acetate/HCl (3.5M), keeping the temperature at 0°C. After the completion of the reaction under the monitor of TLC, they was filtered, and the filter cake was washed with TBME to give 10.4g BP103a as a white solid.

### Preparation of BP103a06

To a 200mL flask were added 5.0g BP103a (1.0eq), 50 ml water, 3.5 g NaHCO₃ (2.0eq), and stirred. A solution of 7.3 gFmoc-HOSU (1.0eq) in 50ml DME (ethylene glycol dimethyl ether) was added dropwise, it was replenished with 50ml THF, and stirred overnight. After the completion of the reaction under the monitor of TLC, the organic solvents were evaporated off, adjusted to pH=2 with dilute hydrochloric acid, extracted with ethyl acetate, washed with water and saturated brine, dried over anhydrous sodium sulfate, and concentrated to give 7.6g compound BP103a06 as an off-white solid.

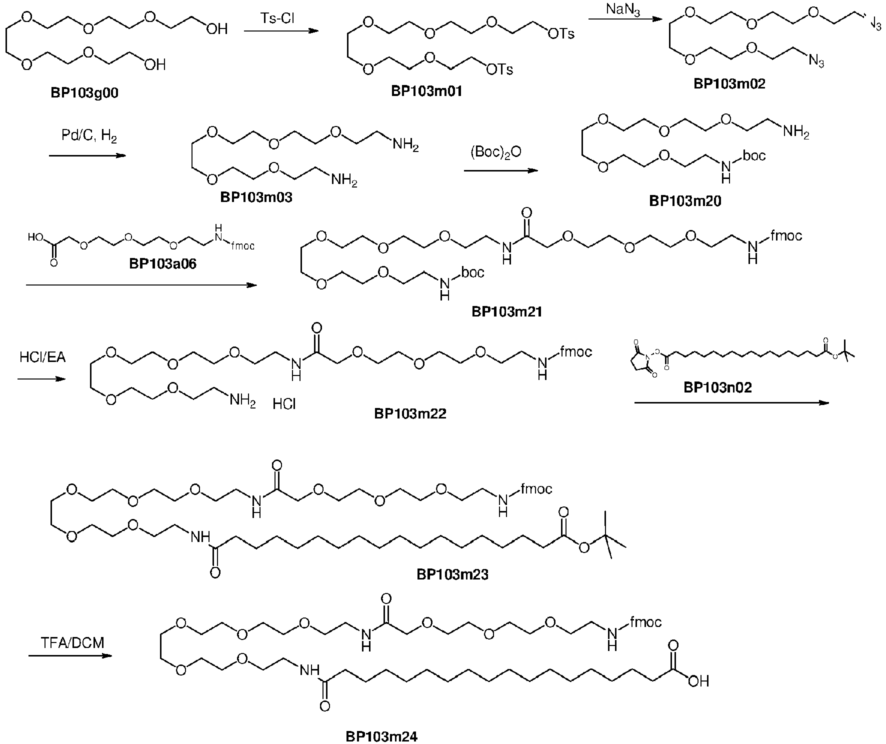

### Preparation of BP103m20

To a 500mL three-necked flask were added compound BP103m03 10.0g (1.0eq), dichloromethane 100ml, (Boc)₂O 7.8g (1.0 eq), stirred and cooled down to 0°C. 4.6g DIEA (1.0eq) was added dropwise, and stirred overnight. After the completion of the reaction under the monitor of TLC, they were washed with water and saturated brine, dried over anhydrous sodium sulfate, and then chromatographed in a column to give 6.2g BP103m20 as an oil.

### Preparation of BP103m21

To a 100mL three-necked flask were added compound BP103m20 6.2g (1.05eq), 6.7g BP103a06 (1.0eq), dichloromethane 50ml, DIEA 6.3g (3.0 eq), DEPC 4.0g (1.5 eq). After the completion of the reaction under the monitor of TLC, they were washed with 0.1mol/L HCl/water, sodium bicarbonate, water, and saturated brine, dried over anhydrous sodium sulfate, and then chromatographed in a column to give 9.5 g BP103m21 as an oil.

### Preparation of BP103m22

To a 100mL flask were added 9.5 g compound BP103m21, 50 ml ethyl acetate, stirred and cooled down to 0°C, with the addition of 50ml 7.0mol/L HCl/ethyl acetate. After the completion of the reaction under the monitor of TLC, the organic solvents were evaporated off, slurried with petroleum ether, suction filtrated, and dried to give 8.3g BP103m22 as an off-white solid.

### Preparation of BP103m23

To a 200mL flask were added 5.0g BP103m22(1.0eq), 50 ml water, 1.2 g NaHCO₃ (2.0eq), stirred. A solution of 3.2g compound BP103n02 (1.0eq) in 50ml DME (ethylene glycol dimethyl ether) was added dropwise, replenished with 50ml THF, and stirred overnight. After the completion of the reaction under the monitor of TLC, the organic solvents were evaporated off, adjusted to pH=4 with acetic acid, extracted with ethyl acetate, dried over anhydrous sodium sulfate, and concentrated to give 5.6g compound BP103m23 as an off-white solid.

### Preparation of BP103m24

To a 100mL flask were added 5.6 g compound BP103m23, 30 ml dichloromethane, 30mlTFA, and stirred at 20°C. After the completion of the reaction under the monitor of TLC, the organic solvents were evaporated off, slurried with petroleum ether, suction filtrated, and dried to give 4.4g BP103m24 as an off-white solid.

### Preparation of Target Peptide

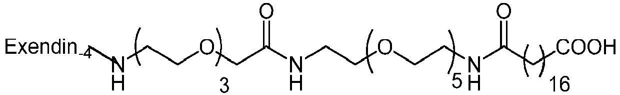

To a 20ml reaction column were added 1.0g 2Cl-Trt resin, 296mg BP103m24, 5ml dichloromethane, and 300ul DIEA, into which nitrogen was bubbled for 40min. 5ml dichloromethane, 1ml methanol, 1ml DIEA were added and reacted for 20min, after which they were washed with DMF, producing BP103m06 resin. 20% piperidine/DMF was used for the removal of Fmoc, the reaction was kept for 20 minutes, HOBT/DIC was used as the coupling reagent, and the reactive solvent was DMF. The reaction was monitored by employing the ninhydrin detection method, successively connecting the following protected amino acids onto the resin: Fmoc-Ser(tBu)-OH, Fmoc-Pro-OH, Fmoc-Pro-OH, Fmoc-Pro-OH, Fmoc-Ala-OH, Fmoc-Gly-OH, Fmoc-Ser(tBu)-OH, Fmoc-Ser(tBu)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Ser(tBu)-OH, Fmoc-Leu-OH, Fmoc-Asp(OtBu)OH, Fmoc-Ser(tBu)-OH, Fmoc-Thr(tBu)-OH, Fmoc-Phe-OH, Fmoc-Thr(tBu)-OH, Fmoc-Gly-OH, Fmoc-Glu(OtBu)-OH, Fmoc-Gly-OH, Fmoc-His(Trt)-OH, with Fmoc protection finally removed. The pyrolysis of the resin was achieved by employing 82.5% TFA/5% phenol/5% water/2.5% EDT/5% thioanisole, and then they were precipitated with ice-cold methyl tert-butyl ether (MTBE), and washed, and crude products were purified by reverse HPLC to give 29mg pure target peptide.
MS(ESI⁺, m/e): 4932.65[M+H]⁺

### Example 12 Preparation of compound 12

Compound 12 was prepared with reference to the method of Example 11.

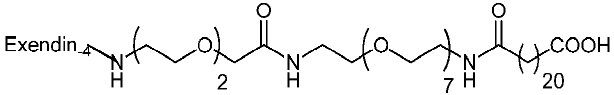

29.1mg pure target peptide was finally obtained.
MS(ESI⁺, m/e): 5032.76[M+H]⁺

### Example 13 Preparation of compound 13

Compound 13 was prepared with reference to the method of Example 11:

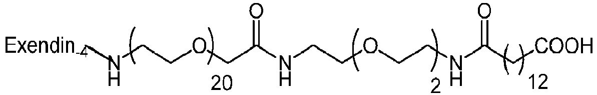

30mg pure target peptide was finally obtained.
MS(ESI⁺, m/e): 5492.99[M+H]⁺

### Example 14 Preparation of compound 14

Compound 14 was prepared with reference to the method of Example 11:

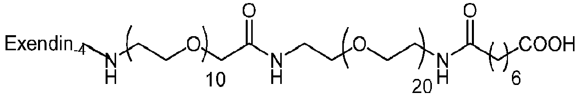

30.2mg pure target peptide was finally obtained.
MS(ESI⁺, m/e): 5761.11[M+H]⁺

### Example 15 Preparation of compound 15

Compound 15 was prepared with reference to the method of Example 11:

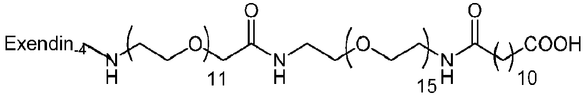

30.4mg pure target peptide was finally obtained.
MS(ESI⁺, m/e): 5641.07[M+H]⁺

### Example 16 Preparation of compound 16

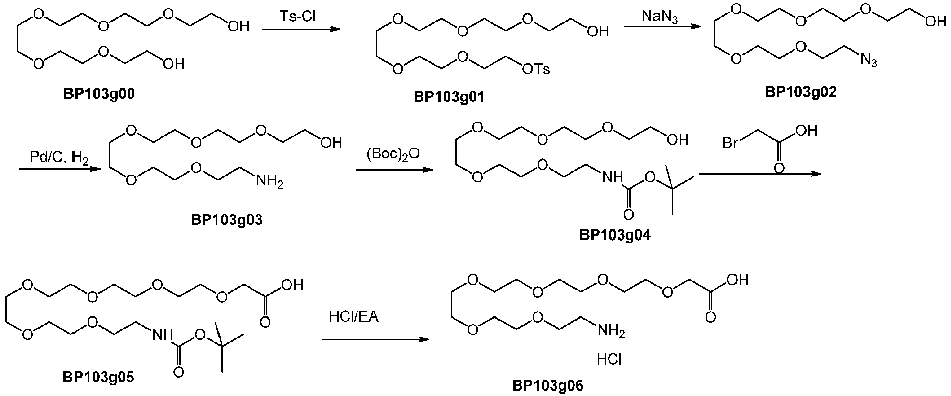

### Preparation of compound BP103g01

Under the protection of nitrogen, to a 500ml three-necked flask were added 200 mL pyridine, 50 g BP103g00 (1.0eq), stirred and cooled down to 0°C. 35.5g TsCl (1.0eq) was added in batches, stirred for 1h, and then slowly warmed up to room temperature, continuing to stir for 3-4h. After the completion of the reaction, the reaction liquid was poured into the ice-cold solution of dilute hydrochloric acid, extracted with ethyl acetate. The ethyl acetate layer was washed once with dilute hydrochloric acid, washed with saturated sodium bicarbonate and saturated brine, and dried over anhydrous Na₂SO₄. The solvents were evaporated off at reduced pressure, and chromatographed in a silica gel column to give 38g pure BP103g01.

### Preparation of compound BP103g02

To a 500 mL three-necked flask were added 38g BP103g01 (1.0eq) and 190mL DMSO, stirred evenly, then added NaN₃ 11.5 g (2.0 eq), heated to 50°C and reacted for 3 hours, cooled down to room temperature. The reaction liquid was poured into water, extracted with ethyl acetate for many times. The organic phases were combined, dried over anhydrous sodium sulfate, and concentrated to give 40g BP103g02 as a colourless liquid.

### Preparation of compound BP103g03

To a 1L hydrogenation reactor were added BP103g02 70g, methanol 500 mL, palladium on carbon 8.0g, stirred, with nitrogen replaced by introducing hydrogen to react for 3-4h. After the completion of the reaction under the monitor of TLC, the reaction liquid was filtered, and the filtrate was concentrated to give 52g BP103g03 as an oil.

### Preparation of compound BP103g04

To a 250mL three-necked flask were added compound BP103g03 10.0g (1.0eq), (Boc)₂O 15.5g (2.0 eq), a mixed solution of methanol:triethylamine (9:1) 200ml, stirred and warmed to reflux, and reacted for 1h. After the completion of the reaction under the monitor of TLC, methanol triethylamine was evaporated off, and dissolved with water. Dichloromethane was extracted for 3 times. The organic layers were combined and washed once with water, dried over anhydrous sodium sulfate, and concentrated to give 9.0g BP103g04 as an oil.

### Preparation of compound BP103g05

To a 250mL three-necked flask were added BP103g04 compound 7.0 g (1.0eq), toluene and THF 40ml for each, bromoacetic acid 7.6 g (3.0eq), stirred, heated to 45∼50°C, then added sodium hydroxide 4.4g, and reacted overnight. After the completion of the reaction under the monitor of TLC, the reaction liquid was evaporated off. The impurities were extracted with water and ethyl acetate, and the aqueous phase was adjusted to pH=3. The aqueous phase was extracted with dichloromethane, and the dichloromethane layers were combined, dried over anhydrous sodium sulfate, and then concentrated to give 4.2g BP103g05 compound as an oil.

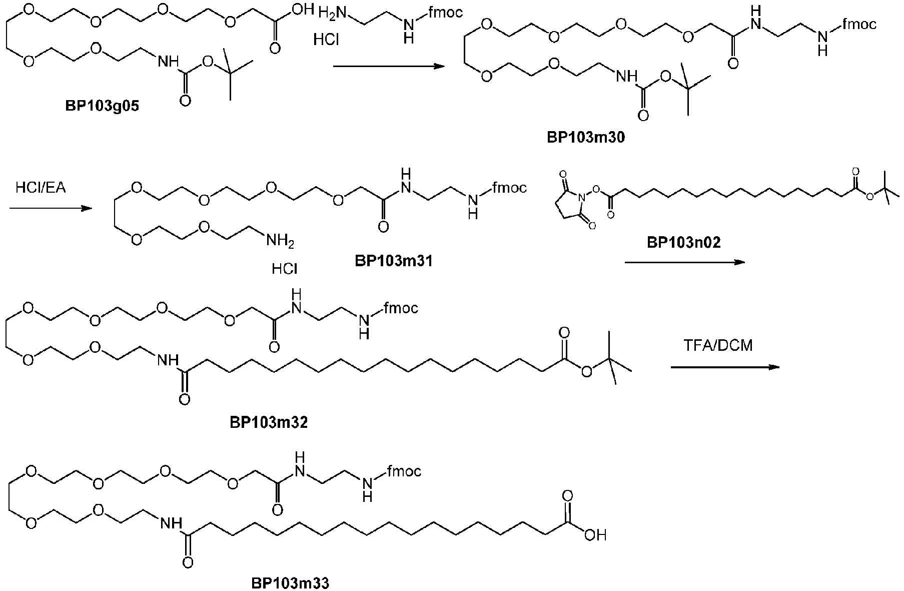

### Preparation of BP103m30

To a 100mL three-necked flask were added compound BP103g05 4.2g (1.05eq), 2.9g Fmoc-ethylenediamine hydrochloride (1. 0eq), dichloromethane 50ml, DIEA 3.7g (3.0 eq), DEPC 2.3g (1.5 eq). After the completion of the reaction under the monitor of TLC, they were washed with 0.1mol/L HCl/water, sodium bicarbonate, water, and saturated brine, dried over anhydrous sodium sulfate, and then chromatographed in a column to give 5.6 g BP103m21 as an oil.

### Preparation of BP103m31

To a 100mL flask were added 5.6g compound BP103m30, 30 ml ethyl acetate, stirred and cooled down to 0°C, with the addition of 30ml 7.0mol/L HCl/ethyl acetate. After the completion of the reaction under the monitor of TLC, the organic solvents were evaporated off, slurried with petroleum ether, suction filtrated, and dried to give 4.8g BP103m31 as an off-white solid.

### Preparation of BP103m32

To a 200mL flask were added 4.6g BP103m31 (1.0eq), 45 ml water, 1.2 g NaHCO₃ (2.0eq), and stirred. A solution of 3.4g compound BP103n02 (1.0eq) in 45ml DME (ethylene glycol dimethyl ether) was added dropwise, replenished with 45ml THF, and stirred overnight. After the completion of the reaction under the monitor of TLC, the organic solvents were evaporated off, adjusted to pH=4 with acetic acid, extracted with ethyl acetate, dried over anhydrous sodium sulfate, and concentrated to give 4.9g compound BP103m32 as an off-white solid.

### Preparation of BP103m33

To a 100mL flask were added 4.5g compound BP103m32, 25 ml dichloromethane, 25ml TFA, and stirred at 20°C. After the completion of the reaction under the monitor of TLC, the organic solvents were evaporated off, slurried with petroleum ether, suction filtrated, and dried to give 3.8g BP103m33 as an off-white solid.

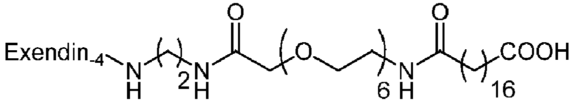

To a 20ml reaction column were added 1.0g 2Cl-Trt resin, 270mg BP103m33, 5ml dichloromethane, 300ul DIEA, into which nitrogen was bubbled for 40min. 5ml dichloromethane, 1ml methanol, 1ml DIEA were added and reacted for 20min, after which they were washed with DMF, producing BP103m06 resin. 20% piperidine/DMF was used for the removal of Fmoc, the reaction was kept for 20 minutes, HOBT/DIC was used as the coupling reagent, and the reactive solvent was DMF. The reaction was monitored by employing the ninhydrin detection method, successively connecting the following protected amino acids onto the resin: Fmoc-Ser(tBu)-OH, Fmoc-Pro-OH, Fmoc-Pro-OH, Fmoc-Pro-OH, Fmoc-Ala-OH, Fmoc-Gly-OH, Fmoc-Ser(tBu)-OH, Fmoc-Ser(tBu)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg (Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Ser(tBu)-OH, Fmoc-Leu-OH, Fmoc-Asp(OtBu)OH, Fmoc-Ser(tBu)-OH, Fmoc-Thr(tBu)-OH, Fmoc-Phe-OH, Fmoc-Thr(tBu)-OH, Fmoc-Gly-OH, Fmoc-Glu(OtBu)-OH, Fmoc-Gly-OH, Fmoc-His(Trt)-OH, with Fmoc protection finally removed. The pyrolysis of the resin was achieved by employing 82.5% TFA/5% phenol/5% water/2.5% EDT/5% thioanisole, and then they were precipitated with ice-cold methyl tert-butyl ether (MTBE), and washed, and crude products were purified by reverse HPLC to give 33mg pure target peptide.
MS(ESI⁺, m/e): 4844.6[M+H]⁺

### Example 17 Preparation of compound 17

Compound 17 was prepared with reference to the method of Example 16.

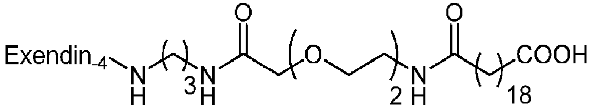

32mg pure target peptide was finally obtained.
MS(ESI⁺, m/e): 4710.54[M+H]⁺

### Example 18 Preparation of compound 18

Compound 18 was prepared with reference to the method of Example 16.

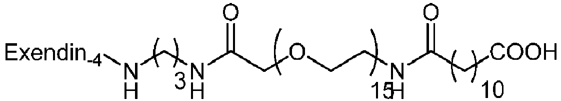

33.5mg pure target peptide was finally obtained.
MS(ESI⁺, m/e): 5170.77[M+H]⁺

### Example 19 Preparation of compound 19

Compound 19 was prepared with reference to the method of Example 16.

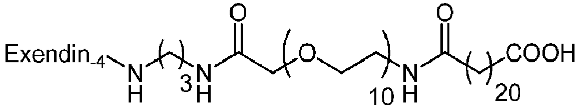

33.1mg pure target peptide was finally obtained.
MS(ESI⁺, m/e): 5090.82 [M+H]⁺

### Example 20 Preparation of compound 20

Compound 20 was prepared with reference to the method of Example 16.

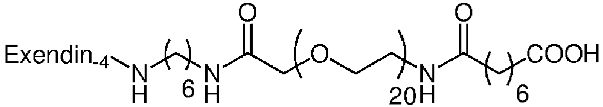

33.1mg pure target peptide was finally obtained.
MS(ESI⁺, m/e): 5090.82 [M+H]⁺

### Example 21 Preparation of compound 21

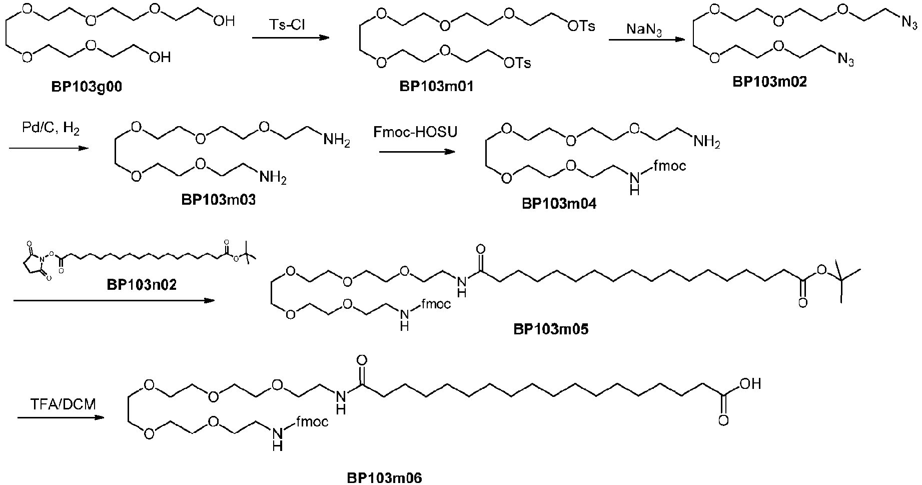

Preparation method of BP103m06 was found in Example 6.

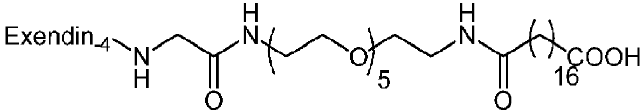

To a 20ml reaction column were added 1.0g 2Cl-Trt resin, 240mg BP103m06, 5ml dichloromethane, 300ul DIEA, into which nitrogen was bubbled for 40min. 5ml dichloromethane, 1ml methanol, 1ml DIEA were added and reacted for 20min, after which they were washed with DMF, producing BP103m06 resin. 20% piperidine/DMF was used for the removal of Fmoc, the reaction was kept for 20 minutes, HOBT/DIC was used as the coupling reagent, and the reactive solvent was DMF. The reaction was monitored by employing the ninhydrin detection method, successively connecting the following protected amino acids onto the resin: Fmoc-Gly-OH, Fmoc-Ser(tBu)-OH, Fmoc-Pro-OH, Fmoc-Pro-OH, Fmoc-Pro-OH, Fmoc-Ala-OH, Fmoc-Gly-OH, Fmoc-Ser(tBu)-OH, Fmoc-Ser(tBu)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg (Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Ser(tBu)-OH, Fmoc-Leu-OH, Fmoc-Asp(OtBu)OH, Fmoc-Ser(tBu)-OH, Fmoc-Thr(tBu)-OH, Fmoc-Phe-OH, Fmoc-Thr(tBu)-OH, Fmoc-Gly-OH, Fmoc-Glu(OtBu)-OH, Fmoc-Gly-OH, Fmoc-His(Trt)-OH, with Fmoc protection finally removed. The pyrolysis of the resin was achieved by employing 82.5% TFA/5% phenol/5% water/2.5% EDT/5% thioanisole, and then they were precipitated with ice-cold methyl tert-butyl ether (MTBE), and washed, and crude products were purified by reverse HPLC to give 42mg pure target peptide.
MS(ESI⁺, m/e): 4800.56 [M+H]⁺

### Example 22 Preparation of compound 22

Compound 22 was prepared with reference to the method of Example 21.

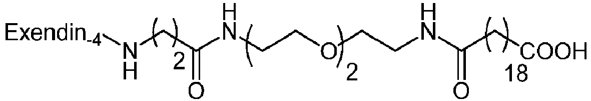

41.7mg pure target peptide was finally obtained.
MS(ESI⁺, m/e): 4710.53 [M+H]⁺

### Example 23 Preparation of compound 23

Compound 23 was prepared with reference to the method of Example 21.

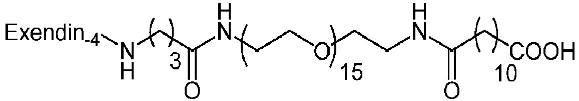

42.5mg pure target peptide was finally obtained.
MS(ESI⁺, m/e): 5184.78[M+H]⁺

### Example 24 Preparation of compound 24

Compound 24 was prepared with reference to the method of Example 21.

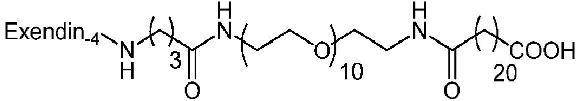

42.1mg pure target peptide was finally obtained.
MS(ESI⁺, m/e): 5104.83[M+H]⁺

### Example 25 Preparation of compound 25

Compound 25 was prepared with reference to the method of Example 21.

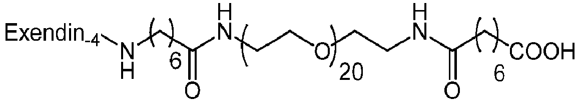

43mg pure target peptide was finally obtained.
MS(ESI⁺, m/e): 5390.91[M+H]⁺

### Example 26

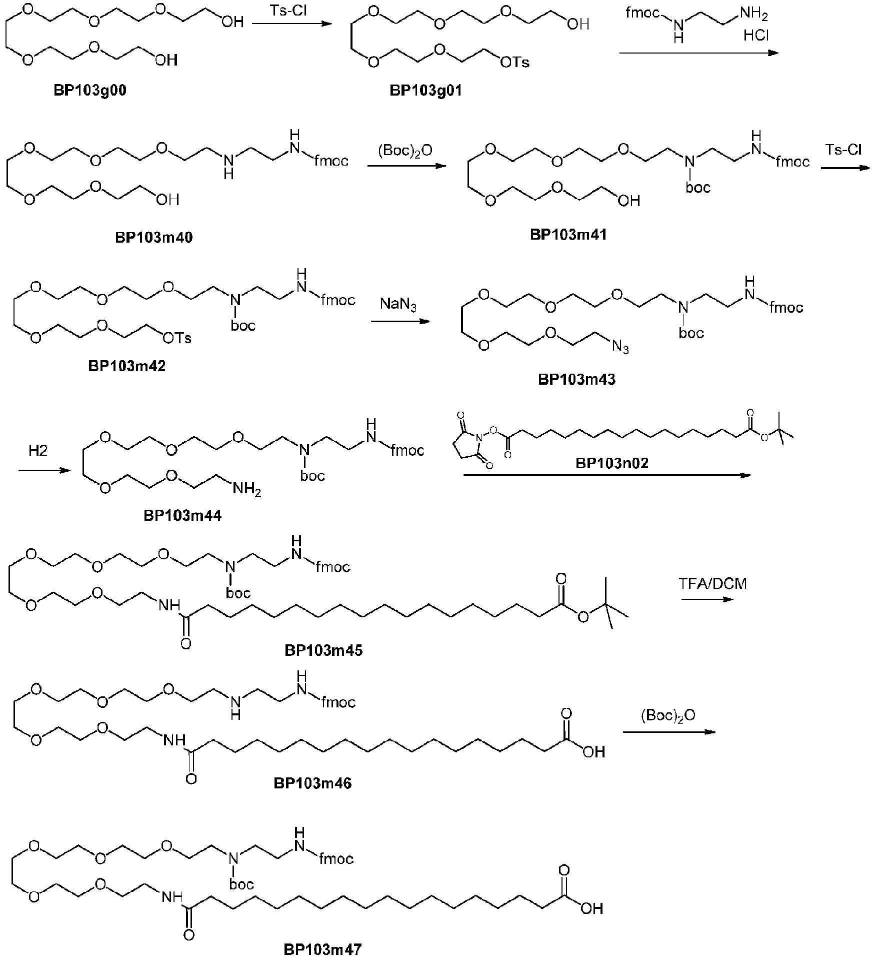

### Preparation of BP103m40

To a 500mL three-necked flask were added compound BP103g01 25.0g (1.0eq), acetonitrile 250ml, Fmoc-ethylenediamine hydrochloride 18.3g (1.0 eq), stirred and cooled down to 0°C, with the addition of 7.9g potassium carbonate (1.0eq). After the completion of the reaction under the monitor of TLC, they were washed with dilute hydrochloric acid and saturated brine, dried over anhydrous sodium sulfate, and then chromatographed in a column to give 25.3g BP103m40 as an oil.

### Preparation of BP103m41

To a 500mL three-necked flask were added compound BP103m40 25.0g (1.0eq), dichloromethane 250ml, (Boc)₂O 19.9g (2.0 eq), into which 17.7g DIEA (3.0eq) was added dropwise. After the completion of the reaction under the monitor of TLC, they were washed with dilute hydrochloric acid, aqueous sodium bicarbonate solution and then saturated brine, dried over anhydrous sodium sulfate, and then chromatographed in a column to give 23.8g BP103m41 as an oil.

### Preparation of BP103m42

Under the protection of nitrogen, to a 1000ml three-necked flask were added 100 mL pyridine, 23.5 g BP103m41 (1.0eq), stirred and cooled down to 0°C. 8.3g TsCl (1.2eq) was added in batches, stirred for 1h, and then slowly warmed up to room temperature, continuing to stir for 3-4h. After the completion of the reaction, most of pyridine was evaporated off, dissolved in ethyl acetate, washed once with dilute hydrochloric acid, washed with saturated sodium bicarbonate and saturated brine, dried over anhydrous Na₂SO₄, and chromatographed in a silica gel column to give 25.3g pure BP103m42.

### Preparation of BP103m43

To a 1000 mL three-necked flask were added 25.0 g BP103m42 (1.0eq) and 100mL DMSO, stirred evenly, then added NaN₃ 4.1 g (2.0 eq), heated to 50°C and reacted for 3 hours, cooled down to room temperature. The reaction liquid was poured into water, extracted with ethyl acetate, The organic phases were combined, dried over anhydrous sodium sulfate, and concentrated to give 22.7g BP103m43 as a colourless liquid.

### Preparation of BP103m44

To a 1L hydrogenation reactor were added 22.7g compound BP103m43, methanol 250 mL, palladium on carbon 5.0g, stirred, with nitrogen replaced by introducing hydrogen to react for 3-4h. After the completion of the reaction under the monitor of TLC, the reaction liquid was filtered, and the filtrate was concentrated to give 19.6g BP103m44 as an oil.

### Preparation of BP103m45

To a 500mL flask were added 10.0g BP103m44 (1.0eq), 100 ml water, 2.6g NaHCO₃(2.0eq), and stirred. A solution of 7.2g compound BP103n02 (1.0eq) in 100ml DME (ethylene glycol dimethyl ether) was added dropwise, replenished with 100ml THF, and stirred overnight. After the completion of the reaction under the monitor of TLC, the organic solvents were evaporated off, adjusted to pH=4 with acetic acid, extracted with ethyl acetate, dried over anhydrous sodium sulfate, and concentrated to give 13.2g compound BP103m45 as an off-white solid.

### Preparation of BP103m46

To a 100mL flask were added 13.2 g compound BP103m45, 15 ml dichloromethane, 15ml TFA, and stirred at 20°C. After the completion of the reaction under the monitor of TLC, the organic solvents were evaporated off, slurried with petroleum ether, suction filtrated, and dried to give 10.5g BP103m46 as an off-white solid.

### Preparation of BP103m47

To a 250ml three-necked flask were added 10.5 g compound BP103m36 (1.0eq), 110ml dichloromethane, 5.4g (Boc)₂O (2.0 eq), into which 4.8g DIEA (3.0eq) was added dropwise. After the completion of the reaction under the monitor of TLC, they were washed with dilute hydrochloric acid, aqueous sodium bicarbonate solution, and then saturated brine, dried over anhydrous sodium sulfate, and chromatographed in a column to give 7.9g BP103m47 as an off-white solid.

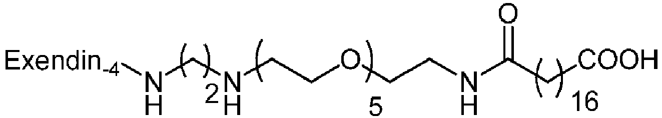

To a 20ml reaction column were added 1.0g 2Cl-Trt resin, 283mg BP103m47, 5ml dichloromethane, and 300ul DIEA, into which nitrogen was bubbled for 40min. 5ml dichloromethane, 1ml methanol, and 1ml DIEA were added and reacted for 20min, after which they were washed with DMF, producing BP103m06 resin. 20% piperidine/DMF was used for the removal of Fmoc, the reaction was kept for 20 minutes, HOBT/DIC was used as the coupling reagent, and the reactive solvent was DMF. The reaction was monitored by employing the ninhydrin detection method, successively connecting the following protected amino acids onto the resin: Fmoc-Ser(tBu)-OH, Fmoc-Pro-OH, Fmoc-Pro-OH, Fmoc-Pro-OH, Fmoc-Ala-OH, Fmoc-Gly-OH, Fmoc-Ser(tBu)-OH, Fmoc-Ser(tBu)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg (Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Ser(tBu)-OH, Fmoc-Leu-OH, Fmoc-Asp(OtBu)OH, Fmoc-Ser(tBu)-OH, Fmoc-Thr(tBu)-OH, Fmoc-Phe-OH, Fmoc-Thr(tBu)-OH, Fmoc-Gly-OH, Fmoc-Glu(OtBu)-OH, Fmoc-Gly-OH, Fmoc-His(Trt)-OH, with Fmoc protection finally removed. The pyrolysis of the resin was achieved by employing 82.5% TFA/5% phenol/5% water/2.5% EDT/5% thioanisole, and then they were precipitated with ice-cold methyl tert-butyl ether (MTBE), and washed, and crude products were purified by reverse HPLC to give 41mg pure target peptide.
MS(ESI⁺, m/e): 4771.57[M+H]⁺

### Example 27 Preparation of compound 27

Compound 27 was prepared with reference to the method of Example 26.

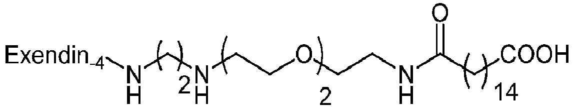

40.5mg pure target peptide was finally obtained.
MS(ESI⁺, m/e): 4611.44[M+H]⁺

### Example 28 Preparation of compound 28

Compound 28 was prepared with reference to the method of Example 26.

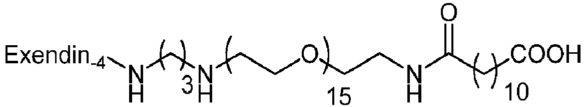

41.6mg pure target peptide was finally obtained.
MS(ESI⁺, m/e): 5141.77[M+H]⁺

### Example 29 Preparation of compound 29

Compound 29 was prepared with reference to the method of Example 26.

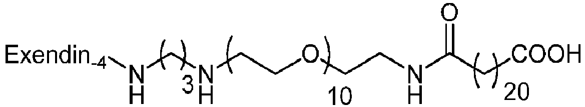

41.2mg pure target peptide was finally obtained.
MS(ESI⁺, m/e): 5061.82[M+H]⁺

### Example 30 Preparation of compound 30

Compound 30 was prepared with reference to the method of Example 26.

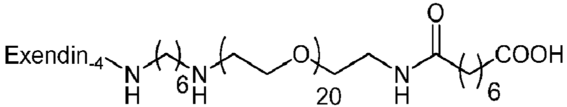

42.3mg pure target peptide was finally obtained.
MS(ESI⁺, m/e): 5347.9[M+H]⁺

### Example 31 Preparation of compound 31

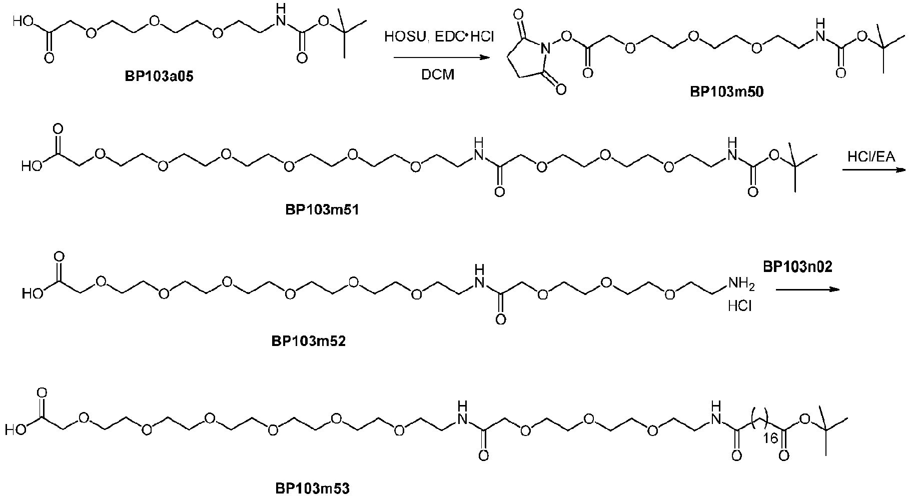

### Preparation of BP103m50

To a 100mL three-necked flask were added 286mg N-hydroxy succinimide (HOSU), 0.50g BP103a05 and 5 ml dichloromethane, into which 477mg EDC·HCl was added and reacted at room temperature for 2h. After the completion of the reaction under the monitor of TLC, they were diluted with dichloromethane, and then washed with 50mmol/L aqueous solution of potassium dihydrogen phosphate at pH=6.0 for 2 times, washed with saturated brine, dried over anhydrous sodium sulfate, and concentrated to give 0.72g compound BP103m50 as an oil.

### Preparation of BP103m51

To a 100mL flask were added 0.62g compound BP103g06(1.0eq), 10 ml water, 0.27 g NaHCO₃ (2.0eq), and stirred. A solution of 0.66 g compound BP103m50 in 10ml DME (ethylene glycol dimethyl ether) was added dropwise, replenished with 5ml THF, and stirred overnight. After the completion of the reaction under the monitor of TLC, the organic solvents were evaporated off, adjusted to pH=4 with dilute hydrochloric acid, extracted with dichloromethane, dried over anhydrous sodium sulfate, and concentrated to give 0.71g compound BP103m51 as an oil.

### Preparation of BP103m52

To a 100mL flask were added 0.71g compound BP103m51 and 5ml ethyl acetate, after being dissolved, they were cooled down to 0°C, into which 5 ml HCl/ethyl acetate(7mol/L) was added, keeping the temperature at 0°C. After the completion of the reaction under the monitor of TLC, they were concentrated to give 0.71g BP103m52 as an oil.

### Preparation of BP103m53

To a 100mL flask were added 640mg compound BP103m52(1.0eq), 15 ml water, 190mg NaHCO₃ (2.0eq), and stirred. A solution of 528mg compound BP103n02 in 15ml DME (ethylene glycol dimethyl ether) was added dropwise, replenished with 15mlTHF, and stirred overnight. After the completion of the reaction under the monitor of TLC, the organic solvents were evaporated off, adjusted to pH=6 with acetic acid, extracted with dichloromethane, dried over anhydrous sodium sulfate, and concentrated to give 0.65g compound BP103m53 as an oil.

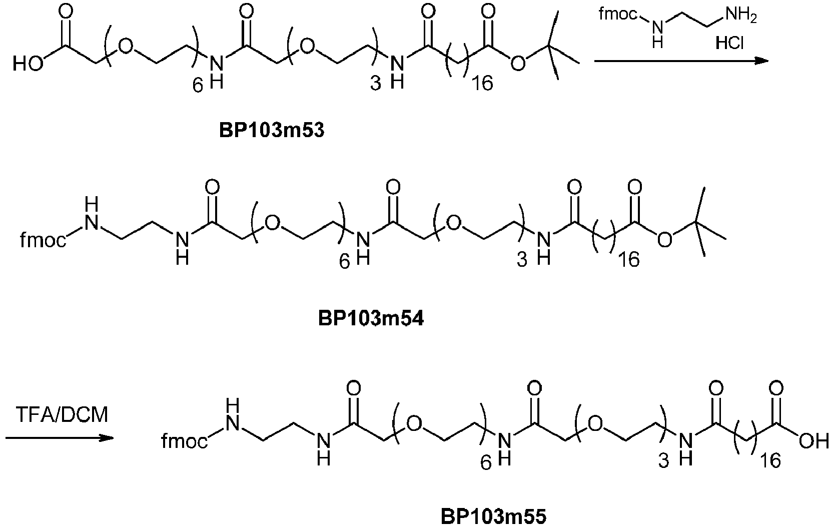

### Preparation of BP103m54

To a 100mL three-necked flask were added compound BP103m53 3.0g (1. 0eq), 1.1g Fmoc-ethylenediamine hydrochloride (1.05eq), dichloromethane 30ml, DIEA 1.3g (3.0 eq), and DEPC 0.8g (1.5 eq). After the completion of the reaction under the monitor of TLC, they were washed with 0.1mol/L HCl/water, sodium bicarbonate, water, and saturated brine, dried over anhydrous sodium sulfate, and then chromatographed in a column to give 2.9g BP103m54 as an oil.

### Preparation of BP103m55

To a 100mL flask were added 2.9g compound BP103m54, 15 ml dichloromethane, 15ml TFA, and stirred at 20°C. After the completion of the reaction under the monitor of TLC, the organic solvents were evaporated off, slurried with petroleum ether, suction filtrated, and dried to give 2.5g BP103m55 as an off-white solid.

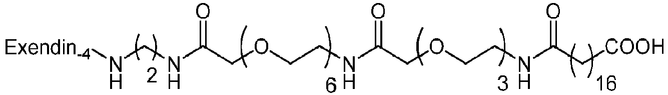

To a 20ml reaction column were added 1.0g 2Cl-Trt resin, 344mg BP103m55, 5ml dichloromethane, and 300ul DIEA, into which nitrogen was bubbled for 40min. 5ml dichloromethane, 1ml methanol, and 1ml DIEA were added and reacted for 20min, after which they were washed with DMF, producing BP103m06 resin. 20% piperidine/DMF was used for the removal of Fmoc, the reaction was kept for 20 minutes, HOBT/DIC was used as the coupling reagent, and the reactive solvent was DMF. The reaction was monitored by employing the ninhydrin detection method, successively connecting the following protected amino acids onto the resin: Fmoc-Ser(tBu)-OH, Fmoc-Pro-OH, Fmoc-Pro-OH, Fmoc-Pro-OH, Fmoc-Ala-OH, Fmoc-Gly-OH, Fmoc-Ser(tBu)-OH, Fmoc-Ser(tBu)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg (Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Ser(tBu)-OH, Fmoc-Leu-OH, Fmoc-Asp(OtBu)OH, Fmoc-Ser(tBu)-OH, Fmoc-Thr(tBu)-OH, Fmoc-Phe-OH, Fmoc-Thr(tBu)-OH, Fmoc-Gly-OH, Fmoc-Glu(OtBu)-OH, Fmoc-Gly-OH, Fmoc-His(Trt)-OH, with Fmoc protection finally removed. The pyrolysis of the resin was achieved by employing 82.5% TFA/5% phenol/5% water/2.5% EDT/5% thioanisole, and then they were precipitated with ice-cold methyl tert-butyl ether (MTBE), and washed, and crude products were purified by reverse HPLC to give 46mg pure target peptide.
MS(ESI⁺, m/e): 5033.72[M+H]⁺

### Example 32 Preparation of compound 32

Compound 32 was prepared with reference to the method of Example 31.

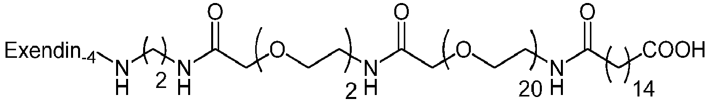

46.8mg pure target peptide was finally obtained.
MS(ESI⁺, m/e): 5578.07[M+H]⁺

### Example 33 Preparation of compound 33

Compound 33 was prepared with reference to the method of Example 31.

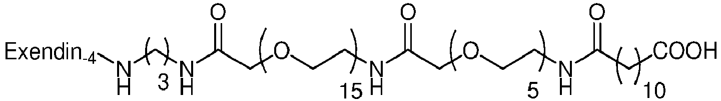

46.4mg pure target peptide was finally obtained.
MS(ESI⁺, m/e): 5447.95[M+H]⁺

### Example 34 Preparation of compound 34

Compound 34 was prepared with reference to the method of Example 31.

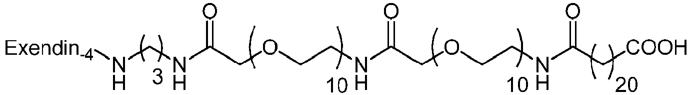

46.5mg pure target peptide was finally obtained.
MS(ESI⁺, m/e): 5588.15 [M+H]⁺

### Example 35 Preparation of compound 35

Compound 35 was prepared with reference to the method of Example 31.

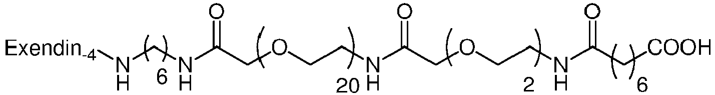

46mg pure target peptide was finally obtained.
MS(ESI⁺, m/e): 5521.99 [M+H]⁺

### Example 36 Preparation of compound 36

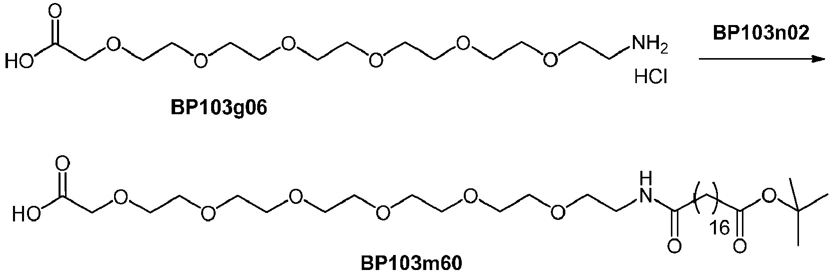

### Preparation of BP103m60

To a 500mL flask were added 10.0g BP103g06(1.0eq), 100 ml water, 4.5g NaHCO₃ (2.0eq), and stirred. A solution of 12.4g compound BP103n02(1.0eq) in 100ml DME (ethylene glycol dimethyl ether) was added dropwise, replenished with 100ml THF, and stirred overnight. After the completion of the reaction under the monitor of TLC, the organic solvents were evaporated off, adjusted to pH=4 with acetic acid, extracted with ethyl acetate, dried over anhydrous sodium sulfate, and concentrated to give 15.2g compound BP103m60 as an off-white solid.

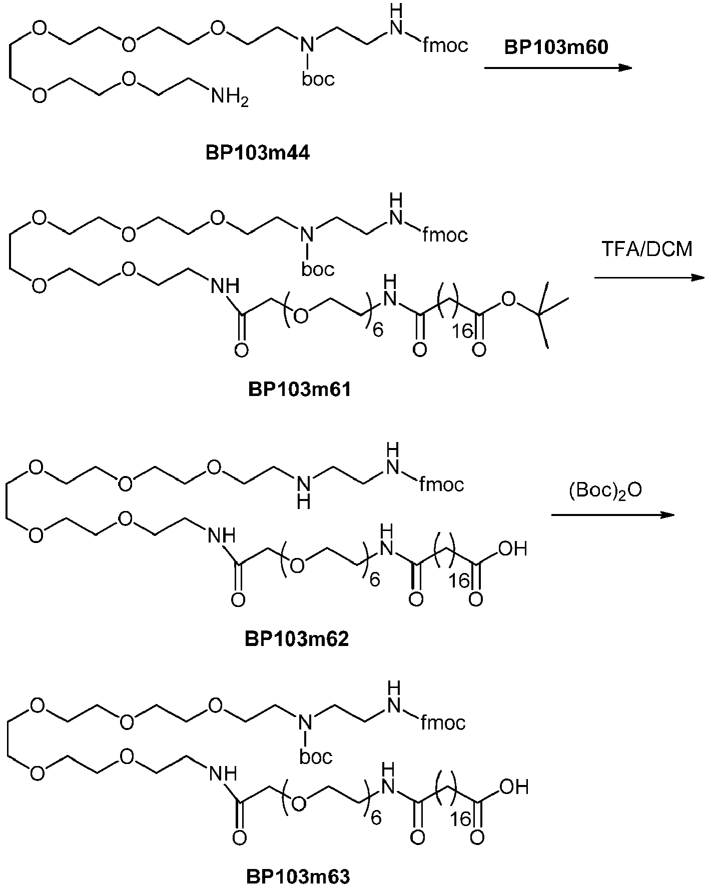

### Preparation of BP103m61

To a 100mL three-necked flask were added compound BP103m44 3.0g (1.05eq), 3.1g (1.0eq) BP103m60, dichloromethane 30ml, DIEA 1.7g (3.0 eq), DEPC 1.1g (1.5 eq). After the completion of the reaction under the monitor of TLC, they were washed with 0.1mol/L HCl/water, sodium bicarbonate, water, and saturated brine, dried over anhydrous sodium sulfate, and then chromatographed in a column to give 4.1g BP103m61 as an oil.

### Preparation of BP103m62

To a 100mL flask were added 4.1g compound BP103m61, 20 ml dichloromethane, 20ml TFA, and stirred at 20°C. After the completion of the reaction under the monitor of TLC, the organic solvents were evaporated off, slurried with petroleum ether, suction filtrated, and dried to give 3.5g BP103m62 as an off-white solid.

### Preparation of BP103m63

To a 250ml three-necked flask were added 3.5 g compound BP103m62 (1.0eq), 50ml dichloromethane, and 1.2g (Boc)₂O (2.0 eq), into which 1.1g DIEA (3.0eq) was added dropwise. After the completion of the reaction under the monitor of TLC, they were washed with dilute hydrochloric acid, aqueous sodium bicarbonate solution, and then saturated brine, dried over anhydrous sodium sulfate, and chromatographed in a column to give 2.6g BP103m63 as an off-white solid.

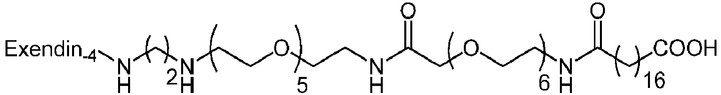

To a 20ml reaction column were added 1.0g 2Cl-Trt resin, 408mg BP103m63, 5ml dichloromethane, 300ul DIEA, into which nitrogen was bubbled for 40min. 5ml dichloromethane, 1ml methanol, and 1ml DIEA were added and reacted for 20min, after which they were washed with DMF, producing BP103m06 resin. 20% piperidine/DMF was used for the removal of Fmoc, the reaction was kept for 20 minutes, HOBT/DIC was used as the coupling reagent, and the reactive solvent was DMF. The reaction was monitored by employing the ninhydrin detection method, successively connecting the following protected amino acids onto the resin: Fmoc-Ser(tBu)-OH, Fmoc-Pro-OH, Fmoc-Pro-OH, Fmoc-Pro-OH, Fmoc-Ala-OH, Fmoc-Gly-OH, Fmoc-Ser(tBu)-OH, Fmoc-Ser(tBu)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg (Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Ser(tBu)-OH, Fmoc-Leu-OH, Fmoc-Asp(OtBu)OH, Fmoc-Ser(tBu)-OH, Fmoc-Thr(tBu)-OH, Fmoc-Phe-OH, Fmoc-Thr(tBu)-OH, Fmoc-Gly-OH, Fmoc-Glu(OtBu)-OH, Fmoc-Gly-OH, Fmoc-His(Trt)-OH, with Fmoc protection finally removed. The pyrolysis of the resin was achieved by employing 82.5% TFA/5% phenol/5% water/2.5% EDT/5% thioanisole, and then they were precipitated with ice-cold methyl tert-butyl ether (MTBE), and washed, and crude products were purified by reverse HPLC to give 55mg pure target peptide.
MS(ESI⁺, m/e): 5107.81[M+H]⁺

### Example 37 Preparation of compound 37

Compound 37 was prepared with reference to the method of Example 36.

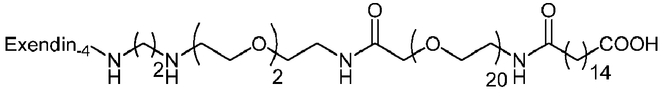

55.7mg pure target peptide was finally obtained.
MS(ESI⁺, m/e): 5564.1 [M+H]⁺

### Example 38 Preparation of Compound 38

Compound 38 was prepared with reference to the method of Example 36.

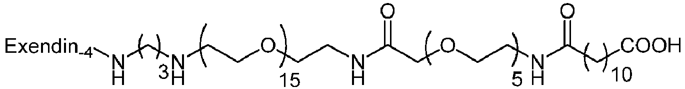

55mg pure target peptide was finally obtained.
MS(ESI⁺, m/e): 5433.98 [M+H]⁺

### Example 39 Preparation of compound 39

Compound 39 was prepared with reference to the method of Example 36.

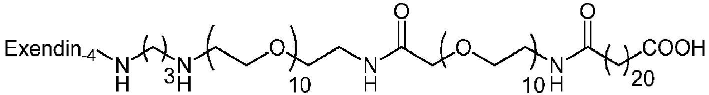

55.2mg pure target peptide was finally obtained.
MS(ESI⁺, m/e): 5574.18 [M+H]⁺

### Example 40 Preparation of compound 40

Compound 40 was prepared with reference to the method of Example 36.

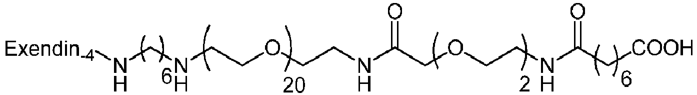

55.3mg pure target peptide was finally obtained.
MS(ESI⁺, m/e): 5508.02[M+H]⁺

### Example 41 Preparation of compound 41

### Preparation of Exendin-4(1-39)-Lys40(Alloc)-NH₂

The solid phase peptides of target peptides were synthesized by empolying the solid phase synthesis of Fmoc process, using Fmoc-Rink MBHA Amide resin, in which 20% piperidine/DMF was used to remove Fmoc, HOBT/DIC was used as the coupling reagent, and the reactive solvent was DMF. The reaction was monitored by employing the ninhydrin detection method, successively connecting the following protected amino acids onto the Rink MBHA Amide resin: Fmoc-Lys(Alloc)-OH, Fmoc-Ser(tBu)-OH, Fmoc-Pro-OH, Fmoc-Pro-OH, Fmoc-Pro-OH, Fmoc-Ala-OH, Fmoc-Gly-OH, Fmoc-Ser(tBu)-OH, Fmoc-Ser(tBu)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg (Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Ser(tBu)-OH, Fmoc-Leu-OH, Fmoc-Asp(OtBu)OH, Fmoc-Ser(tBu)-OH, Fmoc-Thr(tBu)-OH, Fmoc-Phe-OH, Fmoc-Thr(tBu)-OH, Fmoc-Gly-OH, Fmoc-Glu(OtBu)-OH, Fmoc-Gly-OH, Fmoc-His(Trt)-OH, (BOC)₂O (using DIEA, dichloromethane). They were washed with DMF, methanol, and dichloromethane, and then dried to give 12.1g Exendin-4(1-39)-Lys40(Alloc)-NH₂ resin.

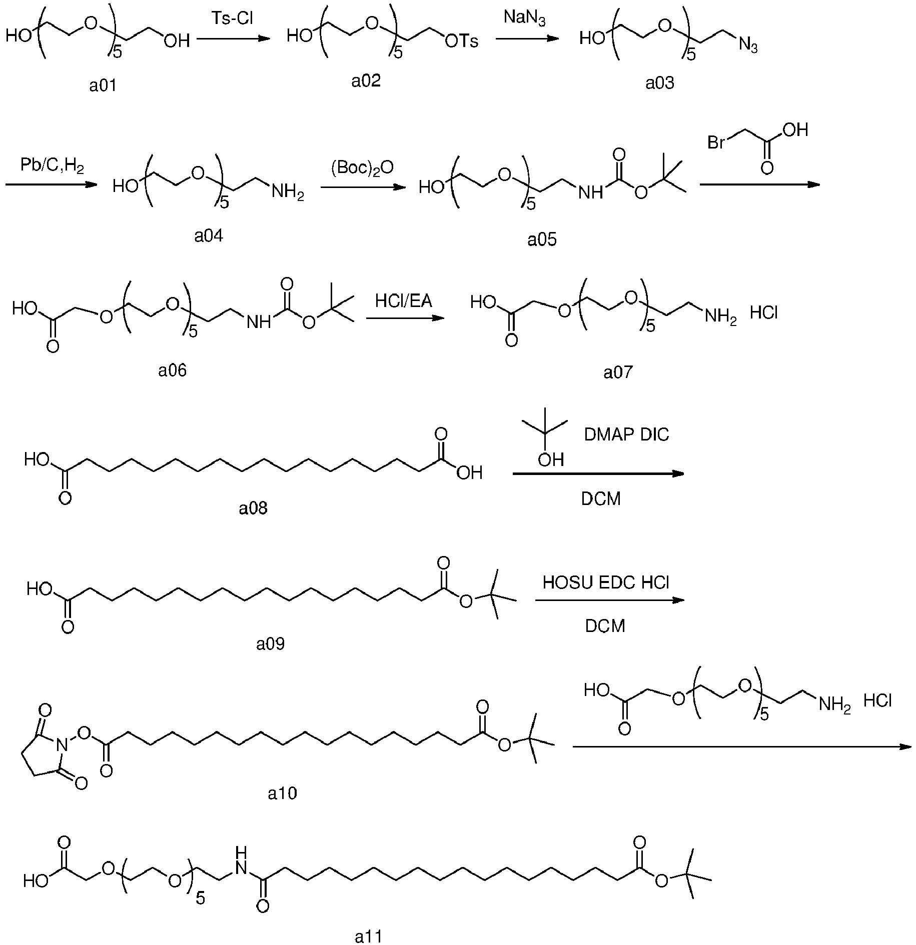

### Preparation of compound a02

Under the protection of nitrogen, to a 500ml three-necked flask were added 200 mL pyridine, 50 g a01 (1.0eq), stirred and cooled down to 0°C, into which 35.5g TsCl(1.0eq) was added in batches, stirred for 1h, and then slowly warmed up to room temperature, continuing to stir for 3-4h. After the completion of the reaction, the reaction liquid was poured into the ice-cold solution of dilute hydrochloric acid, with a solid being generated, which was extracted with ethyl acetate. The ethyl acetate layer was washed once with dilute hydrochloric acid, washed with saturated sodium bicarbonate and saturated brine, and dried over anhydrous Na₂SO₄. The solvents were evaporated off at reduced pressure, and chromatographed in a silica gel column to give 38g pure a02.

### Preparation of compound a03

To a 500 mL three-necked flask were added 38g a (1.0eq) and 190mL DMSO, stirred evenly, then added NaN₃ 11.5 g (2.0 eq), heated to 50°C and reacted for 3 hours, cooled down to room temperature. The reaction liquid was poured into water, extracted with ethyl acetate for many times. The organic phases were combined, dried over anhydrous sodium sulfate, and concentrated to give 40g a03 as a colourless liquid.

### Preparation of compound a04

To a 1L hydrogenation reactor were added a03 70g, methanol 500 mL, palladium on carbon 8.0g, and stirred, with nitrogen replaced by introducing hydrogen to react for 3-4h. After the completion of the reaction under the monitor of TLC, the reaction liquid was filtered, and the filtrate was concentrated to give 52g a04 as an oil.

### Preparation of compound a05

To a 250mL three-necked flask were added compound a04 10.0g (1.0eq), (Boc)₂O 15.5g (2.0 eq), a mixed solution of methanol:triethylamine (9:1) 200ml, stirred, warmed to reflux, and reacted for 1h. After the completion of the reaction under the monitor of TLC, methanol triethylamine was evaporated off, and they were dissolved in water, and extracted with dichloromethane for 3 times. The organic layers were combined and washed once with water, dried over anhydrous sodium sulfate, and concentrated to give 9.0g a05 as an oil.

### Preparation of compound a06

To a 250mL three-necked flask were added a05 compound 7.0 g (1.0eq), toluene and THF 40ml for each, bromoacetic acid 7.6 g (3.0eq), stirred, and heated to 45∼50°C, into which 4.4g sodium hydroxide was then added, and reacted overnight. After the completion of the reaction under the monitor of TLC, the reaction liquid was evaporated off, the impurities were extracted with water and ethyl acetate, and the aqueous phase was adjusted to pH=3. The aqueous phase was extracted with dichloromethane, and the dichloromethane layers were combined, dried over anhydrous sodium sulfate, and then concentrated to give 4.2g a06 compound as an oil.

### Preparation of compound a07

To a 250mL single-neck flask were added compound a06 4.0g and 20ml ethyl acetate, after being dissolved, they were cooled down to 0°C, into which was added 20 ml HCl/ethyl acetate (7mol/L). After the completion of the reaction under the monitor of TLC, they were concentrated to give 4.2g a07 as an oil.

### Preparation of compound a09

To a 50mL three-necked flask were added 1.0g compound a08(1.0eq), 10ml dichloromethane, 10ml tert-butanol, 0.40g DIC(1.0eq), 0.39g DMAP(1.0eq), and stirred overnight at room temperature. After the completion of the reaction under the monitor of TLC, they were diluted with ether, then washed with water for 3 times and washed with saturated brine, dried over anhydrous sodium sulfate, and chromatographed in a column to give 0.4g a09 as a foamy powder.

### Preparation of compound a10

To a 100mL three-necked flask were added 0.95 g N-hydroxy succinimide (HOSU), 2.0g compound a09 and 15 ml dichloromethane, into which 1.58g EDC·HCl was added and reacted for 2h at room temperature. After the completion of the reaction under the monitor of TLC, they were diluted with dichloromethane, then washed with 50mmol/L aqueous solution of potassium dihydrogen phosphate at pH=6.0 for 2 times, and washed with saturated brine, dried over anhydrous sodium sulfate, and concentrated to give 2.6g compound a10 as an oil.

### Preparation of compound a11

To a 100mL flask were added 1.28g compound a07(1.0eq), 20 ml water, 1.16g NaHCO₃ (4.0eq), and stirred. A solution of 1.75g compound a10 in 20ml DME(ethylene glycol dimethyl ether) was added dropwise, replenished with 20ml THF, and stirred overnight. After the completion of the reaction under the monitor of TLC, the organic solvents were evaporated off, adjusted to pH=6 with acetic acid, extracted with ethyl acetate, dried over anhydrous sodium sulfate, and concentrated to give an off-white solid, which was chromatographed in a column to give 0.95g compound a11.

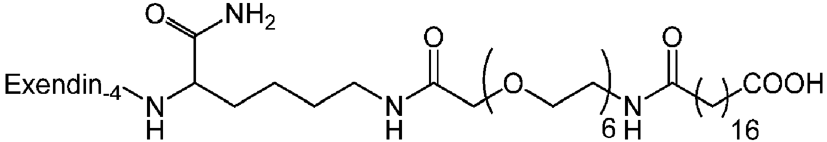

1.5g Exendin-4(1-39)-Lys40-NH₂ resin was swelled in DMF, into which was then added 3eq solution of Pd(PPh₃)₄ in CHCl₃:AcOH:NMM (18:1:0.5). They were reacted for 2h, then washed with chloroform (6 times, 20ml chloroform for each time), washed with 20% solution of HOAc in dichloromethane (6 times, 20ml 20% solution of HOAc in dichloromethane for each time), washed with dichloromethane (6 times, 20ml dichloromethane for each time) and washed with DMF (6 times, 20ml DMF for each time). When it was detected with ninhydrin as positive, 5ml DMF, 415mg compound a11, 150mg HOAT, and 150ul DIC were added and reacted for 4h; and when it was detected with ninhydrin as negative, indicting that the side chain a11 has connected onto Exendin-4(1-39)-Lys40-NH₂ resin. The pyrolysis of the resin was carried out by employing 82.5% TFA/5% phenol/5% water/2.5% EDT/5% thioanisole, and then they were precipitated with ice-cold methyl tert-butyl ether (MTBE), and washed. Crude products were purified by HPLC to give 43mg target compound.
MS(ESI⁺, m/e): 4932.56 [M+H]⁺

### Example 42 Preparation of compound 42

Compound 42 was prepared with reference to the method of Example 41.

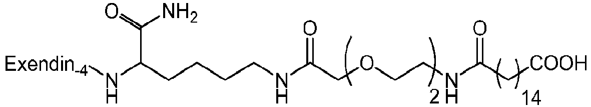

41mg pure target peptide was finally obtained.
MS(ESI⁺, m/e): 4725.38[M+H]⁺

### Example 43 Preparation of compound 43

Compound 43 was prepared with reference to the method of Example 41.

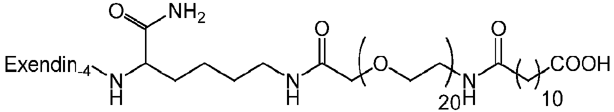

41.5mg pure target peptide was finally obtained.
MS(ESI⁺, m/e): 5461.93[M+H]⁺

### Example 44 Preparation of compound 44

Compound 44 was prepared with reference to the method of Example 41.

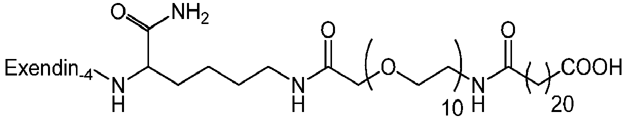

43mg pure target peptide was finally obtained.
MS(ESI⁺, m/e): 5161.83[M+H]⁺

### Example 45 Preparation of compound 45

Compound 45 was prepared with reference to the method of Example 41.

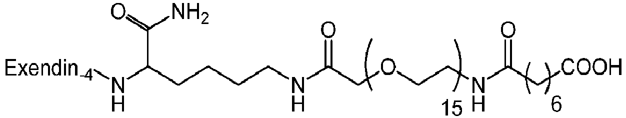

42mg pure target peptide was finally obtained.
MS(ESI⁺, m/e): 5185.7[M+H]⁺

### Example 46 Preparation of compound 46

### Preparation of Exendin-4(1-39)-Orn40(Alloc)-NH₂ resin

Taking 5g Fmoc-Rink MBHA Amide resin, 20% piperidine/DMF was used for the removal of Fmoc, HOBT/DIC was used as the coupling reagent, and the reactive solvent was DMF. The reaction was monitored by employing the ninhydrin detection method, successively connecting the following protected amino acids onto Rink MBHA Amide resin: Fmoc-Orn(Alloc)-OH, Fmoc-Ser(tBu)-OH, Fmoc-Pro-OH, Fmoc-Pro-OH, Fmoc-Pro-OH, Fmoc-Ala-OH, Fmoc-Gly-OH, Fmoc-Ser(tBu)-OH, Fmoc-Ser(tBu)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg (Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Ser(tBu)-OH, Fmoc-Leu-OH, Fmoc-Asp(OtBu)OH, Fmoc-Ser(tBu)-OH, Fmoc-Thr(tBu)-OH, Fmoc-Phe-OH, Fmoc-Thr(tBu)-OH, Fmoc-Gly-OH, Fmoc-Glu(OtBu)-OH, Fmoc-Gly-OH, Fmoc-His(Trt)-OH, (Boc)₂O (using DIEA, dichloromethane). They were washed with DMF, washed with methanol, washed with dichloromethane, and then dried to give 7.8g Exendin-4(1-39)-Orn40(Alloc)-NH₂ resin.

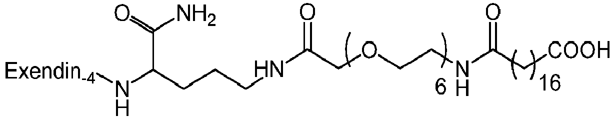

1.5g Exendin-4(1-39)-Orn40(Alloc)-NH₂ resin was swelled in DMF, into which was then added 3eq solution of Pd(PPh₃)₄ in CHCl₃:AcOH:NMM (18:1:0.5). They were reacted for 2h, then washed with chloroform (6 times, 20ml chloroform for each time), washed with 20% solution of HOAc in dichloromethane (6 times, 20ml 20% solution of HOAc in dichloromethane for each time), washed with dichloromethane (6 times, 20ml dichloromethane for each time) and washed with DMF (6 times, 20ml DMF for each time). When it was detected with ninhydrin as positive, 5ml DMF, 415mg compound BP103m60, 150mg HOAT, and 150ul DIC were added and reacted for 4h; and when it was detected with ninhydrin as negative, indicting that the side chain BP103m60 has connected onto Exendin-4(1-39)-Orn40-NH₂ resin. The pyrolysis of the resin was carried out by employing 82.5% TFA/5% phenol/5% water/2.5% EDT/5% thioanisole, and then they were precipitated with ice-cold methyl tert-butyl ether (MTBE), and washed. Crude products were purified by HPLC to give 41mg target compound.
MS(ESI⁺, m/e): 4915.61[M+H]⁺

### Example 47 Preparation of compound 47

Compound 47 was prepared with reference to the method of Example 46.

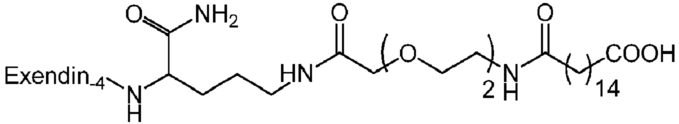

42mg pure target peptide was finally obtained.
MS(ESI⁺, m/e): 4711.45[M+H]⁺

### Example 48 Preparation of compound 48

Compound 48 was prepared with reference to the method of Example 46.

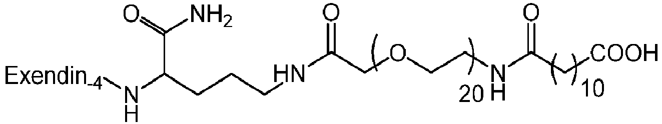

43.4mg pure target peptide was finally obtained.
MS(ESI⁺, m/e): 5447.91 [M+H]⁺

### Example 49 Preparation of compound 49

Compound 49 was prepared with reference to the method of Example 46.

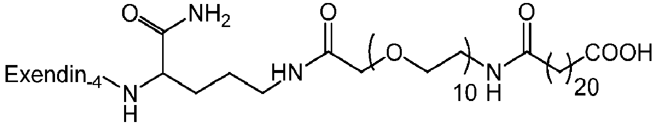

43mg pure target peptide was finally obtained.
MS(ESI⁺, m/e): 5147.81[M+H]⁺

### Example 50 Preparation of compound 50

Compound 50 was prepared with reference to the method of Example 46.

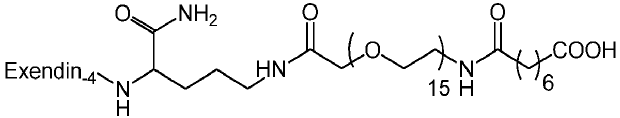

42.8mg pure target peptide was finally obtained.
MS(ESI⁺, m/e): 5171.68[M+H]⁺

### Example 51 Preparation of compound 51

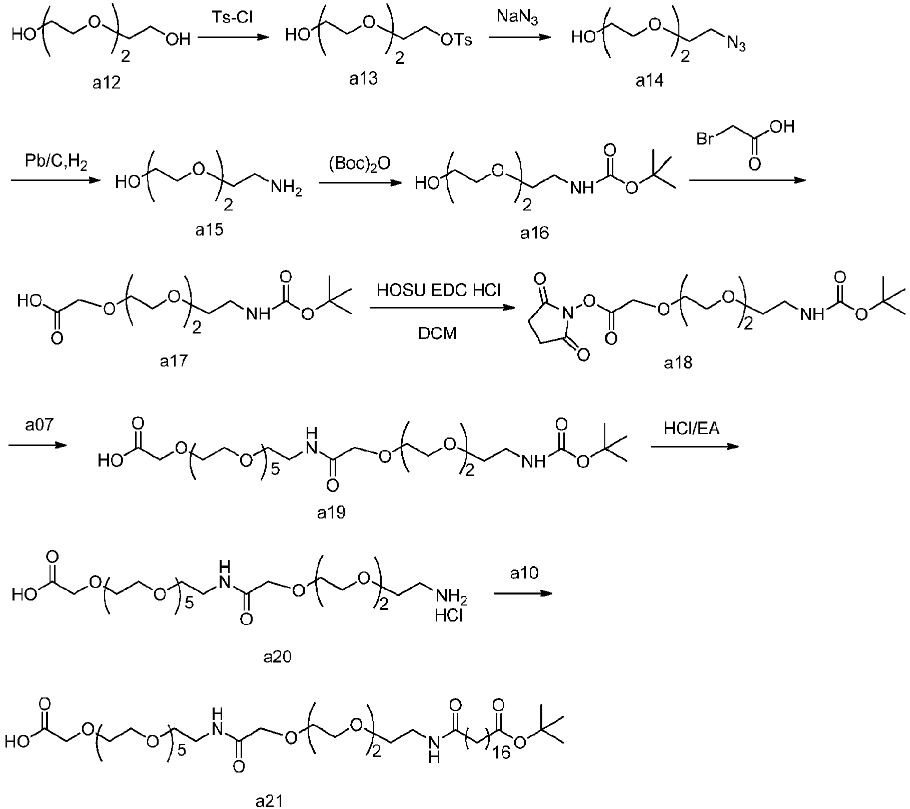

### Preparation of compound a13

Under the protection of nitrogen, to a 1000ml three-necked flask were added 200 mL pyridine, 120 g a12(1.0eq), stirred and cooled down to 0°C. 151.8g TsCl (1.0eq) was added in batches, stirred for 1h, and then slowly warmed up to room temperature, continuing to stir for 3-4h. After the completion of the reaction, the reaction liquid was poured into the ice-cold solution of dilute hydrochloric acid, with a solid being generated, which was extracted with ethyl acetate. The ethyl acetate layer was washed once with dilute hydrochloric acid, washed with saturated sodium bicarbonate and saturated brine, and dried over anhydrous Na₂SO₄. The solvents were evaporated off at reduced pressure to give 119g crude product, which was chromatographed in a silica gel column to give 55g pure a13.

### Preparation of compound a14

To a 1000 mL three-necked flask were added 55 g a13(1.0eq) and 160mL DMSO, stirred evenly, then added NaN₃ 23.52 g (2.0 eq), heated to 50°C and reacted for 3 hours, cooled down to room temperature. The reaction liquid was poured into 1.2L water, extracted with ethyl acetate. The organic phases were combined, dried over anhydrous sodium sulfate, and concentrated to give 29.2g a14 as a colourless liquid.

### Preparation of compound a15

To a 1L hydrogenation reactor were added 29g compound a14, methanol 360mL, palladium on carbon 5.0g, stirred, with nitrogen replaced by introducing hydrogen to react for 3-4h. After the completion of the reaction under the monitor of TLC, the reaction liquid was filtered, and the filtrate was concentrated to give 23.5g a15 as an oil.

### Preparation of compound a16

To a 1 L three-necked flask were added 23.5 g compound a15 (1.0eq), 68.6g (Boc)₂O (2.0 eq), a mixed solution of methanol:triethylamine (9:1) 500ml, stirred and warmed to reflux, and reacted for 1h. After the completion of the reaction under the monitor of TLC, methanol triethylamine was evaporated off, and dissolved in water. Dichloromethane was extracted for 3 times. The organic layers were combined and washed once with water, dried over anhydrous sodium sulfate, evaporated off the solvents, and dried to give 34.8g a16 as solid.

### Preparation of compound a17

To a 1000mL three-necked flask were added 34.8 g compound a16 (1.0eq), toluene and THF 150ml for each, bromoacetic acid 58.2 g (3eq), stirred, heated to 45∼50°C, then added sodium hydroxide 33.5g (6eq), and reacted overnight. After the completion of the reaction under the monitor of TLC, the reaction liquid was evaporated off, extracted with water and ethyl acetate, and the aqueous phase was adjusted to pH 3. The aqueous phase was extracted with dichloromethane, and the dichloromethane layers were combined, dried over anhydrous sodium sulfate, and then concentrated to give 18g a17 oily compound.

### Preparation of compound a18

To a 100mL three-necked flask were added 286mg N-hydroxy succinimide (HOSU), 0.50g a17 and 5 ml dichloromethane, into which was added 477mg EDC•HCl, and reacted for 2h at room temperature. After the completion of the reaction under the monitor of TLC, they were diluted with dichloromethane, and then washed with 50mmol/L aqueous solution of potassium dihydrogen phosphate at pH=6.0 for 2 times, washed with saturated brine, dried over anhydrous sodium sulfate, and concentrated to give 0.72g compound a18 as an oil.

### Preparation of compound a19

To a 100mL flask were added 0.62g compound a07 (1.0eq), 10 ml water, 0.27 g NaHCO₃ (2.0eq), and stirred. A solution of 0.66 g compound a18 in 10ml DME (ethylene glycol dimethyl ether) was added dropwise, replenished with 5ml THF, and stirred overnight. After the completion of the reaction under the monitor of TLC, the organic solvents were evaporated off, adjusted to pH=4 with dilute hydrochloric acid, extracted with dichloromethane, dried over anhydrous sodium sulfate, and concentrated to give 0.71g compound a19 as an oil.

### Preparation of compound a20

To a 100mL flask was added 0.71g compound a19, which was dissolved with 5ml ethyl acetate and then cooled down to 0°C, with the addition of 5 ml HCl/ethyl acetate (7mol/L), keeping the temperature at 0°C. After the completion of the reaction under the monitor of TLC, they were concentrated to give 0.71g a20 as an oil.

### Preparation of compound a21

To a 100mL flask were added 640mg compound a20 (1.0eq), 15 ml water, 190mg NaHCO₃ (2.0eq), and stirred. A solution of 528mg compound a10 in 15ml DME (ethylene glycol dimethyl ether) was added dropwise, replenished with 15ml THF, and stirred overnight. After the completion of the reaction under the monitor of TLC, the organic solvents were evaporated off, adjusted to pH=6 with acetic acid, extracted with dichloromethane, dried over anhydrous sodium sulfate, and concentrated to give 0.65g a21 as an oil.

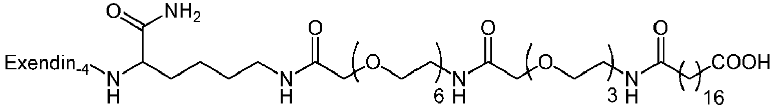

1.5g Exendin-4(1-39)-Lys40(Alloc)-NH₂ resin was swelled in DMF, into which was then added 3eq solution of Pd(PPh₃)₄ in CHCl₃:AcOH:NMM (18:1:0.5). They were reacted for 2h, then washed with chloroform (6 times, 20ml chloroform for each time), washed with 20% solution of HOAc in dichloromethane (6 times, 20ml 20% solution of HOAc in dichloromethane for each time), washed with dichloromethane (6 times, 20ml dichloromethane for each time) and washed with DMF (6 times, 20ml DMF for each time). When it was detected with ninhydrin as positive, 5ml DMF, 528mg compound a21, 150mg HOAT (1-hydroxy-7-azobenzotriazole), and 150ul DIC were added and reacted for 4h; and when it was detected with ninhydrin as negative, indicting that the side chain a21 has connected onto Exendin-4(1-39)-Lys40-NH₂ resin. The pyrolysis of the resin was carried out by employing 82.5% TFA/5% phenol/5% water/2.5% EDT/5% thioanisole, and then they were precipitated with ice-cold methyl tert-butyl ether (MTBE), and washed. Crude products were purified by HPLC to give 48mg target compound.
MS(ESI⁺, m/e): 5123.50[M+H]⁺

### Example 52 Preparation of compound 52

Compound 52 was prepared with reference to the method of Example 51.

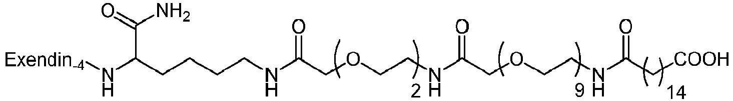

47.5mg pure target peptide was finally obtained.
MS(ESI⁺, m/e): 5178.76[M+H]⁺

### Example 53 Preparation of compound 53

Compound 53 was prepared with reference to the method of Example 51.

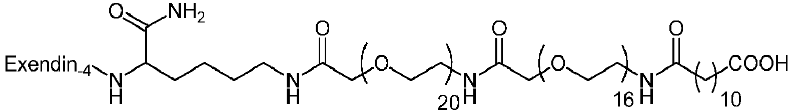

53mg pure target peptide was finally obtained.
MS(ESI⁺, m/e): 6223.43[M+H]⁺

### Example 54 Preparation of compound 54

Compound 54 was prepared with reference to the method of Example 51.

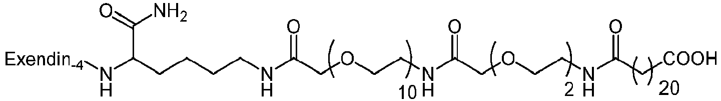

48.3mg pure target peptide was finally obtained.
MS(ESI⁺, m/e): 5306.91[M+H]⁺

### Example 55 Preparation of compound 55

Compound 55 was prepared with reference to the method of Example 51.

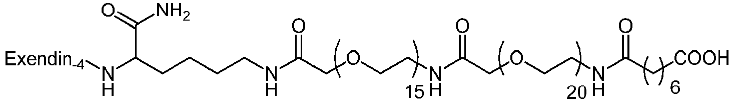

52.8mg pure target peptide was finally obtained.
MS(ESI⁺, m/e): 6123.32[M+H]⁺

### Example 56 Preparation of compound 56

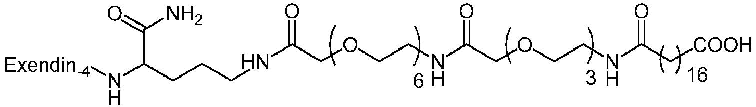

1.5g Exendin-4(1-39)-Orn40(Alloc)-NH₂ resin was swelled in DMF, into which was then added 3eq solution of Pd (PPh₃)₄ in CHCl₃:AcOH:NMM (18:1:0.5). They were reacted for 2h, then washed with chloroform (6 times, 20ml chloroform for each time), 20% solution of HOAc in dichloromethane (6 times, 20ml 20% solution of HOAc in dichloromethane for each time), washed with dichloromethane (6 times, 20ml dichloromethane for each time) and washed with DMF (6 times, 20ml DMF for each time). When it was detected with ninhydrin as positive, 5ml DMF, 528mg compound **BP103m53,** 150mg HOAT, 150ul DIC were reacted for 4h; and when it was detected with ninhydrin as negative, indicting that the side chain **BP103m53** has connected onto Exendin-4(1-39)-Orn40-NH₂ resin. The pyrolysis of the resin was carried out by employing 82.5% TFA/5% phenol/5% water/2.5% EDT/5% thioanisole, and then they were precipitated with ice-cold methyl tert-butyl ether (MTBE), and washed. Crude products were purified by HPLC to give 47mg target compound.
MS(ESI⁺, m/e): 5104.72[M+H]⁺

### Example 57 Preparation of compound 57

Compound 57 was prepared with reference to the method of Example 56.

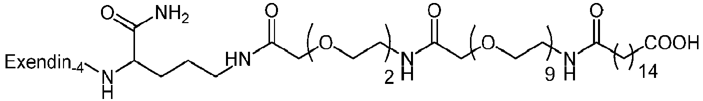

48mg pure target peptide was finally obtained.
MS(ESI⁺, m/e): 5164.74[M+H]⁺

### Example 58 Preparation of compound 58

Compound 58 was prepared with reference to the method of Example 56.

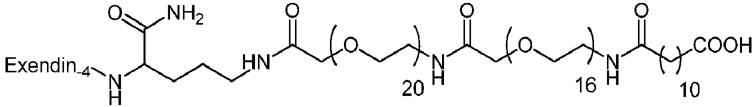

51mg pure target peptide was finally obtained.
MS(ESI⁺, m/e): 6209.41 [M+H]⁺

### Example 59 Preparation of compound 59

Compound 59 was prepared with reference to the method of Example 56.

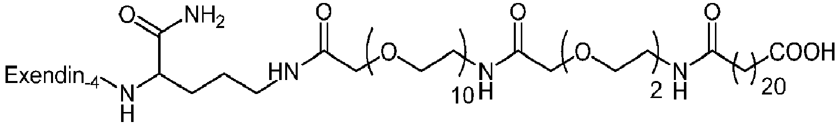

47.6mg pure target peptide was finally obtained.
MS(ESI⁺, m/e): 5292.89[M+H]⁺

### Example 60 Preparation of compound 60

Compound 60 was prepared with reference to the method of Example 56.

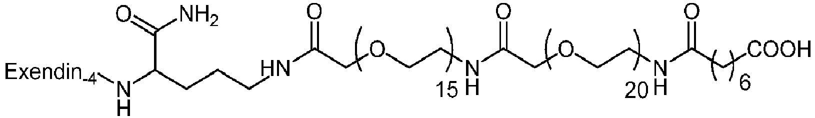

49.7mg pure target peptide was finally obtained.
MS(ESI⁺, m/e): 6109.3[M+H]⁺

### Example 61 Preparation of compound 61

### Preparation of Exendin-4(1-39)-Cys(40)-NH₂

The solid phase peptides of target peptides were synthesized by empolying the solid phase synthesis of Fmoc process, using Fmoc-Rink MBHA Amide resin, in which 20% piperidine/DMF was used to remove Fmoc, HOBT/DIC was used as the coupling reagent, and the reactive solvent was DMF. The reaction was monitored by employing the ninhydrin detection method, successively connecting the following protected amino acids onto the Rink MBHA Amide resin: Fmoc-Cys(Trt)-OH, Fmoc-Ser(tBu)-OH, Fmoc-Pro-OH, Fmoc-Pro-OH, Fmoc-Pro-OH, Fmoc-Ala-OH, Fmoc-Gly-OH, Fmoc-Ser(tBu)-OH, Fmoc-Ser(tBu)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg (Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Ser(tBu)-OH, Fmoc-Leu-OH, Fmoc-Asp(OtBu)OH, Fmoc-Ser(tBu)-OH, Fmoc-Thr(tBu)-OH, Fmoc-Phe-OH, Fmoc-Thr(tBu)-OH, Fmoc-Gly-OH, Fmoc-Glu(OtBu)-OH, Fmoc-Gly-OH, Fmoc-His(Trt)-OH, with Fmoc protection finally removed. They were washed with DMF, dichloromethane, and MeOH, and then dried to give resin with fully protection. The pyrolysis of the resin was achieved by employing 82.5% TFA/5% phenol/5% water/2.5% EDT/5% thioanisole, and then they were precipitated with ice-cold methyl tert-butyl ether (MTBE), and washed, and crude products were purified by reverse HPLC to give pure Exendin-4(1-39)-Cys(40)-NH₂.

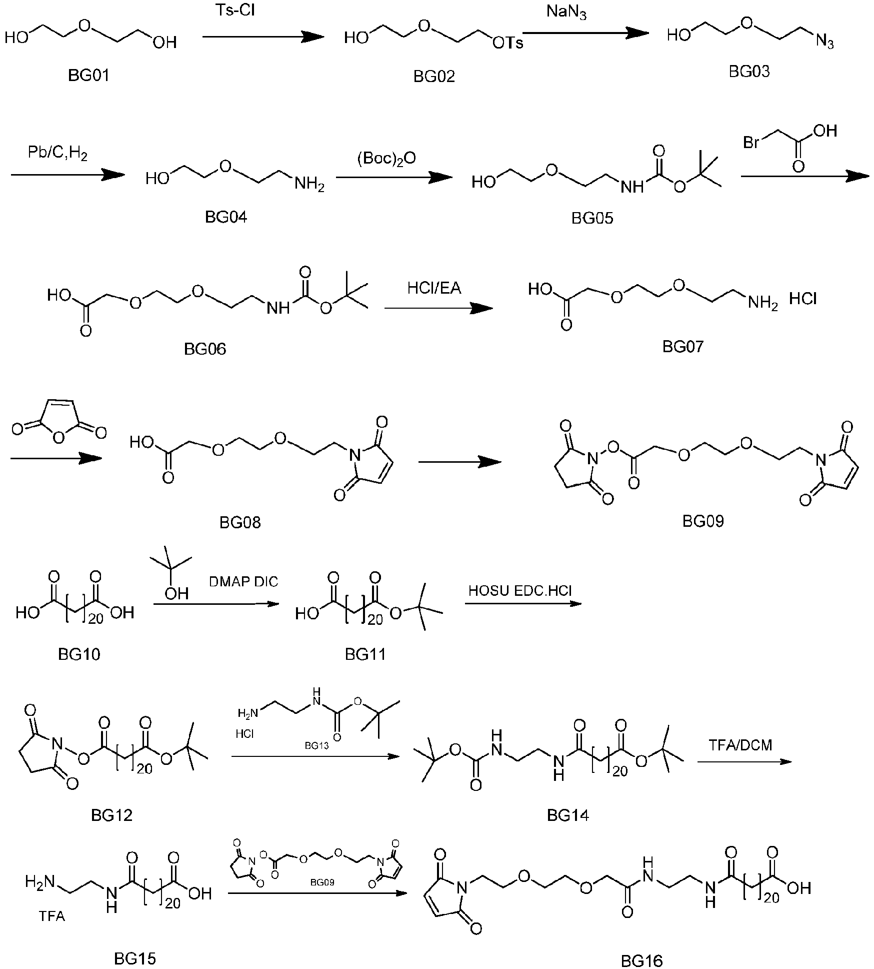

### Preparation of BG02

Under the protection of nitrogen, to a 500ml three-necked flask were added 200 mL pyridine, 18.8 g BG01 (1.0eq), stirred and cooled down to 0°C. 35.5g TsCl (1.0eq) was added in batches, stirred for 1h, and then slowly warmed up to room temperature, continuing to stir for 3-4h. After the completion of the reaction, the reaction liquid was poured into the ice-cold solution of dilute hydrochloric acid, with a solid being generated, which was extracted with ethyl acetate. The ethyl acetate layer was washed once with dilute hydrochloric acid, washed with saturated sodium bicarbonate and saturated brine, and dried over anhydrous Na₂SO₄. The solvents were evaporated off at reduced pressure, and chromatographed in a silica gel column to give 22.7g pure BG02.

### Preparation of BG03

To a 500 mL three-necked flask were added 22.7g BG02(1.0eq) and 190mL DMSO, stirred evenly, then added NaN₃ 11.5 g (2.0 eq), heated to 50°C and reacted for 3 hours, cooled down to room temperature. The reaction liquid was poured into water, extracted with ethyl acetate for many times. The organic phases were combined, dried over anhydrous sodium sulfate, and concentrated to give 17.1g BG03 as a colourless liquid.

### Preparation of BG04

To a 1L hydrogenation reactor were added BG03 30g, methanol 500 mL, palladium on carbon 8.0g, stirred, with nitrogen replaced by introducing hydrogen to react for 3-4h. After the completion of the reaction under the monitor of TLC, the reaction liquid was filtered, and the filtrate was concentrated to give 19.4g BG04 as an oil.

### Preparation of BG05

To a 500mL three-necked flask were added compound BG04 3.7g (1. 0eq), (Boc)₂O 15.5g (2.0 eq), a mixed solution of methanol:triethylamine (9:1) 200ml, stirred and warmed to reflux, and reacted for 1h. After the completion of the reaction under the monitor of TLC, methanol triethylamine was evaporated off, and dissolved in water. Dichloromethane was extracted for 3 times. The organic layers were combined and washed once with water, dried over anhydrous sodium sulfate, and concentrated to give 4.8g BG05 as an oil.

### Preparation of BG06

To a 250mL three-necked flask were added BG05 compound 3.7g (1.0eq), toluene and THF 40ml for each, bromoacetic acid 7.6 g (3.0eq), stirred, heated to 45∼50°C, then added sodium hydroxide 4.4g, and reacted overnight. After the completion of the reaction under the monitor of TLC, the reaction liquid was evaporated off, the impurities were extracted with water and ethyl acetate, and the aqueous phase was adjusted to pH=3. The aqueous phase was extracted with dichloromethane, and the dichloromethane layers were combined, dried over anhydrous sodium sulfate, and then concentrated to give 2.5g BG06 oily compound.

### Preparation of BG07

To a 100mL single-neck flask were added 2.4g compound BG06 and 20ml ethyl acetate, after being dissolved, they were cooled down to 0°C, into which was added 20 ml HCl/ethyl acetate (7mol/L). After the completion of the reaction under the monitor of TLC, they were concentrated to give 2.3g BG07 as an oil.

### Preparation of BG08

To a 200mL three-necked flask were added 3.5g compound BG07(1.0eq), 1.7g maleic anhydride (1.0eq), 70ml acetic acid, and heated to reflux overnight. After the completion of the reaction under the monitor of TLC, the acetic acid was evaporated off, into which was added ethyl acetate to dissolve, and then washed with water for 3 times, washed with saturated sodium chloride for 3 times, dried over anhydrous sodium sulfate, and chromatographed in a column to give 1.8g BG08 as an off-white solid.

### Preparation of BG09

To a 100mL three-necked flask were added 1.30 g (1.53eq) N-hydroxy succinimide (HOSU), 3.1g compound, BG08 1.8g and 15 ml dichloromethane, into which was added 2.16g EDC·HCl (1.53eq) and reacted for 2h at room temperature. After the completion of the reaction under the monitor of TLC, they were diluted with dichloromethane, and then washed with 50mmol/L aqueous solution of potassium dihydrogen phosphate at pH=6.0 for 2 times, washed with saturated brine, dried over anhydrous sodium sulfate, and concentrated to give 2.2g off-white compound BG09.

### Preparation of BG11

To a 50mL three-necked flask were added 2.17g compound BG10 (1.0eq), 10ml dichloromethane, 10ml tert-butanol, 0.40g DIC (1.0eq), and 0.39g DMAP (1.0eq), and stirred at room temperature overnight. After the completion of the reaction under the monitor of TLC, they were diluted with ether, and then washed with water for 3 times, washed with saturated brine, dried over anhydrous sodium sulfate, and chromatographed in a column to give 0.6g foamy powder BG11.

### Preparation of BG12

To a 100mL three-necked flask were added 0.95 g N-hydroxy succinimide (HOSU), 3.1g compound BG11 and 15 ml dichloromethane, into which was added 1.58g EDC·HCl, and reacted for 2h at room temperature. After the completion of the reaction under the monitor of TLC, they were diluted with dichloromethane, and then washed with 50mmol/L aqueous solution of potassium dihydrogen phosphate at pH=6.0 for 2 times, washed with saturated brine, dried over anhydrous sodium sulfate, and concentrated to give 3.3g compound BG12 as a white solid.

### Preparation of BG14

To a 200mL flask were added 2.8g compound BG13 (1.05eq), 50 ml water, 1.8 g NaHCO₃ (2.0eq), and stirred. A solution of 6.4g compound BG12 (1.0eq) in 50ml DME (ethylene glycol dimethyl ether) was added dropwise, replenished with 50ml THF, and stirred overnight. After the completion of the reaction under the monitor of TLC, the organic solvents were evaporated off, adjusted to pH=4 with acetic acid, extracted with ethyl acetate, dried over anhydrous sodium sulfate, and concentrated to give 6.9g compound BG14 as an off-white solid.

### Preparation of BG15

To a 100mL flask were added 6.9g compound BG14, 30 ml dichloromethane, 30ml TFA, and stirred at 20°C. After the completion of the reaction under the monitor of TLC, the organic solvents were evaporated off, slurried with petroleum ether, suction filtrated, and dried to give 5.35g BG15 as an off-white solid.

### Preparation of BG16

To a 100mL flask were added 1.27g compound BG15 (1.0eq), 10 ml water, 0.36 g NaHCO₃ (2.0eq), and stirred. A solution of 0.72g compound BG09 (1.0eq) in 10ml DME (ethylene glycol dimethyl ether) was added dropwise, replenished with 10ml THF, and stirred overnight. After the completion of the reaction under the monitor of TLC, the organic solvents were evaporated off, adjusted to pH=6 with acetic acid, and extracted with ethyl acetate. The organic phases were washed with water and saturated brine, and then dried over anhydrous sodium sulfate, concentrated, and chromatographed in a column to give 0.9g compound BG16 as an off-white solid.

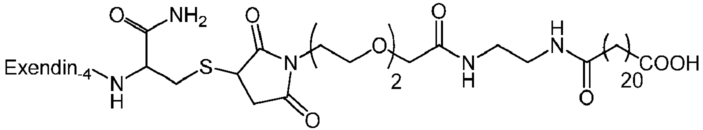

50.0mg Exendin-4(1-39)-Cys(40)-NH₂ was dissolved in 10ml sodium phosphate buffer (pH 6.5) at 20mM, into which was added 16mg BG16 and stirred for 1 hour at the condition of 20°C. After the completion of the reaction under the monitor of HPLC, the reaction was stopped with excess cysteine solution (0.5ml 0.5M cysteine solution), prepared with HPLC and then lyophilized to give 30mg coupling compound.
MS(ESI⁺, m/e): 4926.49[M+H]⁺.

### Example 62 Preparation of compound 62

Compound 62 was prepared with reference to the method of Example 61.

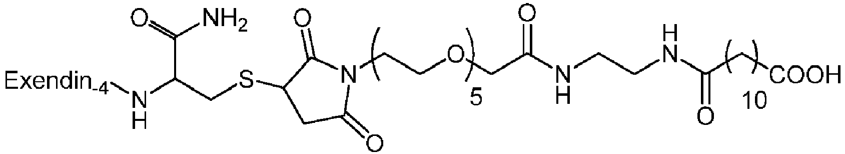

Crude products were purified by HPLC to give 31mg target compound.
MS(ESI⁺, m/e):4918.41[M+H]⁺.

### Example 63 Preparation of compound 63

Compound 63 was prepared with reference to the method of Example 61.

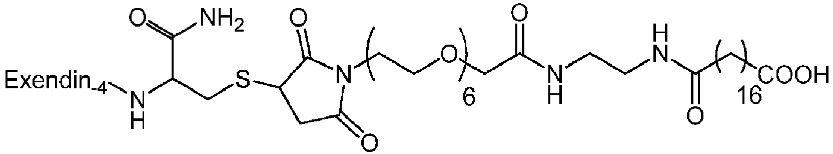

Crude products were purified by HPLC to give 31mg target compound.
MS(ESI⁺, m/e): 5046.53[M+H]⁺.

### Example 64 Preparation of compound 64

Compound 64 was prepared with reference to the method of Example 61.

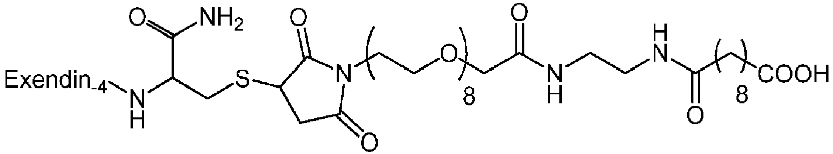

Crude products were purified by HPLC to give 29mg target compound.
MS(ESI⁺, m/e): 5022.46[M+H]⁺.

### Example 65 Preparation of compound 65

Compound 65 was prepared with reference to the method of Example 61.

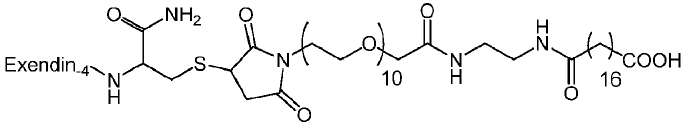

Crude products were purified by HPLC to give 34mg target compound.
MS(ESI⁺, m/e): 5222.63[M+H]⁺.

### Example 66 Preparation of compound 66

Compound 66 was prepared with reference to the method of Example 61.

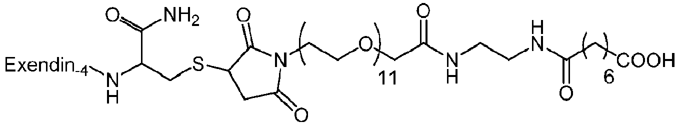

Crude products were purified by HPLC to give 32mg target compound.
MS(ESI⁺, m/e): 5126.5[M+H]⁺.

### Example 67 Preparation of compound 67

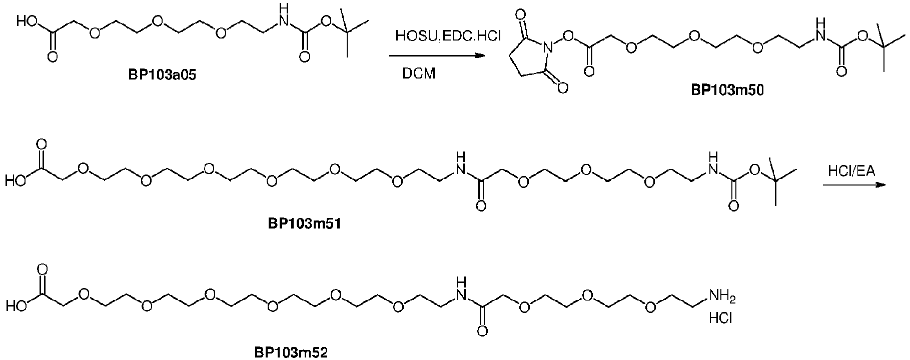

### Preparation of compound BP103m50

To a 100mL three-necked flask were added 286mg N-hydroxy succinimide (HOSU), 0.50g BP103a05 and 5 ml dichloromethane, into which was added 477mg EDC.HCl and reacted for 2h at room temperature. After the completion of the reaction under the monitor of TLC, they were diluted with dichloromethane, and then washed with 50mmol/L aqueous solution of otassium dihydrogen phosphate at Ph=6.0 for 2 times, washed with saturated brine, dried over anhydrous sodium sulfate, and concentrated to give 0.72g compound BP103m50 as an oil.

### Preparation of compound BP103m51

To a 100mL flask were added 0.62g compound BP103g06 (1.0eq), 10 ml water, 0.27 g NaHCO₃ (2.0eq), and stirred. A solution of 0.66 g compound BP103m50 in 10ml DME (ethylene glycol dimethyl ether) was added dropwise, replenished with 5ml THF, and stirred overnight. After the completion of the reaction under the monitor of TLC, the organic solvents were evaporated off, adjusted to pH=4 with dilute hydrochloric acid, extracted with dichloromethane, dried over anhydrous sodium sulfate, and concentrated to give 0.71g compound BP103m51 as an oil.

### Preparation of compound BP103m52

To a 100mL flask were added 0.71g compound BP103m51 and 5ml ethyl acetate, after being dissolved, they were cooled down to 0°C, into which was added 5 ml HCl/ethyl acetate (7mol/L), keeping the temperature at 0°C. After the completion of the reaction under the monitor of TLC, they were concentrated to give 0.71g BP103m52 as an oil.

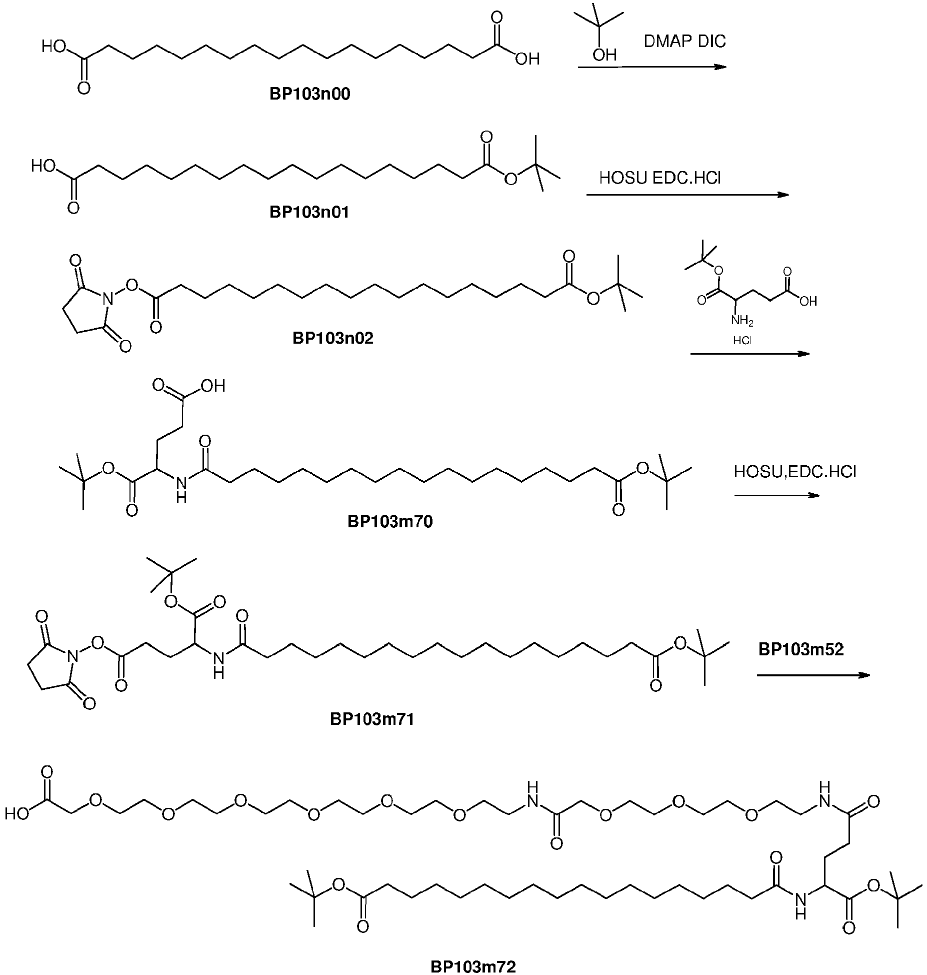

### Preparation of compound BP103n01

To a 50mL three-necked flask were added 1.0g compound BP103n00 (1.0eq), 10ml dichloromethane, 10ml tert-butanol, 0.40g DIC (1.0eq), and 0.39g DMAP (1.0eq), and stirred overnight at room temperature. After the completion of the reaction under the monitor of TLC, they were diluted with ether, and washed with water for 3 times, washed with saturated brine, dried over anhydrous sodium sulfate, and chromatographed in a column to give 0.4g BP103n01 as a foamy powder.

### Preparation of compound BP103n02

To a 100mL three-necked flask were added 0.95 g N-hydroxy succinimide (HOSU), 2.0g compound BP103n01 and 15 ml dichloromethane, into which was added 1.58g EDC.HCl and reacted for 2h at room temperature. After the completion of the reaction under the monitor of TLC, they were diluted with dichloromethane, and then washed with 50mmol/L aqueous solution of potassium dihydrogen phosphate at pH=6.0 for 2 times, washed with saturated brine, dried over anhydrous sodium sulfate, and concentrated to give 2.6g compound BP103n02 as a white solid.

### Preparation of compound BP103m70

To a 100mL flask were added 0.50 g compound H-Glu-OtBu.HCl (1.0eq), 10 ml water, 350mg NaHCO₃(2.0eq), and stirred. A solution of 0.96 g compound BP103n02 (1.0eq) in 10ml DME (ethylene glycol dimethyl ether) was added dropwise, replenished with 10ml THF, and stirred overnight. After the completion of the reaction under the monitor of TLC, the organic solvents were evaporated off, adjusted to pH=6 with acetic acid, extracted with dichloromethane, dried over anhydrous sodium sulfate, and concentrated to give 1.09g compound BP103m70 as an oil.

### Preparation of compound BP103m71

To a 100mL three-necked flask were added 1.0g compound BP103m70, 317mg N-hydroxy succinimide (HOSU) (1.53eq), and 10 ml dichloromethane, into which was added 528mg EDC.HCl (1.53eq) and reacted for 2h at room temperature. After the completion of the reaction under the monitor of TLC, they were diluted with dichloromethane, and then washed with 50mmol/L aqueous solution of potassium dihydrogen phosphate at pH=6.0 for 2 times, washed with saturated brine, dried over anhydrous sodium sulfate, and concentrated to give 1.07g compound BP103m71 as a white solid.

### Preparation of compound BP103m72

To a 100mL flask were added 0.87 g compound BP103m52 (1.0eq), 10 ml water, 300mg NaHCO₃ (2.0eq), and stirred. A solution of 1.00 g compound BP103m71 (1.0eq) in 10ml DME (ethylene glycol dimethyl ether) was added dropwise, replenished with 10ml THF, and stirred overnight. After the completion of the reaction under the monitor of TLC, the organic solvents were evaporated off, adjusted to pH=6 with acetic acid, extracted with dichloromethane, dried over anhydrous sodium sulfate, and concentrated to give 1.22g compound BP103m72 as an oil.

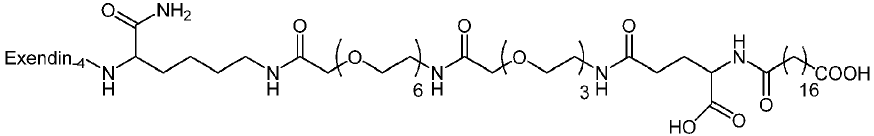

### Synthesis of Target Peptide

1.5g Exendin-4(1-39)-Lys40(Alloc)-NH₂ resin was swelled in DMF, into which was then added 3eq solution of Pd (PPh₃)₄ in CHCl₃:AcOH:NMM (18:1:0.5). They were reacted for 2h, then washed with chloroform (6 times, 20ml chloroform for each time), washed with 20% solution of HOAc in DCM (dichloromethane) (6 times, 20ml 20% solution of HOAc in DCM for each time), washed with DCM (6 times, 20ml DCM for each time) and washed with DMF (6 times, 20ml DMF for each time). When it was detected with ninhydrin as positive, 5ml DMF, 640mg compound BP103m72, 150mg HOAT, and 150ul DIC were added and reacted for 4h; and when it was detected with ninhydrin as negative, indicting that the side chain BP103m72 has connected onto Exendin-4(1-39)-Lys40-NH₂ resin. The pyrolysis of the resin was carried out by employing 82.5% TFA/5% phenol/5% water/2.5% EDT/5% thioanisole, and then they were precipated with ice-cold methyl tert-butyl ether (MTBE), and washed. Crude products were purified by HPLC to give 48mg target compound.
MS(ESI⁺, m/e): 5252.54[M+H]⁺.

## Claims

1. An exenatide modifier or pharmaceutically acceptable salts thereof having GLP-1 receptor agonist activity, as shown in formula (I):
(Ex-4)-L-Y (I)
wherein, Ex-4 is Exendin-4; L is 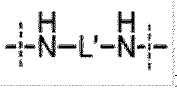 for connecting Ex-4 with Y; L' is a hydrophilic chain containing an ether group; Y is an aliphatic chain with a terminal carboxyl group, wherein the exenatide modifier is: 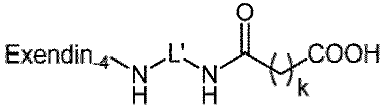 k is any integer between 6-20.

2. The exenatide modifier or pharmaceutically acceptable salts thereof according to claim 1, wherein L is selected from:
(1) 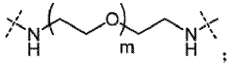
(2) 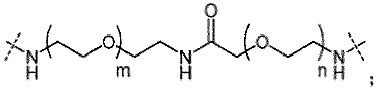
(3) 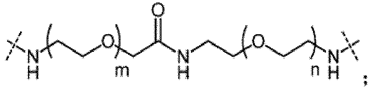
(4) 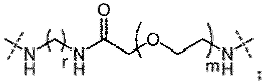
(5) 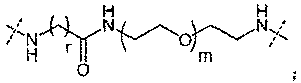
(6) 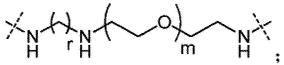
(7) 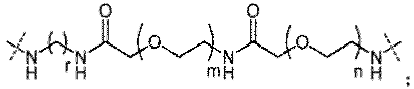
(8) 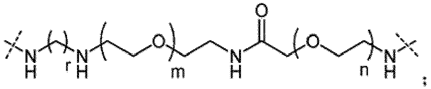
(9) 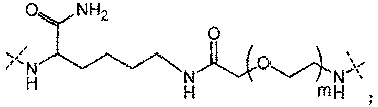
(10) 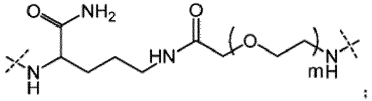
(11) 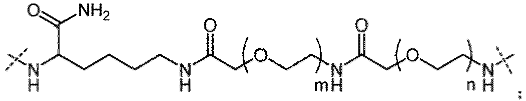
(12) 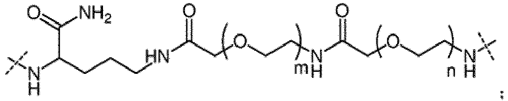
(13) 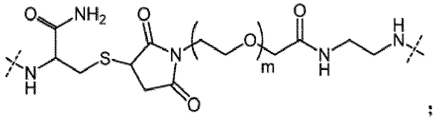
(14) 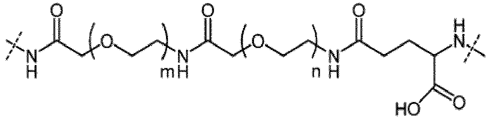 wherein, m is any integer between 2-20; n is any integer between 2-20; r is any integer between 1-6.

3. The exenatide modifier or pharmaceutically acceptable salts thereof according to claim 1, wherein the exenatide modifier is: 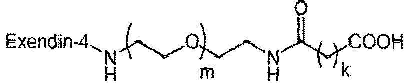 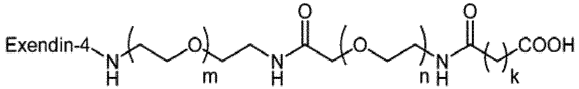 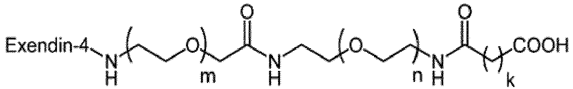 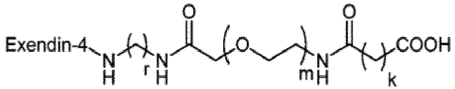 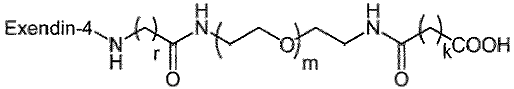 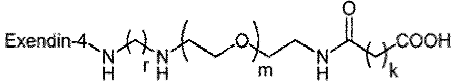 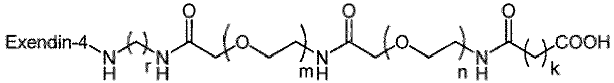 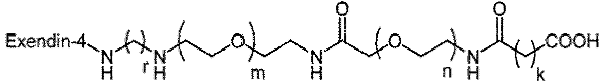 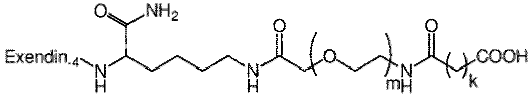 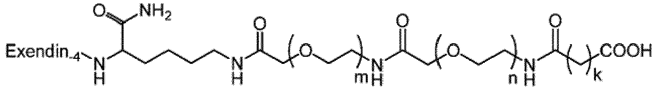 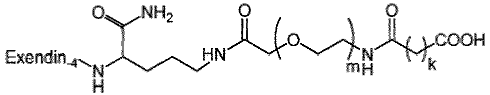 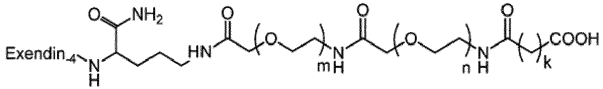 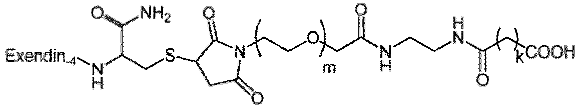 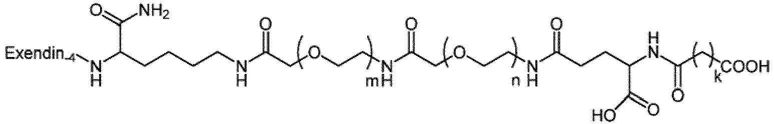 wherein m is any integer between 2-20; n is any integer between 2-20; r is any integer between 1-6; k is any integer between 6-20.

4. The exenatide modifier or pharmaceutically acceptable salts thereof according to claim 3, comprising the following compounds:

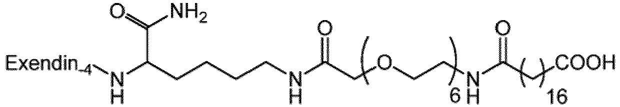

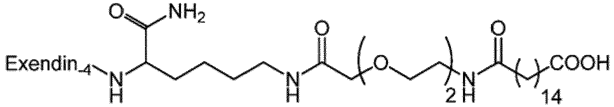

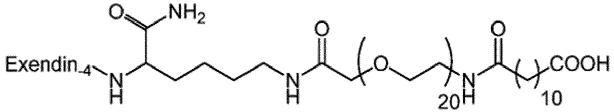

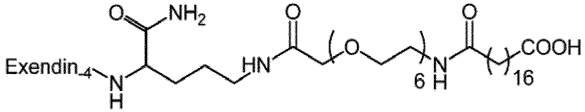

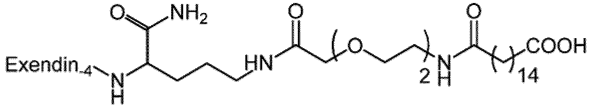

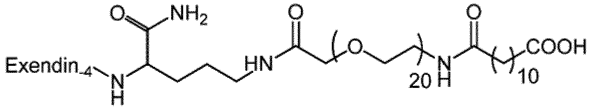

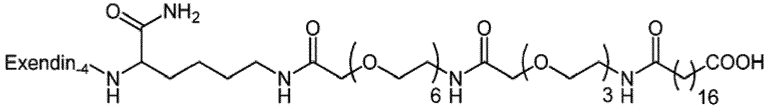

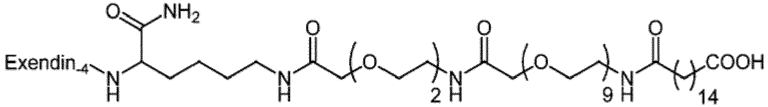

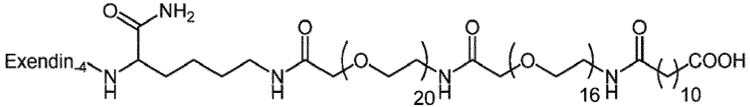

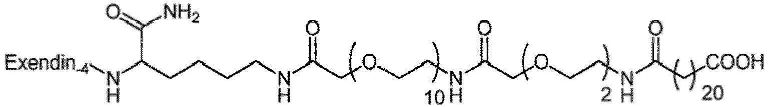

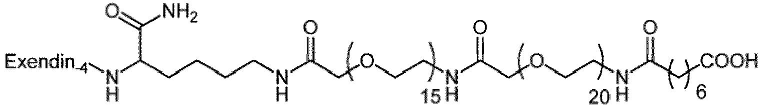

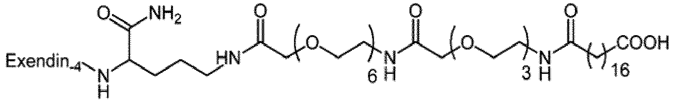

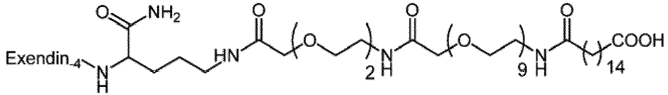

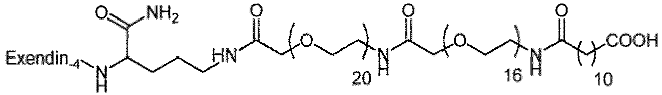

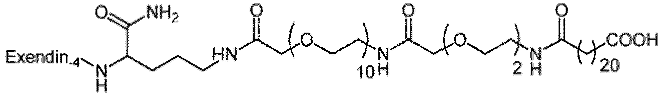

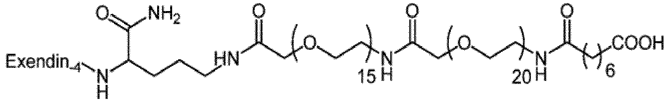

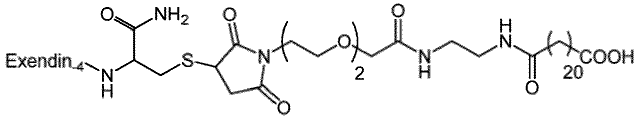

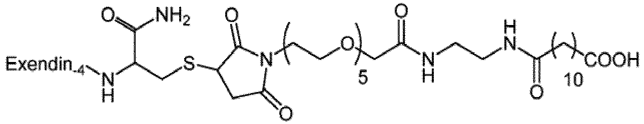

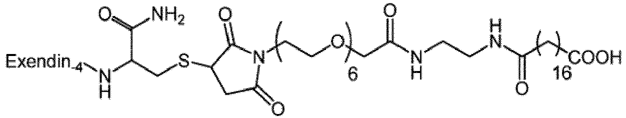

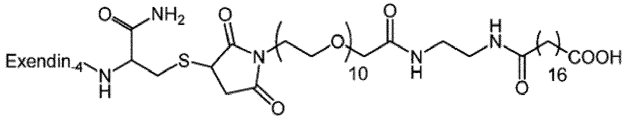

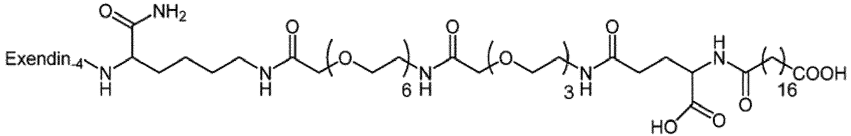

5. The exenatide modifier or pharmaceutically acceptable salts thereof according to claim 1 or 2 for
use in the treatment of diseases and/or symptoms associated with glycometabolism,
or for use in the treatment of diabetes,
or for use in the treatment of fatty liver,
or for use in the method for losing weight.

6. A pharmaceutical composition comprising the exenatide modifier or pharmaceutically acceptable salts thereof according to claim 1 or 2 and optionally pharmaceutically acceptable carriers.

7. The composition of claim 6 for use in the treatment of diseases and/or symptoms associated with
glycometabolism,
or for use in the treatment of diabetes,
or for use in the treatment of fatty liver,
or for use in the method for losing weight.

## Patentansprüche

1. Exenatidmodifikator oder pharmazeutisch annehmbare Salze davon mit GLP-1-Rezeptoragonistenaktivität, wie in Formel (I) gezeigt:
(Ex-4)-L-Y (I)
wobei Ex-4 Exendin-4 ist; L 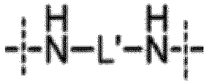 zum Verbinden von Ex-4 mit Y ist; L'eine hydrophile Kette ist, die eine Ethergruppe enthält; Y eine aliphatische Kette mit einer endständigen Carboxylgruppe ist, wobei der Exenatidmodifikator ist: 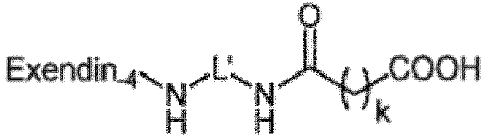 k eine beliebige ganze Zahl zwischen 6 - 20 ist.

2. Exenatidmodifikator oder pharmazeutisch annehmbare Salze davon nach Anspruch 1, wobei L ausgewählt ist aus:
(1) 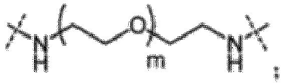
(2) 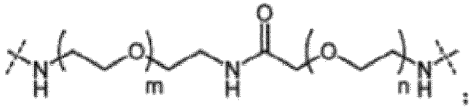
(3) 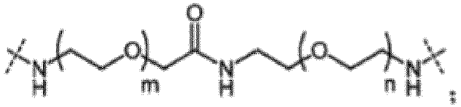
(4) 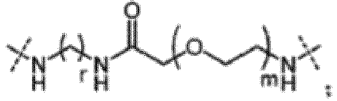
(5) 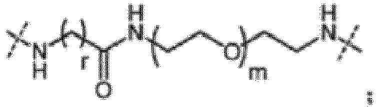
(6) 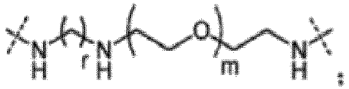
(7) 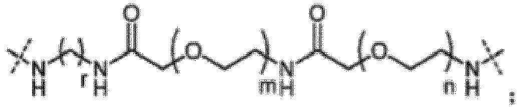
(8) 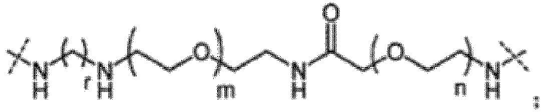
(9) 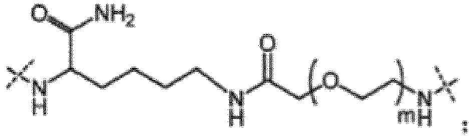
(10) 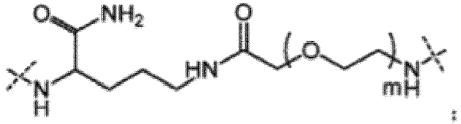
(11) 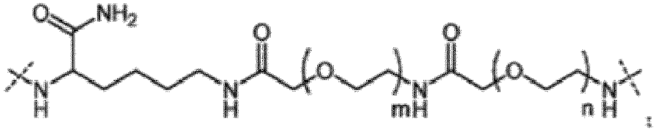
(12) 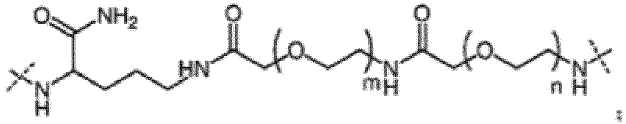
(13) 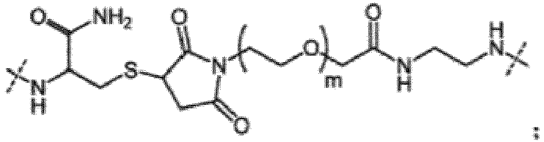
(14) 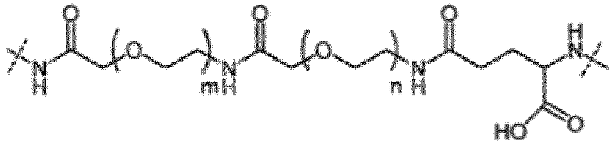 wobei m eine beliebige ganze Zahl zwischen 2 - 20 ist; n eine beliebige ganze Zahl zwischen 2 - 20 ist; r eine beliebige ganze Zahl zwischen 1 - 6 ist.

3. Exenatidmodifikator oder pharmazeutisch annehmbare Salze davon nach Anspruch 1, wobei der Exenatidmodifikator ist: 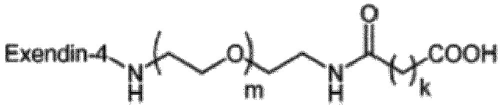 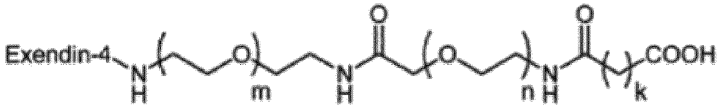 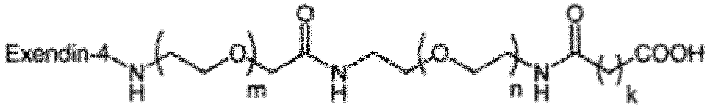 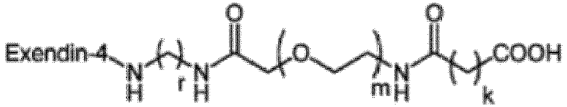 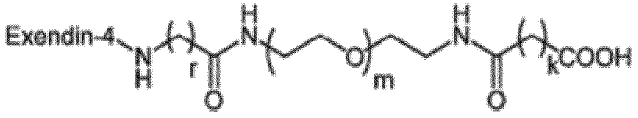 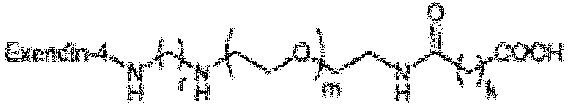 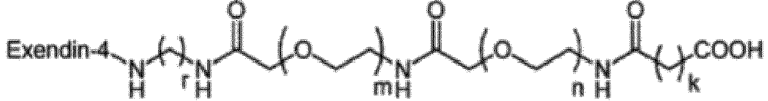 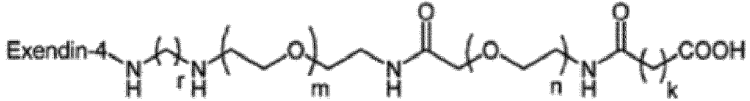 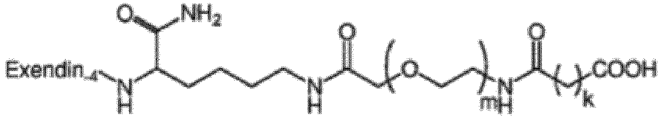 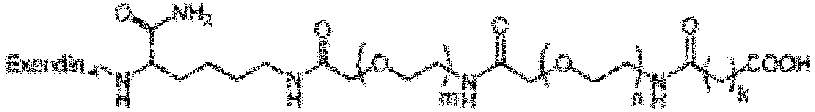 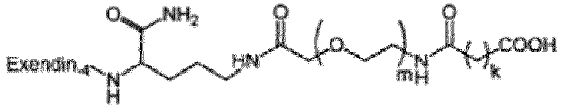 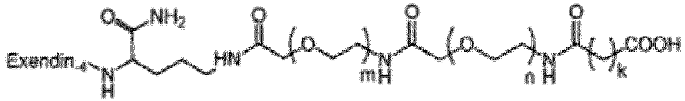 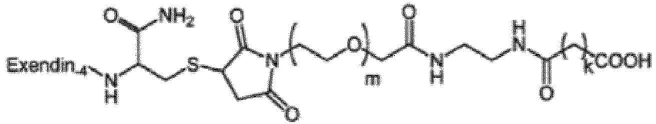 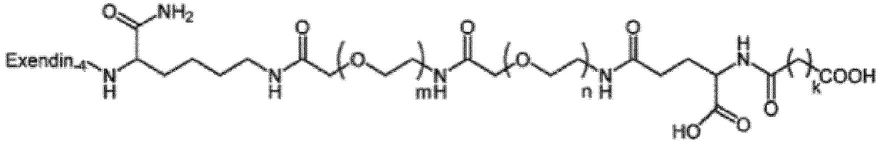 wobei m eine beliebige ganze Zahl zwischen 2 - 20 ist; n eine beliebige ganze Zahl zwischen 2 - 20 ist; r eine beliebige ganze Zahl zwischen 1 - 6 ist; k eine beliebige ganze Zahl zwischen 6 - 20 ist.

4. Exenatidmodifikator oder pharmazeutisch annehmbare Salze davon nach Anspruch 3, umfassend die folgenden Verbindungen:

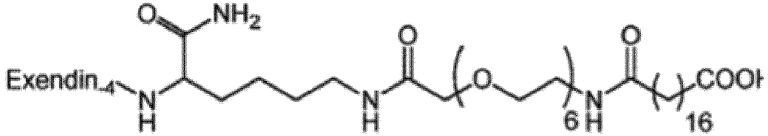

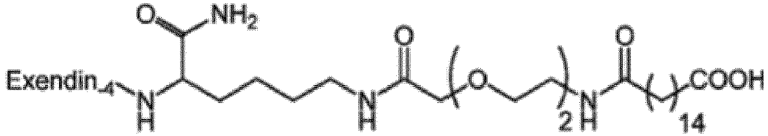

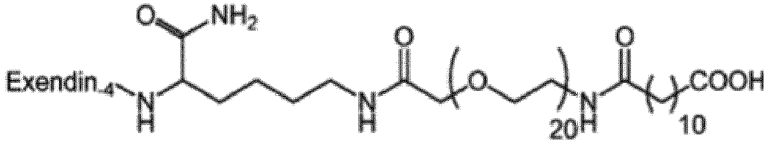

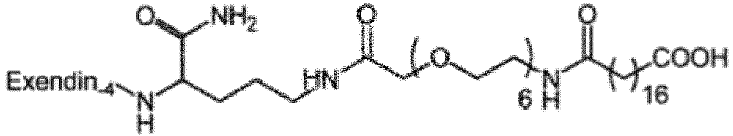

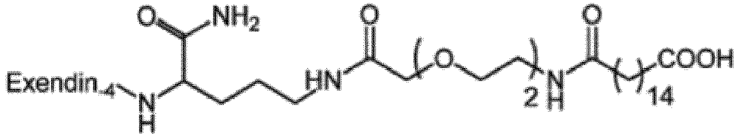

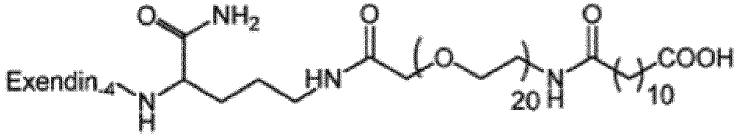

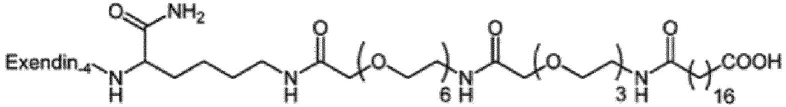

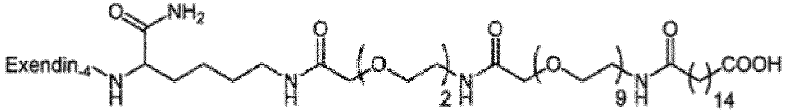

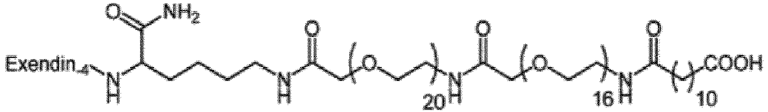

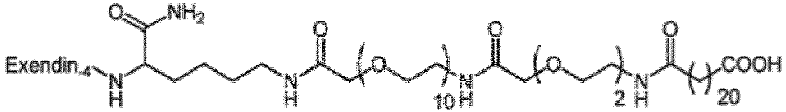

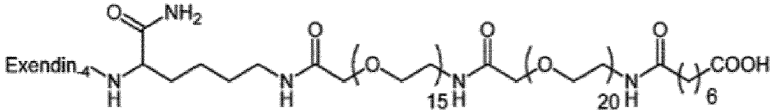

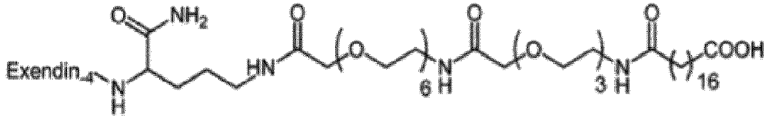

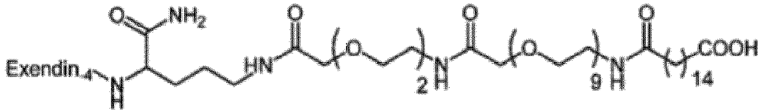

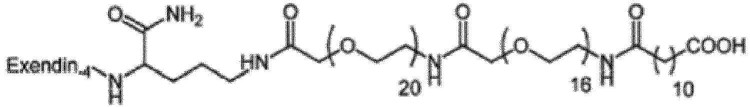

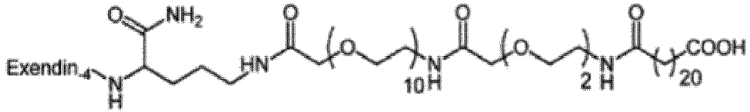

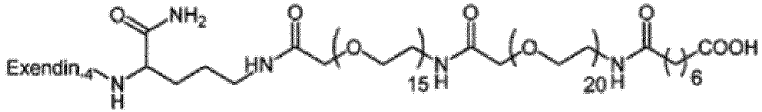

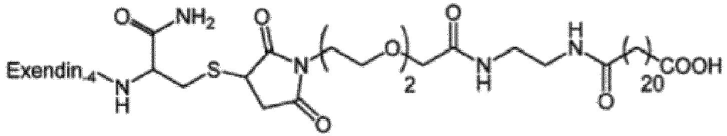

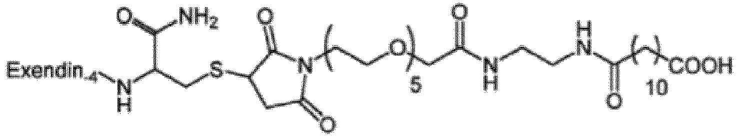

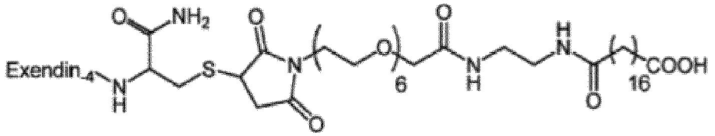

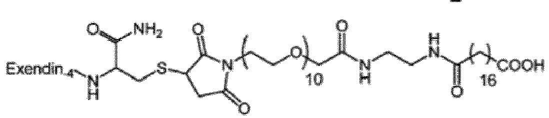

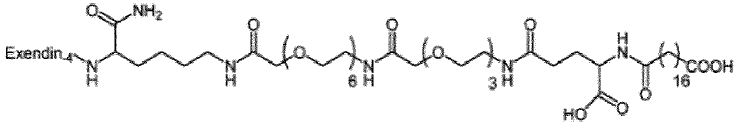

5. Exenatidmodifikator oder pharmazeutisch annehmbare Salze davon nach Anspruch 1 oder 2 zur
Verwendung bei der Behandlung von Krankheiten und/oder Symptomen, die mit dem Glykometabolismus verbunden sind,
oder zur Verwendung bei der Behandlung von Diabetes,
oder zur Verwendung bei der Behandlung von Fettleber,
oder zur Verwendung beim Verfahren zur Gewichtsabnahme.

6. Pharmazeutische Zusammensetzung umfassend den Exenatidmodifikator oder pharmazeutisch annehmbare Salze davon nach Anspruch 1 oder 2 und optional pharmazeutisch annehmbare Träger.

7. Zusammensetzung nach Anspruch 6 zur Verwendung bei der Behandlung von Krankheiten und/oder Symptomen, die Glycometabolismus zugeordnet sind,
oder zur Verwendung bei der Behandlung von Diabetes,
oder zur Verwendung bei der Behandlung von Fettleber,
oder zur Verwendung beim Verfahren zur Gewichtsabnahme.

## Revendications

1. Modificateur d'exénatide ou ses sels pharmaceutiquement acceptables ayant une activité agoniste du récepteur GLP-1, comme indiqué dans la formule (I) :
(Ex-4)-L-Y (I)
dans lequel Ex-4 est Exendin-4 ; L est 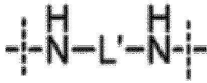 pour connecter Ex-4 avec Y ; L' est une chaîne hydrophile contenant un groupe éther ; Y est une chaîne aliphatique avec un groupe carboxyle terminal, dans lequel le modificateur d'exénatide est 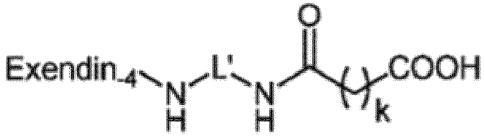 k est un entier compris entre 6 et 20.

2. Modificateur d'exénatide ou ses sels pharmaceutiquement acceptables selon la revendication 1, dans lequel L est choisi parmi :
(1) 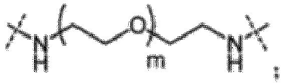
(2) 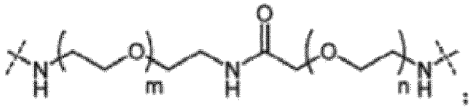
(3) 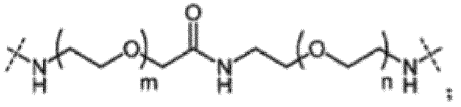
(4) 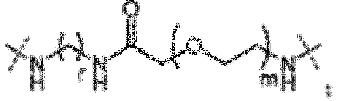
(5) 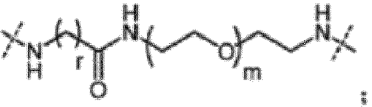
(6) 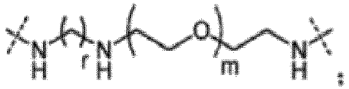
(7) 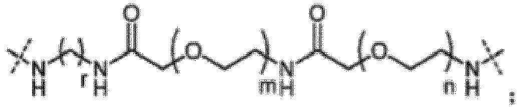
(8) 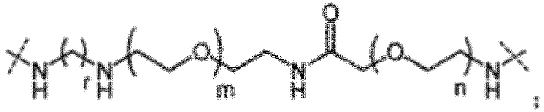
(9) 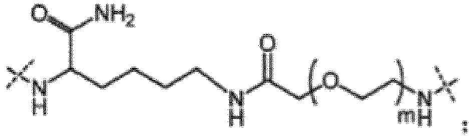
(10) 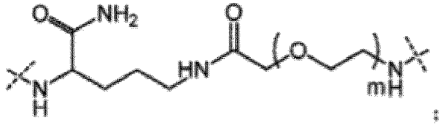
(11) 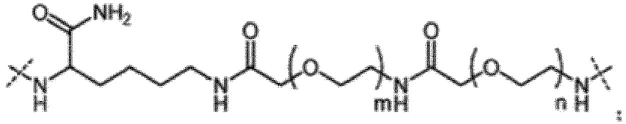
(12) 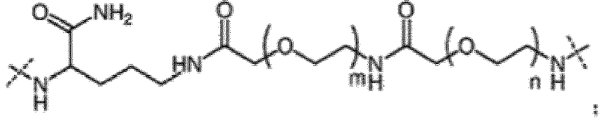
(13) 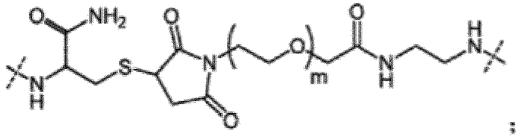
(14) 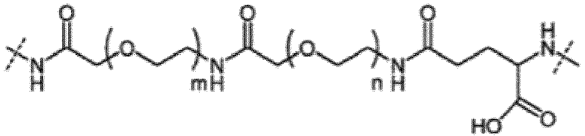 dans lequel m est un entier compris entre 2-20 ; n est un entier compris entre 2-20 ; r est un entier compris entre 1 et 6.

3. Modificateur d'exénatide ou ses sels pharmaceutiquement acceptables selon la revendication 1, dans lequel le modificateur d'exénatide est : 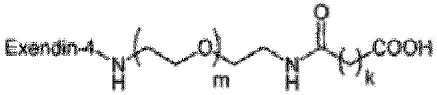 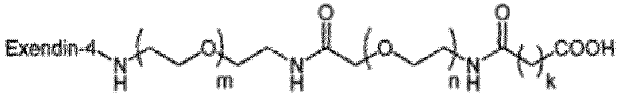 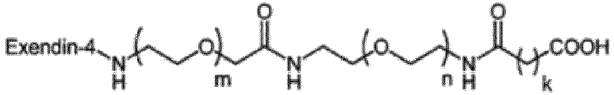 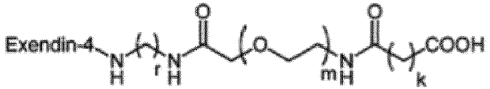 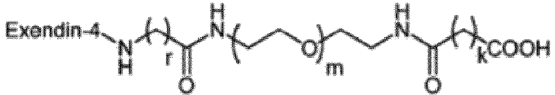 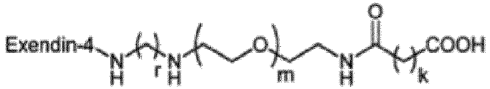 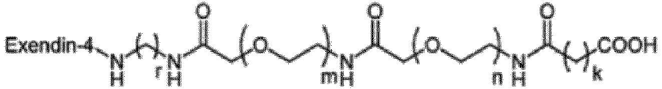 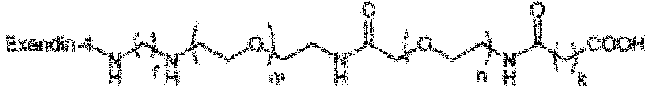 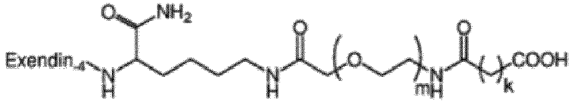 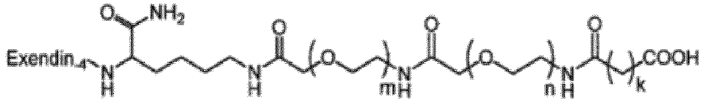 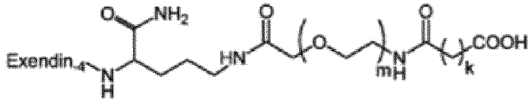 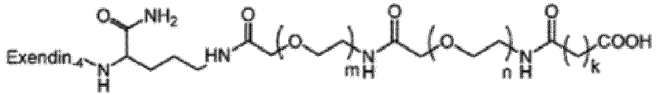 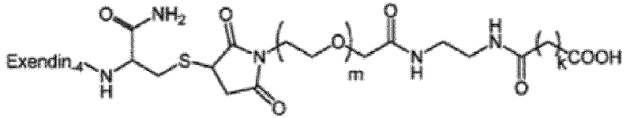 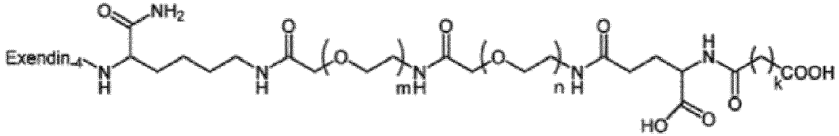 dans lequel m est un entier compris entre 2-20 ; n est un entier compris entre 2-20 ; r est un entier compris entre 1-6; k est un entier compris entre 6 et 20.

4. Modificateur d'exénatide ou ses sels pharmaceutiquement acceptables selon la revendication 3, comprenant les composés suivants :

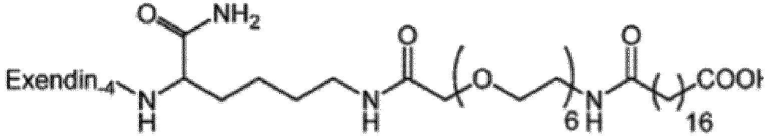

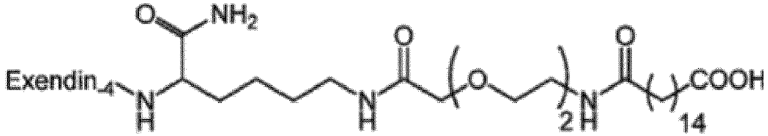

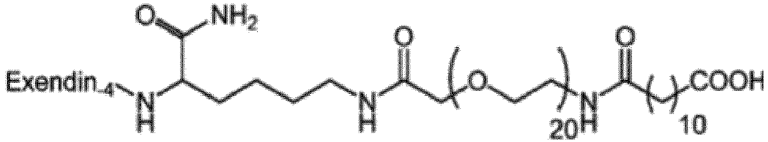

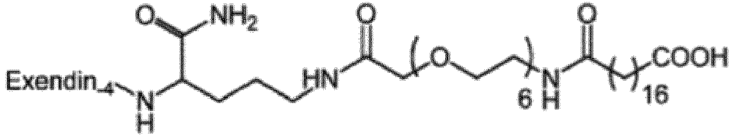

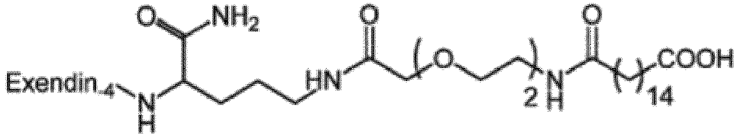

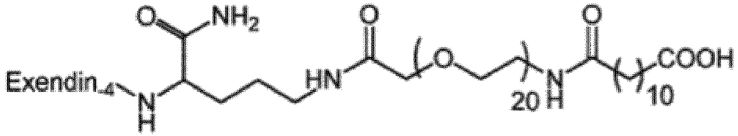

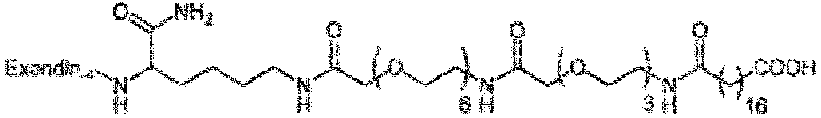

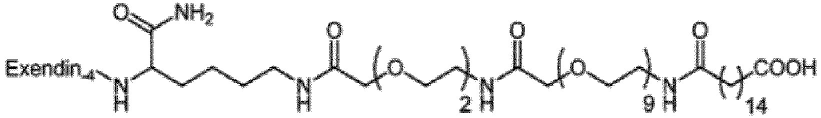

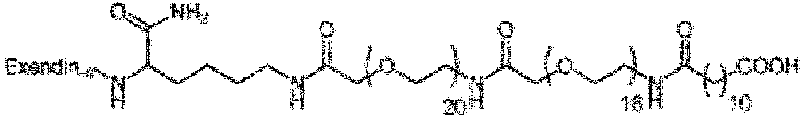

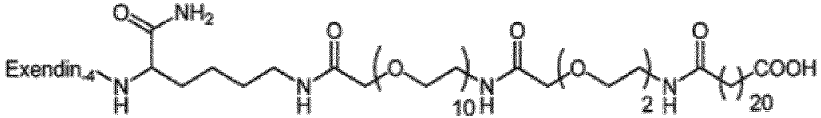

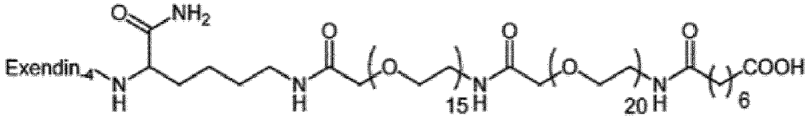

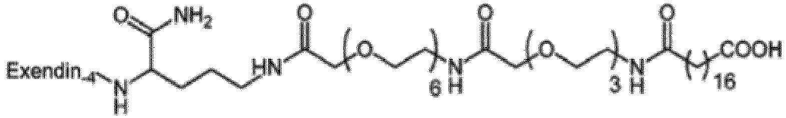

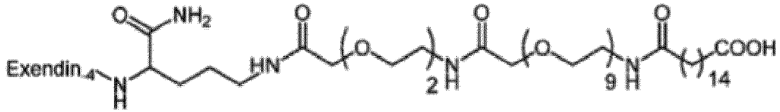

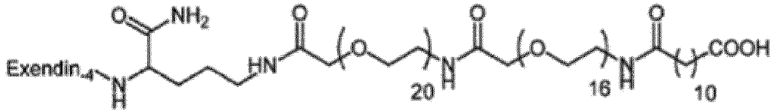

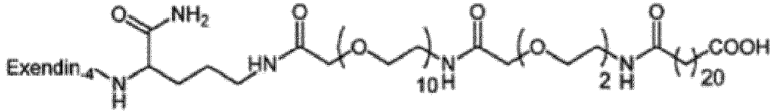

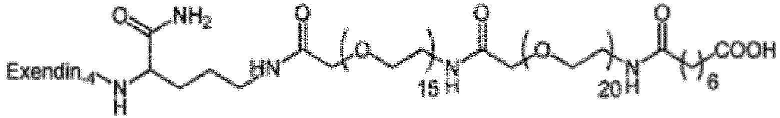

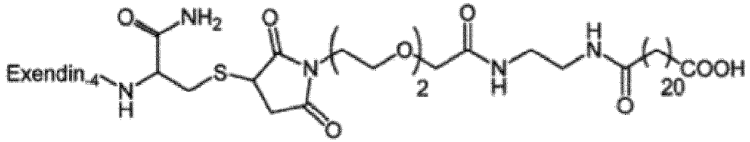

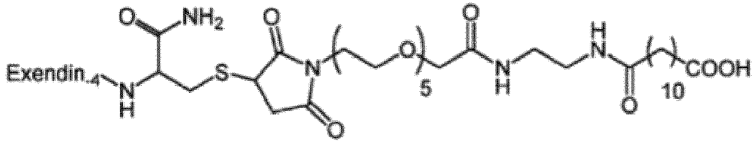

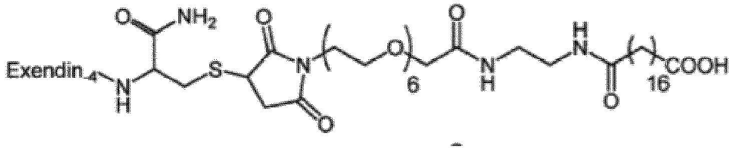

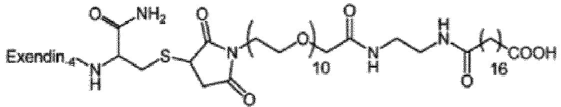

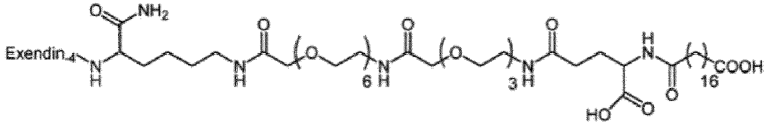

5. Modificateur d'exénatide ou ses sels pharmaceutiquement acceptables selon la revendication 1 ou 2 pour
utilisation dans le traitement des maladies et / ou des symptômes associés au glyco-métabolisme,
ou pour une utilisation dans le traitement du diabète,
ou pour une utilisation dans le traitement de la stéatose hépatique,
ou pour une utilisation dans un procédé pour perdre du poids.

6. Composition pharmaceutique comprenant le modificateur d'exénatide ou ses sels pharmaceutiquement acceptables selon la revendication 1 ou 2 et éventuellement des supports pharmaceutiquement acceptables.

7. Composition selon la revendication 6, pour utilisation dans le traitement de maladies et / ou de symptômes associés au glyco-métabolisme,
ou pour une utilisation dans le traitement du diabète,
ou pour une utilisation dans le traitement de la stéatose hépatique,
ou pour une utilisation dans un procédé pour perdre du poids.
